# EUROPEAN PATENT APPLICATION

(11) **EP 3 692 794 A1**
(43) Date of publication of application: **12.08.2020**
(21) Application number: 20156608.0
(22) Date of filing: 14.09.2012
(51) Int. Cl.: A01N 63/00, C12N 5/0783, A61K 39/00, A61P 35/00, C07K 14/725, C07K 14/54

(54) **TARGETING THE TUMOR MICROENVIRONMENT USING MANIPULATED NKT CELLS**

(30) Priority: 16.09.2011 US 201161535719 P
(62) Divisional of application: 17194467.1
(71) Applicant: Baylor College of Medicine, Houston, TX 77030 (US)
(72) Inventor: METELITSA, Leonid S., Sugar Land, Texas 77479 (US); LIU, Daofeng, Houston, Texas 77025 (US); DOTTI, Gianpietro, Houston, Texas 77030 (US); HECZEY, Andras, Houston, Texas 77025 (US)
(74) Representative: Sterling IP

(57) **Abstract**

The present invention regards methods and/or compositions related to Natural Killer T cells that are engineered to harbor an expression construct that encodes IL-2, IL-4, IL-7, and/or IL-15 and additionally or alternatively comprise a chimeric antigen receptor (CAR). In specific embodiments, the CAR is a CAR that targets the GD2 antigen, for example in neuroblastoma.

SEQUENCE LISTING

<110>  Baylor College Of Medicine

<120>  TARGETING THE TUMOR MICROENVIRONMENT USING MANIPULATED NKT CELLS

<130>  P000180_EPDiv

<160>  24

<170>  PatentIn version 3.5

<210>  1
<211>  19
<212>  DNA
<213>  Artificial Sequence

<220>
<223>  Synthetic Primer

<400>  1
ggtcacacct ctgagtatg                                                    19


<210>  2
<211>  20
<212>  DNA
<213>  Artificial Sequence

<220>
<223>  Synthetic Primer

<400>  2
tggctgctcg tctcaaagta                                                   20


<210>  3
<211>  20
<212>  DNA
<213>  Artificial Sequence

<220>
<223>  Synthetic Primer

<400>  3
cgtgtgaagc ccacaataaa                                                   20


<210>  4
<211>  20
<212>  DNA
<213>  Artificial Sequence

<220>
<223>  Synthetic Primer

<400>  4
cagagctggg ctcctactgt                                                   20


<210>  5
<211>  20
<212>  DNA

<213>  Artificial Sequence

<220>
<223>  Synthetic Primer

<400>  5
gcaggtacag cgtacggttc                                                   20


<210>  6
<211>  20
<212>  DNA
<213>  Artificial Sequence

<220>
<223>  Synthetic Primer

<400>  6
tggagcagtc ctcagatgtc                                                   20


<210>  7
<211>  20
<212>  DNA
<213>  Artificial Sequence

<220>
<223>  Synthetic Primer

<400>  7
catcattggt gccactcaga                                                   20


<210>  8
<211>  20
<212>  DNA
<213>  Artificial Sequence

<220>
<223>  Synthetic Primer

<400>  8
atgcaaagac agcgtcctct                                                   20


<210>  9
<211>  20
<212>  DNA
<213>  Artificial Sequence

<220>
<223>  Synthetic Primer

<400>  9
caatccccga gtaagcatgt                                                   20


<210>  10
<211>  20
<212>  DNA
<213>  Artificial Sequence

<220>

<223>  Synthetic Primer

<400>  10
tgtactcccg aacccatttc                                                    20


<210>  11
<211>  20
<212>  DNA
<213>  Artificial Sequence

<220>
<223>  Synthetic Primer

<400>  11
ggattcactg ggatcagcac                                                    20


<210>  12
<211>  19
<212>  DNA
<213>  Artificial Sequence

<220>
<223>  Synthetic Primer

<400>  12
aggtgactgg ggcattgat                                                     19


<210>  13
<211>  20
<212>  DNA
<213>  Artificial Sequence

<220>
<223>  Synthetic Primer

<400>  13
gtggaggaga agagaaactc                                                    20


<210>  14
<211>  20
<212>  DNA
<213>  Artificial Sequence

<220>
<223>  Synthetic Primer

<400>  14
tgcaaccagt tctctgcatc                                                    20


<210>  15
<211>  20
<212>  DNA
<213>  Artificial Sequence

<220>
<223>  Synthetic Primer

<400>  15

gctgctttga tgtcagtgct                                                    20

<210>  16
<211>  20
<212>  DNA
<213>  Artificial Sequence

<220>
<223>  Synthetic Primer

<400>  16
gcattgaccc gaagctaaag                                                    20

<210>  17
<211>  20
<212>  DNA
<213>  Artificial Sequence

<220>
<223>  Synthetic Primer

<400>  17
cagcagagga acctccagtc                                                    20

<210>  18
<211>  20
<212>  DNA
<213>  Artificial Sequence

<220>
<223>  Synthetic Primer

<400>  18
cttctctgca gcacatccac                                                    20

<210>  19
<211>  20
<212>  DNA
<213>  Artificial Sequence

<220>
<223>  Synthetic Primer

<400>  19
acacagatcc tgcacacacc                                                    20

<210>  20
<211>  20
<212>  DNA
<213>  Artificial Sequence

<220>
<223>  Synthetic Primer

<400>  20
caaacatgag tgtgaagggc                                                    20

<210>  21
<211>  20
<212>  DNA
<213>  Artificial Sequence

<220>
<223>  Synthetic Primer

<400>  21
ctacacgagg ttccagctcc                                                   20


<210>  22
<211>  20
<212>  DNA
<213>  Artificial Sequence

<220>
<223>  Synthetic Primer

<400>  22
tacaccagtg gcaagtgctc                                                   20


<210>  23
<211>  20
<212>  DNA
<213>  Artificial Sequence

<220>
<223>  Synthetic Primer

<400>  23
cttcctcgca actttgtggt                                                   20


<210>  24
<211>  20
<212>  DNA
<213>  Artificial Sequence

<220>
<223>  Synthetic Primer

<400>  24
gcctccagca tgaaagtctc                                                   20

## Description

This application claims priority to U.S. Provisional Patent Application Serial No. 61/535,719, filed September 16, 2012, which application is incorporated by reference herein in its entirety.

### STATEMENT REGARDING FEDERALLY SPONSORED RESEARCH OR DEVELOPMENT

This invention was made with government support under Grant No. 2ROICA116548 and Grant No. ROICA142636, both awarded by the National Institutes of Health, and Grant No. W81XWH-IO-I0425 awarded by the Department of Defense. The government has certain rights in the invention.

### TECHNICAL FIELD

The field of the present invention includes at least biology, cell biology, immunotherapy, and medicine.

### BACKGROUND OF THE INVENTION

Vα24-invariant NKT cells (NKTs) are an evolutionary conserved sub-lineage of T cells that are characterized by the expression of an invariant TCR α-chain, Vα24-Jα18 and reactivity to self- and microbial-derived glycolipids presented by monomorphic HLA class-I-like molecule CD1 d (Kronenberg and Gapin, 2001). The anti-tumor potential of NKTs has been demonstrated in numerous tumor models (Swann *et al.*, 2004; Swann *et al.*,m 2007; Berzofsky and Terabe, 2009). Selective decrease of NKT-cell number and/or their functional activity have been reported in patients with diverse types of cancer (Yanagisawa *et al.*, 2002; Tahir et al,. 2001; Dhodapkar et al,. 2003), suggesting that NKTs may play an important role in the anti-tumor immune responses and, conversely, that an escape from NKTs may contribute to tumor progression. NKTs infiltrate primary human tumors in a subset of children with neuroblastoma (NB) and that NKT-cell infiltration is associated with an improved long-term disease-free survival (Metelitsa *et al.*, 2004). NKT-cell infiltration in primary tumors also served as a prognostic factor of favorable outcome in patients with colorectal cancers (Tachibana *et al.*, 2005) while low levels of circulating NKTs predicted a poor clinical outcome in patients with head and neck squamous cell carcinoma (Molling *et al.*, 2007).

The majority of solid tumors are CD1 d-negative so that tumor cells cannot be a direct target for NKT-cell cytotoxicity (Swann *et al.*, 2007; Metelitsa *et al.*, 2001). Instead, tumor-associated monocytes/macrophages (TAMs) are the only cells in primary NB tumors that have detectable CD1 d expression (Song *et al.*, 2009). Moreover, upon recognition of tumor-derived glycolipids, NKTs produce IFNγ and kill monocytic cells in a CD1d-dependent manner. Since TAMs provide a critical stromal support for tumor cell growth in NB and many other types of cancer (Mantovani *et al.*, 2008; Sica *et al.*, 2008; Sica *et al.*, 2007), NKT cell-mediated killing or inhibition of TAMs explains how NKTs may indirectly impede tumor growth. Other recent reports have generated additional evidence for the importance of NKT-cell interactions with monocytic cells and other myeloid cells in viral and tumor immunity (De Santo *et al.*, 2008; De *et al.*, 2010) and in the potential mechanism of anti-tumor activity of NKTcell ligand, β-Manosylceramide (O'Konek *et al.*, 2011).

Monocytes and other immature myelomonocytic precursors of TAMs localize to the tumor site in response to CCL2, the same chemokine that attracts NKTs (Metelitsa *et al.*, 2004). Monocytic cells, however, respond to multiple other tumorderived chemotactic signals that are not recognized by NKT or other T cells (Allavena *et al.*, 2008). The majority of these factors (VEGF, endothelin, angiopoietin-2) are produced in hypoxic conditions and drive TAM migration to the hypoxic areas (Mantovani *et al.*, 2008; Allavena *et al.*, 2008; Mantovani *et al.*, 2006). Importantly, hypoxic signaling amplifies NF-kB-activation in TAMs leading to high levels of IL6 production and sustained STAT3 activation in tumor cells that in turn promote inflammatory responses in TAMs, providing a positive feedback loop that plays an essential role in tumor progression (Grivennikov *et al.*, 2010).

Although NKTs co-localize with IL-6-producing TAMs in primary NB tissues (Song *et al.*, 2009), the mechanism of this colocalization is not undestood, nor is it clear how TAMs evade the inhibitory activities of NKTs. An understanding of the NKT-TAM interaction in the context of tumor microenvironment is useful for the development of rational cancer immunotherapy that targets tumor-supportive stroma given that NKTs are the only known immune effector cells that specifically recognize and negatively regulate TAMs. As described herein, NKT-cell localization to NB depends not only on tumor-derived CCL2, but also on CCL20, which is produced by TAMs in response to tumor-induced inflammation and hypoxia, which in turn inhibits NKT-cell viability and function. Also as shown herein, IL-15 protects NKTs from hypoxia and transgenic expression of IL-15 in NKTs strongly enhances their anti-tumor efficacy in a metastatic NB model in humanized NOD/SCID/IL2rgamma(null) (hu-NSG) mice.

**Vα24-invariant Natural Killer T cells (NKTs) in tumor immunity and immunotherapy.** As noted above, NKTs are an evolutionary conserved sub-lineage of T cells that are characterized by reactivity to self- and microbial-derived glycolipids presented by monomorphic HLA class-I-like molecule CD1d. They express an invariant TCR α-chain Vα14-Jα18 which is preferentially paired with Vβ11(Porcelli *et al.*, 1993; Lantz and Bendelac, 1994; Bendelac *et al.*, 1995). NKTs are long-lived lymphocytes that develop in the thymus and are present even in neonates as functional cells with effector-memory phenotype (Baev *et al.*, 2004; Godfrey *et al.*, 2010). The first ligand discovered for NKTs was α-Galactosylceramide (αGalCer, KRN7000), which demonstrated potent antitumor properties in mice (Swann *et al.*, 2007; Kronenberg and Gapin, 2002; Benedelac *et al.*, 2007). Despite the fact that the majority of solid tumors both in humans and mice are CD1d-negative, the antitumor potential of NKTs has been demonstrated in numerous models of cancer (Swann *et al.*, 2007) although results from phase I/II clinical trials are still inconclusive beyond the demonstration of safety (Nieda *et al.*, 2004; Ishikawa *et al.*, 2005; Chang *et al.*, 2005; Motohashi *et al.*, 2009; Kunii *et al.*, 2009). NKT-cell infiltration of primary tumors was associated with good outcome in children with neuroblastomas (NB)(Metelitsa *et al.*, 2004), a finding that has been since extended to other malignancies (Dhodapkar, 2009; Tachibana *et al.*, 2005; Molling *et al.*, 2007). NKTs co-localize with tumor-associated macrophages (TAMs) in primary NBs and, upon recognition of tumor-derived glycolipids, specifically kill these cells in a CD1d-dependent manner (Song *et al.*, 2009). Because TAMs provide a critical stromal support for tumor cell growth in many types of cancer, NKT cell-mediated killing of TAMs explains how NKTs indirectly control tumor growth. However, this may not be sufficient for tumor eradication.

**Immunotherapy with CAR.GD2+ EBV-CTLs.** T cells engineered to force expression of chimeric proteins known as chimeric antigen receptors (CARs) can afford a means of combining the targeting properties of antibodies with the "active" biodistribution and effector function of T cells (Dotti *et al.*, 2009). Based on the observations that CTLs specific to EBV (EBV-CTLs), survive more than 10 years after adoptive transfer (Pule *et al.*, 2008), there is a recently performed clinical trial in which patients with relapsed/refractory NB received both EBV-CTLs and autologous T cells (ATCs), each expressing a distinguishable CAR that targeted GD2 antigen expressed by neuroblasts. The results demonstrated that CAR-modified EBV-CTLs had superior persistence and cytotoxicity compared to CAR-modified ATCs (Pule *et al.*, 2008; Louis *et al.*, 2011). The infusion of CAR.GD2+ CTLs was safe and resulted in tumor responses (including a complete remission) in 4/8 patients with refractory/relapsed disease (Pule *et al.*, 2008). Some patients on this study also received leukocyte-depleting anti-CD45 mAb as a part of conditioning. One of the most striking observations was that tumor responses in two such patients receiving anti-CD45 were associated with rapid and extensive tumor necrosis, which was disproportionate to the number of infiltrating T cells observed on biopsy. This clinical observation suggested that targeting tumor-supportive cells of hematopoietic origin could have contributed to the efficacy of NB immunotherapy with CAR.GD2+ CTLs. Because NKTs actively localize to NB tumors and kill TAMs using their native TCR specificity, in embodiments of the present invention expression of CAR.GD2 in therapeutic NKTs enables them to kill both TAMs and neuroblasts that lead to tumor eradication.

**The importance of costimulation for T and NKT cell function.** In the initial human trials, T lymphocytes expressing first-generation CARs showed limited expansion and relatively short persistence (Pule *et al.*, 2008; Till *et al.*, 2008; Kershaw *aet al.*, 2006). This result likely reflects the failure of artificial CAR molecules to fully activate T cells after antigen engagement on tumor cells, especially when the tumor cells lack expression of costimulatory molecules (such as CD80 and CD86) that are required for sustained T cell activation, growth, and survival (Zou, 2005). To provide the costimulation lacking in tumor cell targets and thereby overcome the above limitations, several groups have incorporated costimulatory endodomains, including CD28 and CD137 (Maher *et al.*, 2002; Porter e tal., 2011). In a recently reported clinical trial, patients with B cell lymphomas were simultaneously infused with 2 autologous T cell products expressing CARs with the same specificity for the CD19 antigen. One CAR encoded both the costimulatory CD28 and the ζ-endodomains, while the other encoded only the ζ-endodomain. CAR⁺ T cells containing the CD28 endodomain showed strikingly enhanced expansion and persistence compared with CAR⁺ T cells lacking this endodomain (Savoldo *et al.*, 2011). Another recent phase I/IIa clinical trial showed that T-cells engineered to express CD19 CAR with 4-1BB (CD137) costimulatory domain induced persistent remissions in resistant chronic lymphoid leukemia patients, even those with bulky disease (Porter *et al.*, 2011). There is a growing evidence that co-stimulation plays a critical role in the activation, expansion, and survival of NKTs. Several reports demonstrated that B7:CD28, CD40:CD40L, and OX40:OX40L pathways are important for the expansion and subsequent systemic cytokine production by NKT cells (Uldrich *et al.*, 2005). NKTs also express OX40 and intratumoral administration of DCs modified to express OX40L induced OX40-dependent NKT cell accumulation and IFN-gamma production at the tumor site that resulted in a potent suppression of tumor growth (Zaini *et al.*, 2007). CD137 is not expressed on quiescent NKTs but was rapidly induced upon TCR engagement, and CD137 stimulation by an agonistic anti-4-1BB mAb promoted NKT cell activation resulting in enhanced cytokine production of NKT cells in response to αGalCer (Vinay *et al.*, 2004; Kim *et al.*, 2008). Therefore, in embodiments of the invention expression of CARs with co-stimulatory endodomains derived from CD28, OX40, and/or CD137 (as examples) provides optimal co-stimulation, leading to improved antitumor efficacy of CAR-modified NKTs.

**The role of IL-15 in NKT-cell homeostasis and potential application for cancer immunotherapy.** The tumor microenvironment may affect NKT-cell viability and function, suggesting that an effective immunotherapeutic strategy with NKTs should consider their homeostatic requirements. Studies in mice have demonstrated that NKT-cell development and homeostatic maintenance largely depend on IL-15 (Matsuda *et al.*, 2002). IL-15 also stimulates proliferation and enhances survival of human NKTs (Baev *et al.*, 2004). Unlike in the mouse, however, human CD4-negative (mostly CD8/CD4-double negative, DN) NKTs express much higher levels of IL-2Rβ than CD4+ subset so that IL-15 preferentially expands DN NKTs (Baev *et al.*, 2004). Importantly, DN NKTs are more cytotoxic than CD4+ NKTs, and a recent report demonstrated that only DN NKTs are required for antitumor responses *in vivo* (Crowe *et al.*, 2005). IL-15 protects human NKTs from hypoxia and transgenic expression of IL-15 in adoptively transferred NKTs strongly enhances their anti-metastatic activity in a clinically relevant model of NB in humanized NOD/SCID/IL-2Ry(null) (hu-NSG) mice (Liu *et al.*, 2012). This indicates that expression of IL-15 in NKTs for therapeutic purposes supports expansion, persistence, and antitumor activity of NKTs in cancer patients. In the present invention, co-expression of IL-15 improves *in vivo* persistence and anti-tumor activity of CAR.GD2 NKTs.

**Expression of the inducible Caspase-9 (iCasp-9) suicide gene in transgenic cells.** The use of IL15-expressing NKTs in clinical adoptive transfer may raise potential concerns because of reported leukemic transformation in IL-15 transgenic mice (Fehniger *et al.*, 2001; Sato *et al.*, 2011) and in a human T cell clone (Hsu *et al.*, 2007). Although malignant transformation of engineered T cells is a rare event (Hsu *et al.*, 2007), one can incorporate a suicide gene that allows the elimination of transgeneic cells upon its pharmacologic activation (Quintarelli *et al.*, 2007). Caspase-9 is a major downstream activator of apoptosis (Riedl and Salvesen, 2007), and the iCasp-9 gene has been used as a suicide gene for T cell therapy (Quintarelli *et al.*, 2007; Di *et al.*, 2011; Straathof *et al.*, 2005; Tey *et al.*, 2007). Briefly, caspase-9 cDNA is fused in frame with a 12-kDa human FK506 binding domain (FKBP12) that contains an F36V mutation, allowing dimerization of the caspase-9 and activation of the apoptotic pathway after exposure to the FK506 analog AP1903. T cells expressing the iCasp-9 molecule are efficiently eliminated upon the pharmacologic activation of the suicide gene both *in vitro* and *in vivo* (Quintarelli *et al.*, 2007; Tey *et al.*, 2007). A recently reported clinical trial tested the activity and safety of the iCasp-9/AP1903 system by introducing the gene into donor T cells given to enhance immune reconstitution in recipients of haploidentical stem-cell transplants. A single dose of dimerizing drug, given to four patients in whom GVHD developed, eliminated modified T cells and ended the GVHD without recurrence (Di *et al.*, 2011). One can employ the same safety switch to control gene-modified NKTs in embodiments of the invention.

The present invention provides a solution for a long-felt need in the art to secure methods and compositions that are suitable to target cancer cells and cells that support the cancer cell environment.

### BRIEF SUMMARY OF THE INVENTION

The present invention is directed to methods and compositions associated with cancer therapy, including cell therapy for cancer. Although the cancer may be of any kind, in specific embodiments the cancer is neuroblastoma or melanoma. In particular embodiments of the invention, there is cell therapy for neuroblastoma. In specific embodiments, the cell therapy comprises cell therapy with Natural Killer T cells (NKTs). In particular embodiments of the invention, there is NKT targeting of cells in a tumor microenvironment, such as tumor-associated macrophages (TAMs). In certain embodiments, there is NKT targeting of tumor cells directly. In some embodiments, there is NKT targeting of both TAMs and tumor cells. In specific embodiments, there are NKT cells that harbor one or more expression constructs that allow the NKT cells to target a tumor microenvironment and/or tumor cells.

In particular embodiments of the invention, there are NKT cells that harbor a chimeric antigen receptor that targets GD2 ((2R,4R,5S,6S)-2-[3-[(2S,3S,4R,6S)-6-[(2S,3R,4R,5S,6R)-5-[(2S,3R,4R,5R,6R)-3-acetamido-4,5-dihydroxy-6-(hydroxymethyl)oxan-2-yl]oxy-2-[(2R,3S,4R,5R,6R)-4,5-dihydroxy-2-(hydroxymethyl)-6-[(E)-3-hydroxy-2-(octadecanoylamino)octadec-4-enoxy]oxan-3-yl]oxy-3-hydroxy-6-(hydroxymethyl)oxan-4-yl]oxy-3-amino-6-carboxy-4-hydroxyoxan-2-yl]-2,3-dihydroxypropoxy]-5-amino-4-hydroxy-6-(1,2,3-trihydroxypropyl)oxane-2-carboxylic acid) expressed by cancer cells, and in specific embodiments the cancer cells are neuroblastoma cells; in specific aspects such NKT cells retain the ability to kill TAMs and have TCR specificity. In specific embodiments, the NKT cells alternatively or additionally express IL-15, IL-2, IL-4, IL-7, or a combination thereof, such as from an expression construct in the cells. In particular embodiments, the NKT cells comprise a bi/tricistronic vector that encodes CAR.GD2, optionally encodes a co-stimulatory endodomain; that encodes IL-15; and that optionally comprises a suicide switch, such as an inducible suicide switch.

In some embodiments of the invention, there is an engineered natural killer T-cell comprising a cDNA selected from the group consisting of an IL-2 cDNA, an IL-4 cDNA, an IL-7 cDNA, an IL-15 cDNA, or a mixture thereof. In specific embodiments the cDNA is an IL-2 cDNA or an IL-15 cDNA, including from human. In specific embodiments, the cell encompasses an expression construct that further comprises an inducible suicide gene, such as an inducible caspase-9 suicide gene or the inducible suicide gene is thymidine kinase (sr39 TK). In a specific embodiment, the cell further comprises a CD34 tag.

In some embodiments of the invention, there is a method for treating a cancer comprising administering a therapeutically effective amount of NKT cells of the invention to a subject in need thereof. In the methods, the NKT cell may comprise an inducible suicide gene. In specific embodiments of the method, it further comprises the step of inducing the elimination of the administered natural killer T-cell by activating the inducible suicide gene. In specific embodiments, the inducible suicide gene is inducible caspase-9 suicide gene. The method may further comprise administering AP20187, AP1903, or a mixture thereof to the subject to activate the inducible caspase-9 suicide gene. In some cases the inducible suicide gene is thymidine kinase (sr39 TK) and the method further comprises administering ganciclovir to the subject to activate the thymidine kinase.

In some embodiments of the method, the individual is in need of treatment for cancer and in specific embodiments the cancer is a tumor; in particular cases the tumor microenvironment is hypoxic, such as comprising less than 15% O₂, less than 10% O₂, less than 5% O₂, less than 4% O₂, less than 3% O₂, less than 2% O₂, or less than 1% O₂.

In specific embodiments, the cancer to be treated is selected from the group consisting of neuroblastoma, breast cancer, cervical cancer, ovary cancer, endometrial cancer, melanoma, bladder cancer, lung cancer, pancreatic cancer, colon cancer, prostate cancer, hematopoietic tumors of lymphoid lineage, leukemia, acute lymphocytic leukemia, chronic lymphocytic leukemia, B-celllymphoma, Burkitt's lymphoma, multiple myeloma, Hodgkin's lymphoma, NonHodgkin's lymphoma, myeloid leukemia, acute myelogenous leukemia (AML), chronic myelogenous leukemia, thyroid cancer, thyroid follicular cancer, myelodysplastic syndrome (MDS), tumors of mesenchymal origin, fibrosarcoma, rhabdomyosarcomas, melanoma, uveal melanoma, teratocarcinoma, neuroblastoma, glioma, glioblastoma, benign tumor of the skin, renal cancer, anaplastic large-cell lymphoma, esophageal squamous cells carcinoma, hepatocellular carcinoma, follicular dendritic cell carcinoma, intestinal cancer, muscle-invasive cancer, seminal vesicle tumor, epidermal carcinoma, spleen cancer, bladder cancer, head and neck cancer, stomach cancer, liver cancer, bone cancer, brain cancer, cancer of the retina, biliary cancer, small bowel cancer, salivary gland cancer, cancer of uterus, cancer of testicles, cancer of connective tissue, prostatic hypertrophy, myelodysplasia, Waldenstrom's macroglobinaemia, nasopharyngeal, neuroendocrine cancer myelodysplastic syndrome, mesothelioma, angiosarcoma, Kaposi's sarcoma, carcinoid, oesophagogastric, fallopian tube cancer, peritoneal cancer, papillary serous mullerian cancer, malignant ascites, gastrointestinal stromal tumor (GIST), and a hereditary cancer syndrome selected from Li-Fraumeni syndrome and Von Hippel-Lindau syndrome (VHL).

In particular methods of the invention, the natural killer T-cell is derived from cells from the subject being treated, although the cell may come from another individual. In some cases of the invention, administration of the cells is systemic, and the administration may be parenteral in some cases. In particular embodiments, the natural killer T-cell is administered locally to the tumor. In some embodiments of the methods, they further comprise administering additional cancer therapies to the subject.

In some embodiments of the invention, there is included a diagnosis of cancer, including a diagnosis of neuroblastoma, for example. Diagnosis of neuroblastoma may occur by standard means, such as identification of an unusual lump or mass, for example in the child's abdomen, causing it to swell. Diagnosis may also include one or more of assaying for catecholamines in the blood or urine, imaging tests, CT scans, PET scans, and/or biopsy, such as bone marrow aspiration and biopsy.

In some embodiments, there is an engineered natural killer T-cell comprising an expression construct that encodes IL-2, IL-4, IL-7, IL-15, or a combination thereof. In specific embodiments, the construct encodes IL-2 or IL-15. In certain embodiments, the construct encodes IL-15, including human IL-15, for example. In particular cases the expression construct comprises or is a vector, such as a retroviral vector, lentiviral vector, adenoviral vector, adeno-associated viral vector, or plasmid.

In some embodiments, the NKT cell comprises an expression construct that encodes a chimeric antigen receptor (CAR) that targets GD2. In specific embodiments, the CAR comprises co-stimulatory endodomains selected from the group consisting of CD28, CD137, OX40, CD40, or a combination thereof. In particular embodiments, the expression construct that encodes IL-2, IL-4, IL-7, IL-15, or a combination thereof and the expression construct that encodes a CAR that targets GD2 are the same construct. In specific embodiments, expression of the IL-2, IL-4, IL-7, IL-15, or a combination thereof and expression of the CAR are regulated by the same regulatory sequence(s). The expression construct comprises an inducible suicide gene, in particular cases, such as an inducible caspase-9 suicide gene or where the inducible suicide gene is thymidine kinase (sr39 TK). In specific cases, of the cell, the NKT cell comprises a CD34 tag.

In some embodiments, there is method for treating a cancer comprising administering a therapeutically effective amount of an engineered NKT cell to a subject in need thereof. In specific embodiments, the natural killer T-cell comprises an inducible suicide gene. In some embodiments, of the method it further comprises the step of inducing the elimination of the administered natural killer Tcell by activating the inducible suicide gene. In some cases the inducible suicide gene is inducible caspase-9 suicide gene and the method further comprises administering AP20187, AP1903, or a mixture thereof to the subject to activate the inducible caspase-9 suicide gene. In some cases the inducible suicide gene is thymidine kinase (sr39 TK) and the method further comprises administering ganciclovir to the subject to activate the thymidine kinase. In some cases of methods of the invention, the NKT cell comprises a CD34 tag.

In some methods of the invention, the cancer is a tumor and in particular aspects the tumor microenvironment is hypoxic. In certain cases, the tumor microenvironment comprises less than 15% O₂, less than 10% O₂, less than 5% O₂, less than 4% O₂, less than 3% O₂, less than 2% O₂, or less than 1% O₂. In specific embodiments, the cancer is selected from the group consisting of breast cancer, cervical cancer, ovary cancer, endometrial cancer, melanoma, bladder cancer, lung cancer, pancreatic cancer, colon cancer, prostate cancer, hematopoietic tumors of lymphoid lineage, leukemia, acute lymphocytic leukemia, chronic lymphocytic leukemia, B-celllymphoma, Burkitt's lymphoma, multiple myeloma, Hodgkin's lymphoma, NonHodgkin's lymphoma, myeloid leukemia, acute myelogenous leukemia (AML), chronic myelogenous leukemia, thyroid cancer, thyroid follicular cancer, myelodysplastic syndrome (MDS), tumors of mesenchymal origin, fibrosarcoma, rhabdomyosarcomas, melanoma, uveal melanoma, teratocarcinoma, neuroblastoma, glioma, glioblastoma, benign tumor of the skin, renal cancer, anaplastic large-cell lymphoma, esophageal squamous cells carcinoma, hepatocellular carcinoma, follicular dendritic cell carcinoma, intestinal cancer, muscle-invasive cancer, seminal vesicle tumor, epidermal carcinoma, spleen cancer, bladder cancer, head and neck cancer, stomach cancer, liver cancer, bone cancer, brain cancer, cancer of the retina, biliary cancer, small bowel cancer, salivary gland cancer, cancer of uterus, cancer of testicles, cancer of connective tissue, prostatic hypertrophy, myelodysplasia, Waldenstrom's macroglobinaemia, nasopharyngeal, neuroendocrine cancer myelodysplastic syndrome, mesothelioma, angiosarcoma, Kaposi's sarcoma, carcinoid, oesophagogastric, fallopian tube cancer, peritoneal cancer, papillary serous mullerian cancer, malignant ascites, gastrointestinal stromal tumor (GIST), and a hereditary cancer syndrome selected from Li-Fraumeni syndrome and Von Hippel-Lindau syndrome (VHL). The cancer is a neuroblastoma in particular embodiments. In some cases, the IL-15 is human IL-15. The NKT cells may be derived from cells from the subject or from another individual. Administration of the cells may be systemic or parenteral. In certain aspects, the natural killer T-cell is administered locally to the tumor. In some cases, the method further comprises administering one or more additional cancer therapies to the subject.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description. It is to be expressly understood, however, that the disclosure is provided for the purpose of illustration and description only and is not intended as a definition of the limits of the present invention.

### DESCRIPTION OF THE DRAWINGS

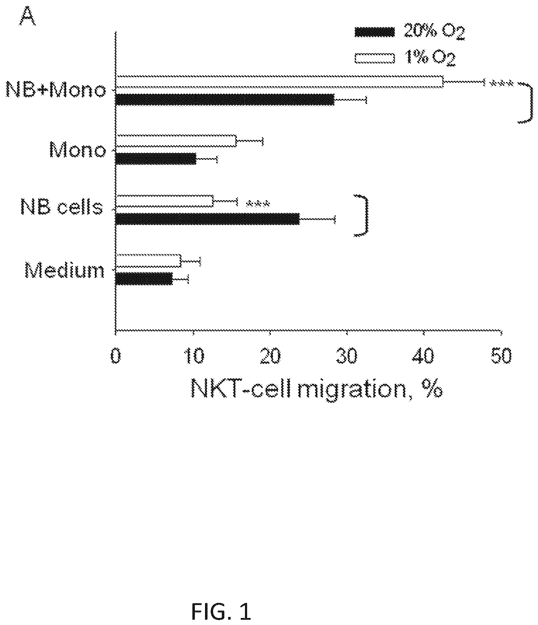

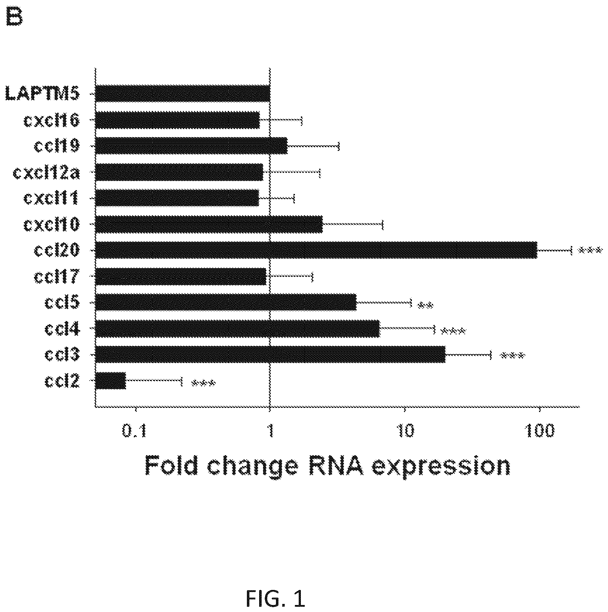

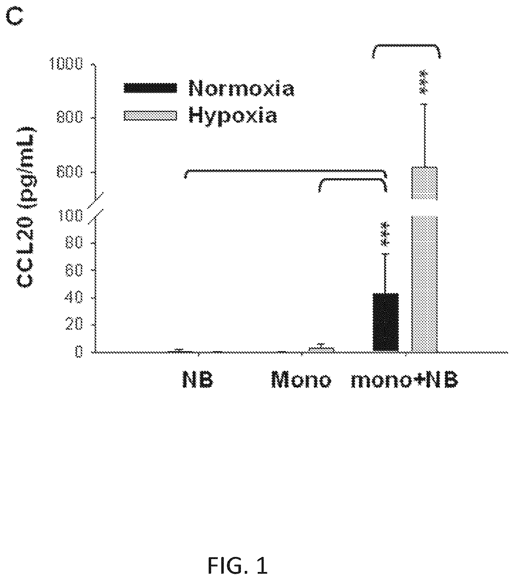

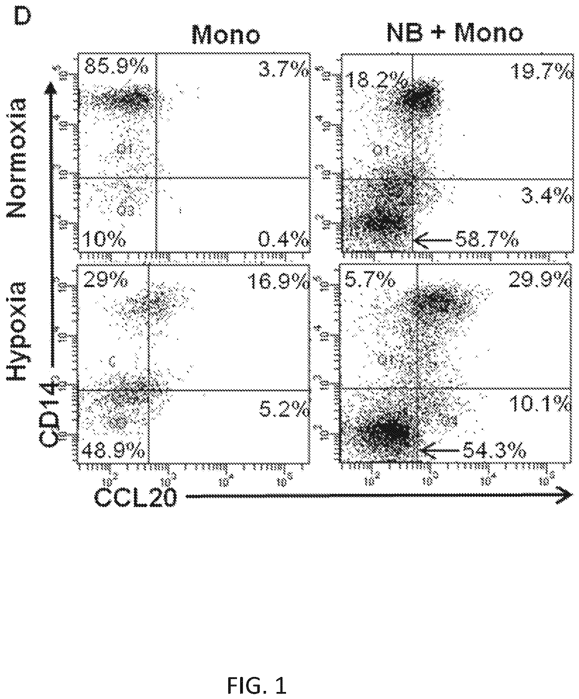

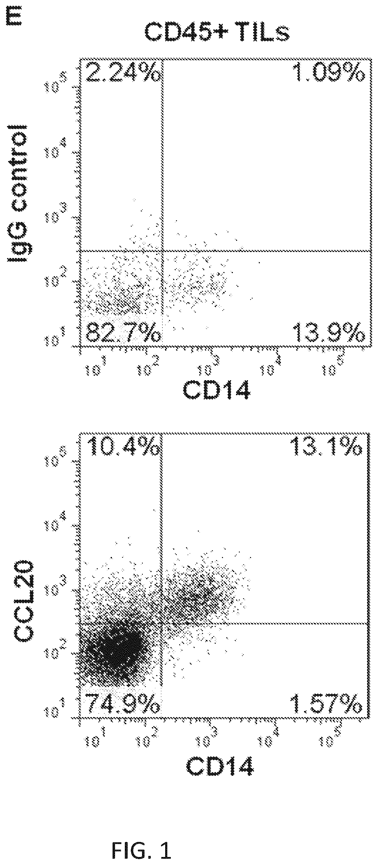

Figure 1: Contact with NB cells and hypoxia synergistically induce CCL20 in human monocytes. (A) Primary monocytes were co-cultured with CHLA-255 NB cells (1:1 ratio) for 48 hr in normoxic (20% O₂) or hypoxic (1% O₂ conditions and supernatants were placed in bottom chambers of dual-chambers plates with 5 µM-pore membranes with or without addition of the indicated neutralizing antibodies or their isotype control. NKTs were placed in the upper chambers and allowed to migrate for 3 h. The rate of NKT-cell migration was quantified by FACS. Results are Mean ± SD from 3 experiments in triplicates, **p< 0.01, ***P<0.001, one-way ANOVA. (B) Monocytes were co-cultured with or without CHLA-255 NB cells for 36 h in normoxic or hypoxic conditions followed by mRNA isolation and quantitative real-time PCR analysis of 11 chemokine genes that are known to attract human NKTs. Data are from a representative of 3 experiments in triplicates. (C) Monocytes were cultured alone or with CHLA-255 NB cells in hypoxic or normoxic conditions for 48 h. CCL20 concentration was quantified in the supernatants from indicated conditions using ELISA. (D) Cells were cultured as in C and analazed for intracellular CCL20 accumulation in CD14⁺ monocytes and CD14^{neg} NB cells. The regions were set using correspoding isotype controls. (E). Tumor infiltrating leukocytes were isolated from a cell suspension of freshly resected primary NB by a gradient centrifugation and cultured with GolgiStop™ for 4 h followed by FACS. After gating on CD45+ events, CCL20 accumulation was examined in CD14⁺ TAMs (bottom plot) and compared to the the corresponding isotype control (upper plot). Data are from a representative of three experiments.

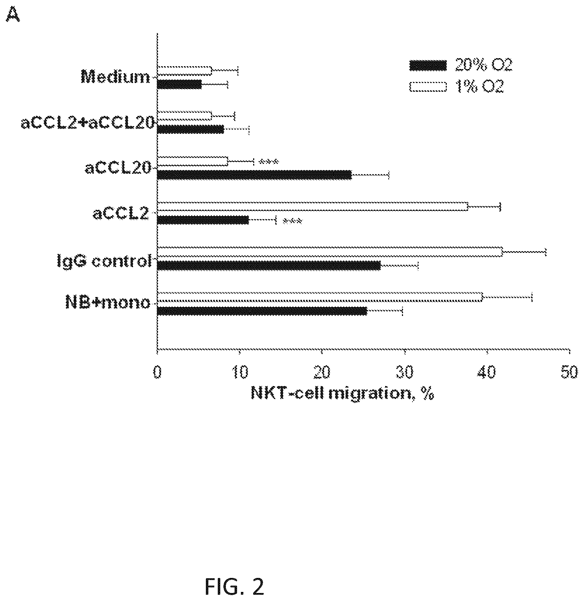

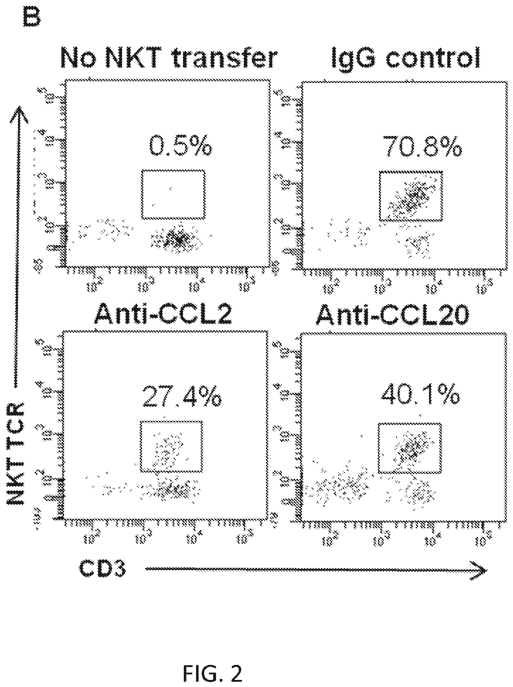

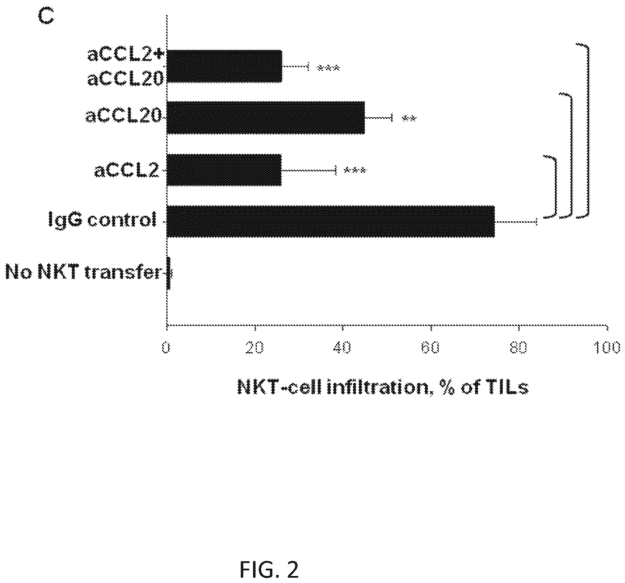

Figure 2: CCL20 is required for NKT-cell migration toward hypoxic NB/monocyte culture and NB tumors in hu-NSG mice. (A) Monocytes were co-cultured with CHLA-255 NB cells for 48 hr in normoxic or hypoxic conditions followed by analysis of NKT-cell *in vitro* migration with or without indicated neutralizing antibodies or their isotype control as in Fig. 1A. Results are Mean ± SD from 3 experiments in triplicates, P<0.001, one-way ANOVA. (B) Xenografts of CHLA255/luc cells were established under renal capsule of huNSG mice followed by i/v transfer of ex-vivo expanded human NKTs (5X10⁷ per mouse) or PBS (control). Just before NKT-cell transfer mice received i/p injections of the indicated neutralizing antibodies or their isotype control. The tumor-infiltrating leukocytes were analyzed by FACS on day 3 after NKT-cell transfer. After gating on hCD45⁺ cells, NKTs were identified as CD3⁺Va24-Ja18⁺ events. Data are from a representative of five mice per group. (C) Bar graphs represent M ± SD values of tumor-infiltrating NKT-cell frequency from the experiment described in B. **p< 0.01, ***P<0.001, one-way ANOVA.

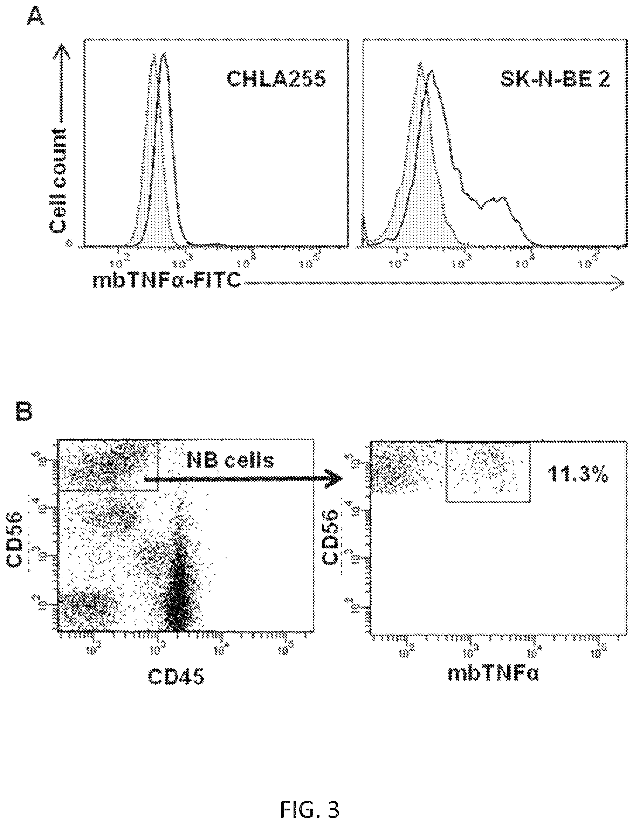

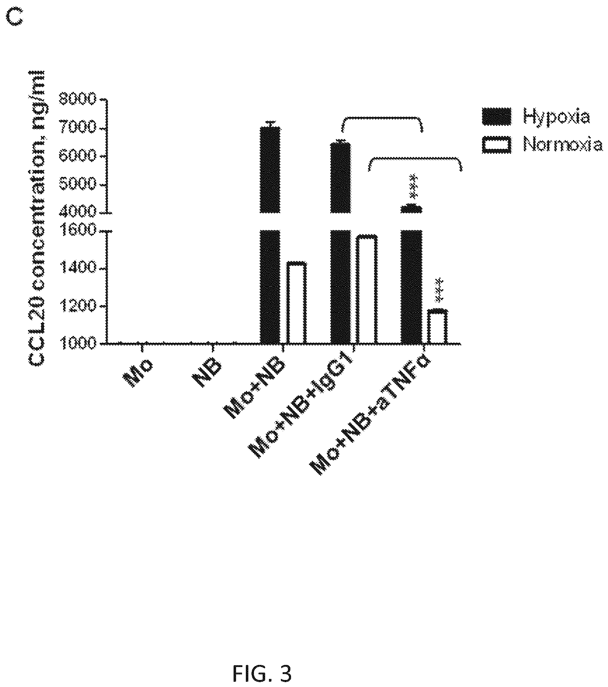

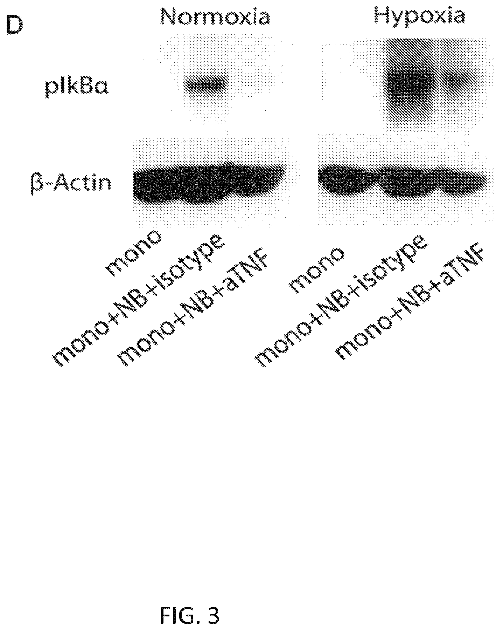

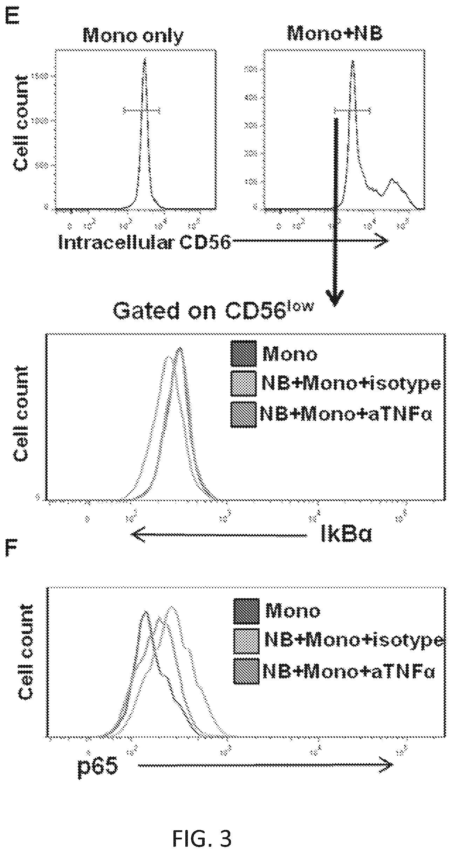

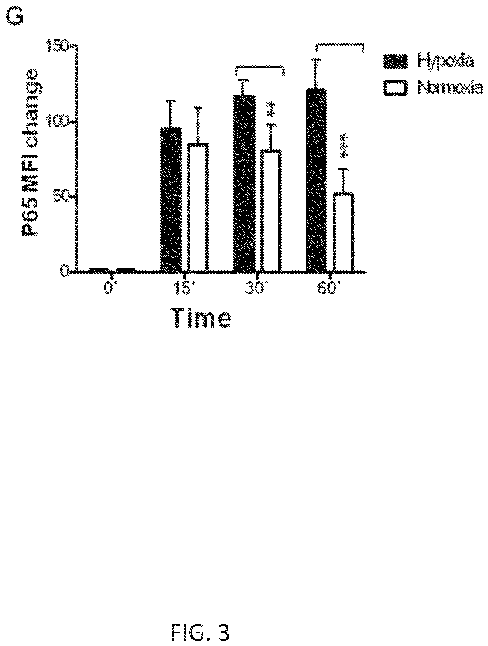

Figure 3: mbTNFα on NB cells induces NF-kB activation in monocytes. (A) Cultured NB cells were suspended using 2% EDTA without trypsin and analyzed by FACS for the cell surface expression of mbTNFα in two representative NB cell lines (tinted: isotype control, open: anti-mbTNFα). (B) Cell suspensions from freshly resected primary NB tumors were stained for the indicated surface markers. mbTNFα expression on NB cell (right) was analyzed after gating on CD56^{high}CD45^{neg} events (left). (C) NB cells were pre-incubated with 50 ng/mL of anti-human TNFα (Clone 1825, R&D system) or isotype control mAb for 1 hr before addition of monocytes. NB and monocytes alone were used as controls. CCL20 concentration in the culture supernatant was determined by ELISA after 36 h. Results are Mean ± SD from 3 experiments in triplicates, ***P<0.001, one-way ANOVA (D). Monocytes were cultured alone in non-adherent plates or on top of NB-cell monolayer and with addition (when indicated) anti-TNFα or isotype control mAb in normoxia or hypoxia for 16 h followed by monocyte detachment and western blotting for pIκBα using β-actin as a loading control. (E) The experiment was set-up as in 0 followed by intracellular staining for IκBα or (F) phospho-p65 in monocytes after gating out NB cells as CD56^{high} events. (G) Kinetics of phospho-p65 expression in monocytes upon co-culture with NB cells in normoxic and hypoxic conditions. Data are from a representative of three experiments.

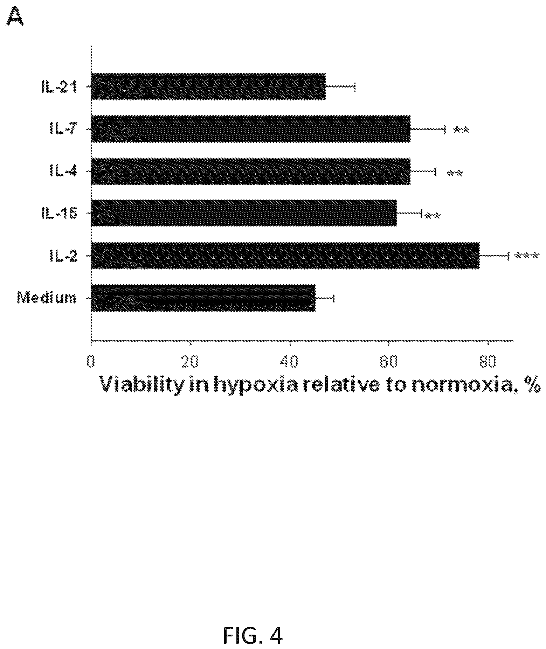

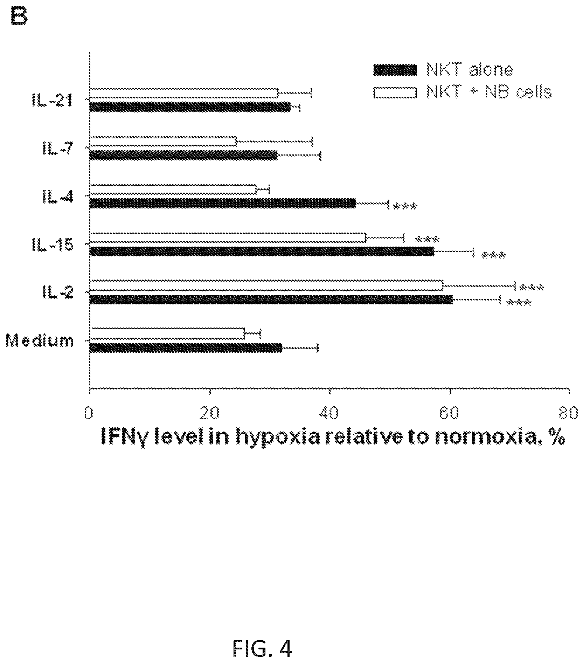

Figure 4: NKT-cell viability and function are inhibited by hypoxia and protected by cytokines. (A) Resting NKTs were culture under hypoxia or normoxia in the presence of absence of the indicated cytokines at 200 U/ml for 24 h. Cell viability was assessed by trypan blue staining. B. NKTs were cultured for 24 h as in A followed by TCR stimulation with 6B11 mAb. Cytokine release was quantified by CBAPlex assay from 24-hr supernatants. The cytokine amount was normalized by the percent viable cells in the corresponding conditions. Results are Mean ± SD from 3 experiments in triplicates, **P<0.01 ***P<0.001, one-way ANOVA.

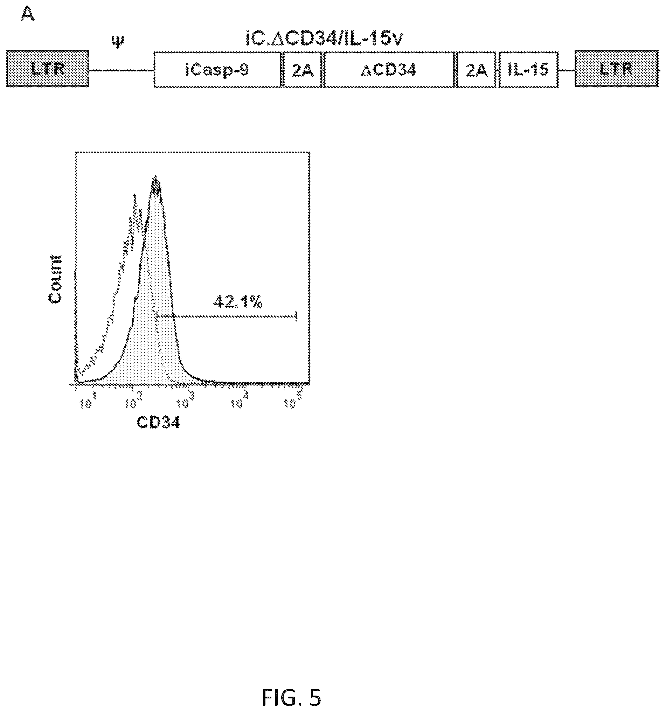

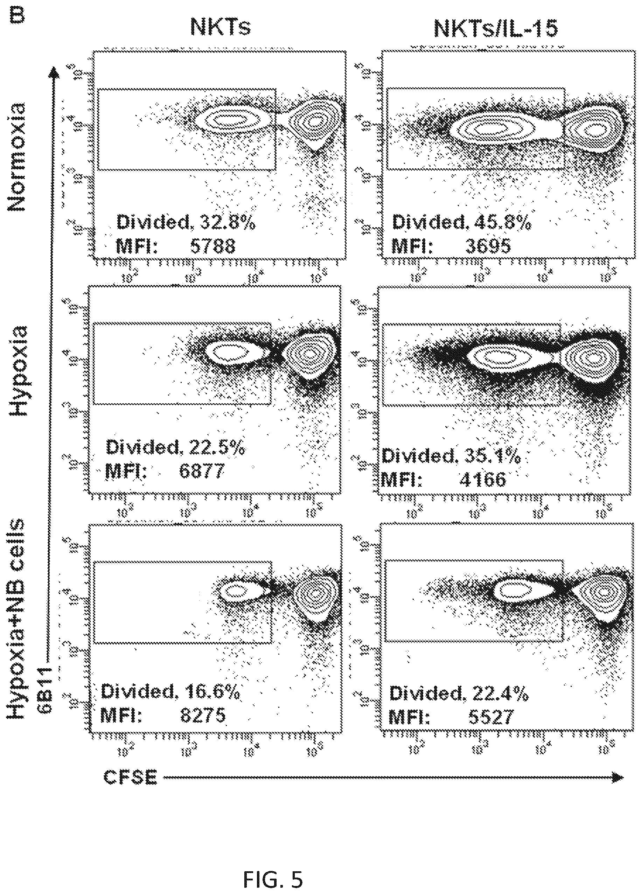

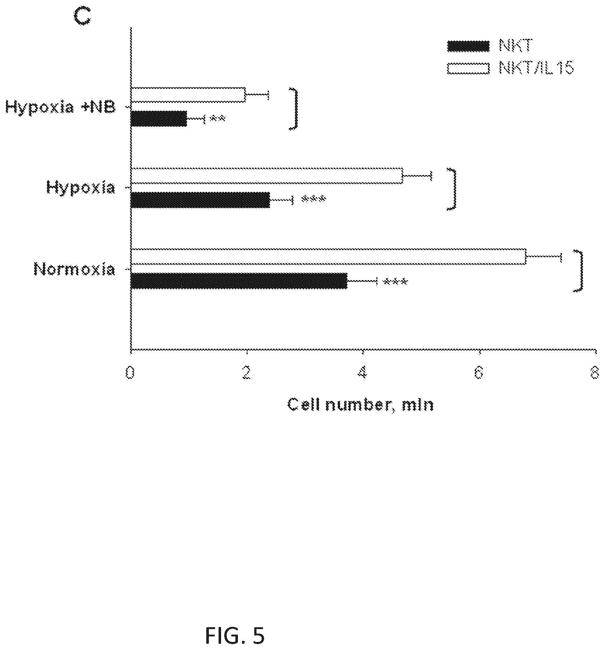

Figure 5. Transgenic expression of IL-15 in NKTs protects them from hypoxia. (A) The schema of the retroviral construct used to transduce NKTs. Proliferating NKTs were transduced with the IL-15-containing retroviral vector and the transduced cells were identified by FACS using ΔCD34 tag. (B) NKT and NKT/IL15 cells were labeled with CFSE and TCR-stimulated with OKT3 mAb in the absence or presence of NB cells (1:1 ratio) in normoxia or hypoxia (1 mln cells/well). The percent of proliferated cells (loss of CFSE expression) was quantified by FACS after 5-day culture with IL-2 (50 U/ml). Data are from a representative of four experiments with NKTs from four donors. (C) The absolute number of viable NKTs was quantified after 5-day culture using hemocytometer and trypan blue staining. Shown are Mean ± SD of cells per condition from four experiments, **P<0.01 ***P<0.001..

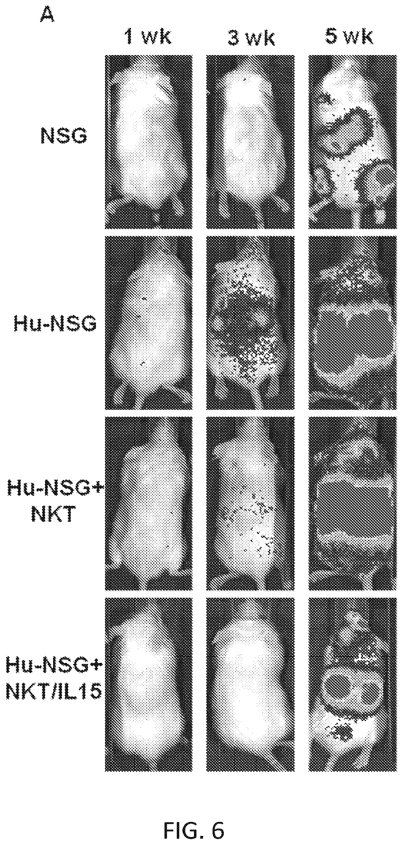

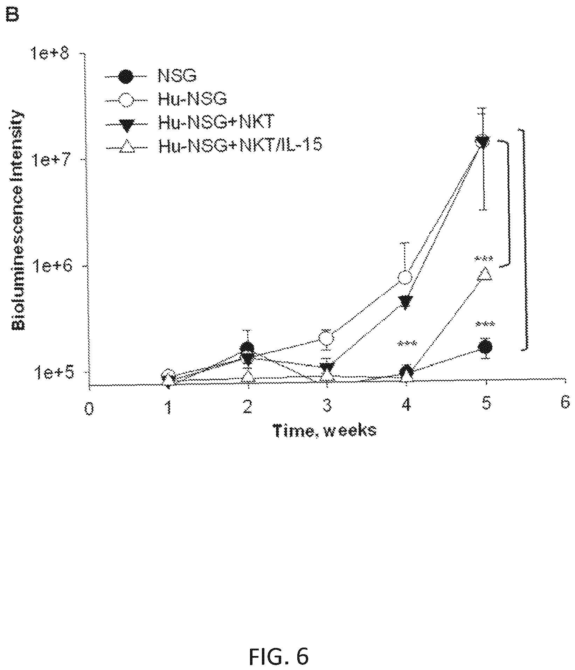

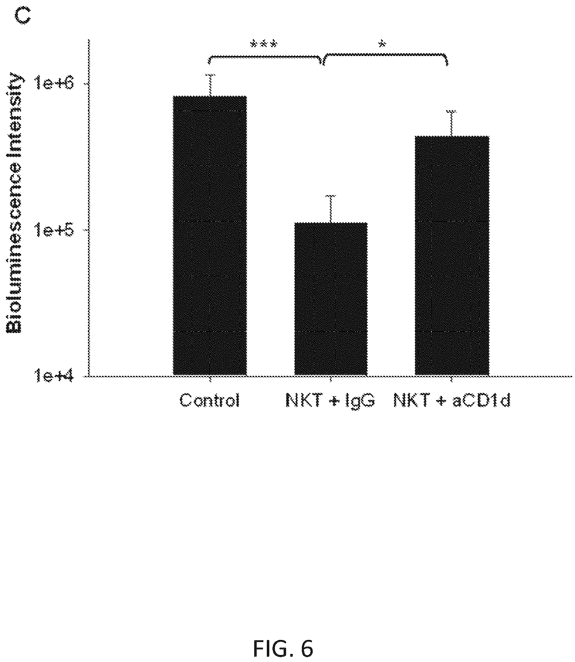

Figure 6. IL-15-transduced NKTs have potent anti-tumor activity in a metastatic NB model in hu-NSG mice. NSG mice were sublethally irradiated and transplanted with human cord blood CD34+ stem cells (hu-NSG) or used as a control (NSG). Three months after stem cell transfer, mice received i/v injection of 10⁶ CHLA-255/luc NB cells alone or followed by 10⁷ NKT or NKT/IL-15 cells. The metastatic tumor growth was monitored by weekly BL imaging. (A) Representative BL images of mice in each group at the indicated time intervals after tumor cell injection. (B) M ± SD values from of one of two experiments with 5 mice per group. ***P<0.001. (C) Mice were pre-treated with anti-CD1d blocking or isotype control mAb before transfer of NKT/IL15 cells. M ± SD values at week 5 from of one of two experiments with 5 mice per group. *P<0.05, ***P<0.001.

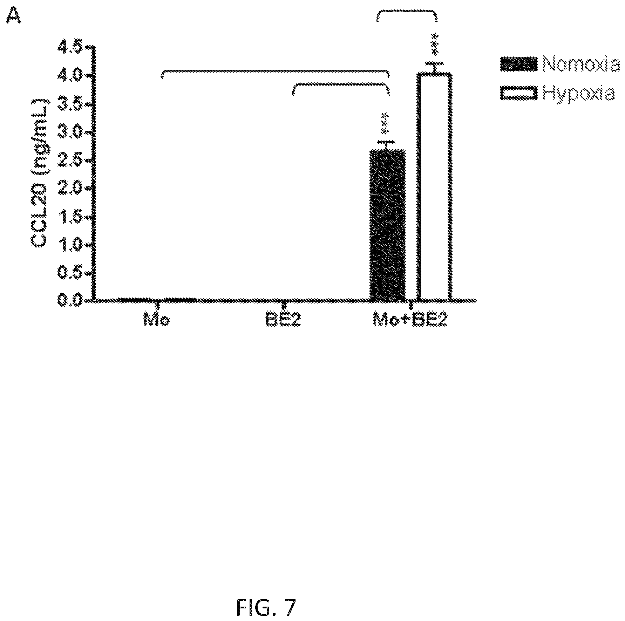

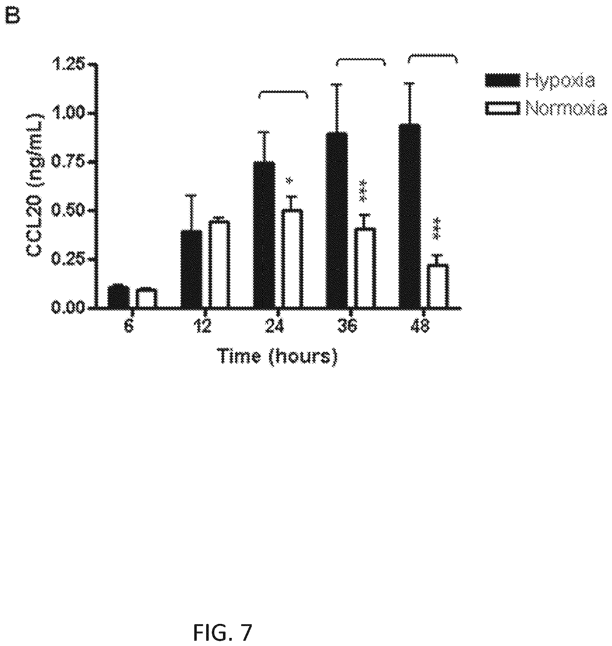

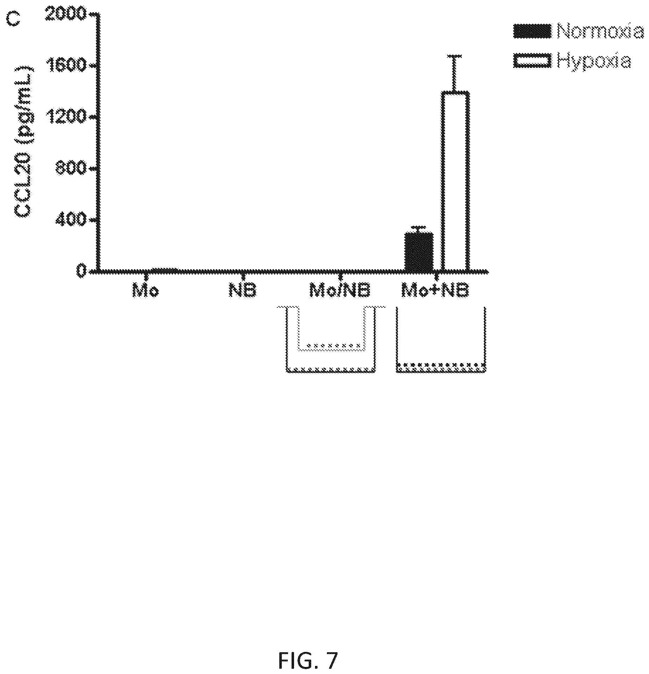

Figure 7: Contact with NB cells and hypoxia synergistically induce CCL20 in human monocytes. (A) Monocytes of donor-3 were co-cultured with CHLA-255 NB cells under normoxic or hypoxic conditions. Supernatants were collected at indicated time points and CCL20 concentration was measured by ELISA. (B) Monocytes from donor-11 and NB cells were cultured alone or co-cultured in the same wells or in Transwell chambers, separated by 0.4 µm membrane for 36 hrs. CCL20 concentration was measured by ELISA. Data are from a representative of three experiments. (C) Monocytes and NB cells were cultured alone or co-cultured in the same wells or in Transwell chambers, separated by 0.4 µm membrane for 36 hrs. CCL20 concentration was measured by ELISA. Data are from a representative of three experiments.

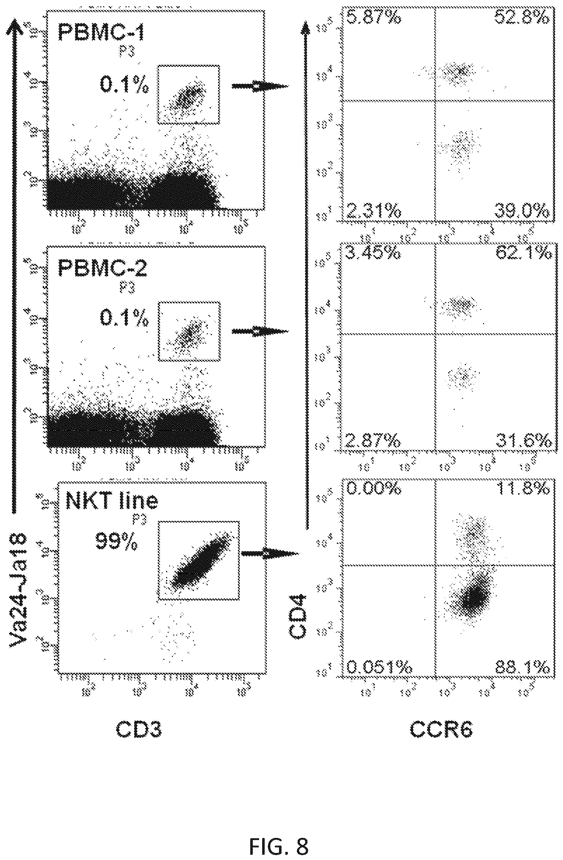

Figure 8: CCR6 expression on NKT cells. Primary NKT cells from freshly isolated PBMC of two individuals (PBMC-1 and -2) and *ex vivo* expanded NKT cells (NKT line) were identified by FACS as CD3⁺Va24-Ja18⁺ events (left panel) and analyzed for CD4 and CCR6 expression (right panel).

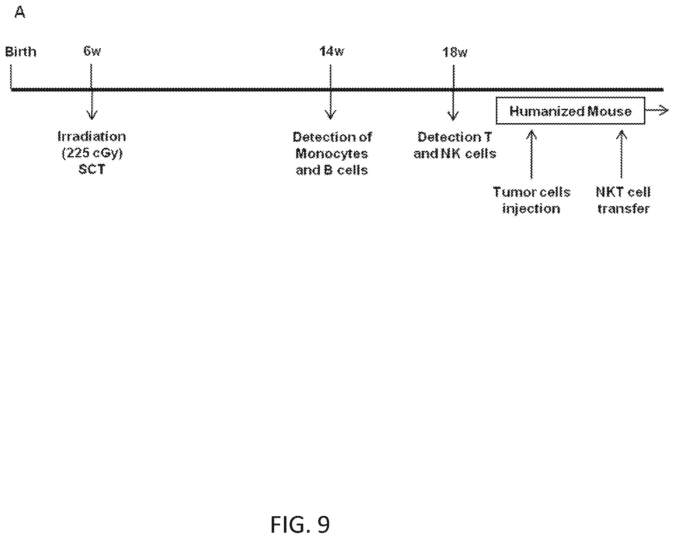

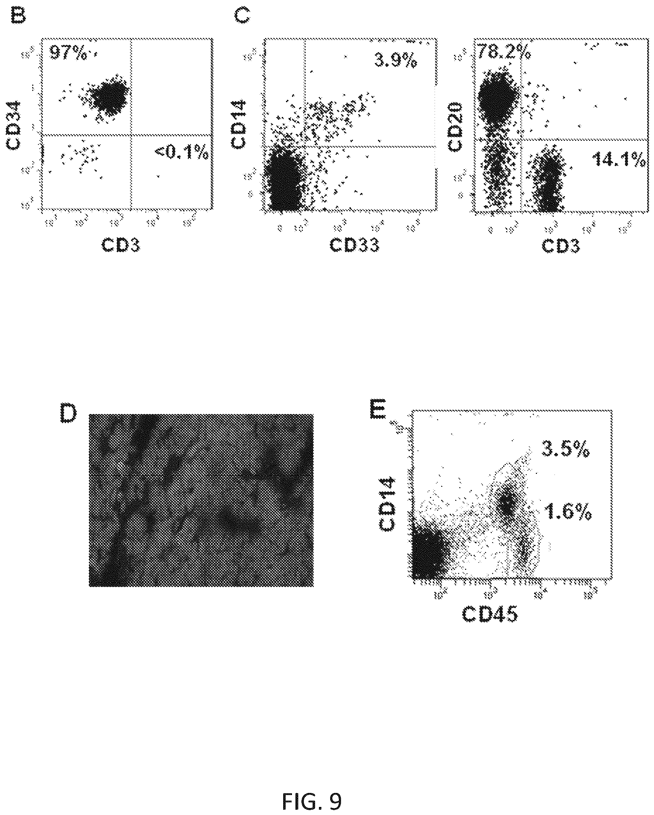

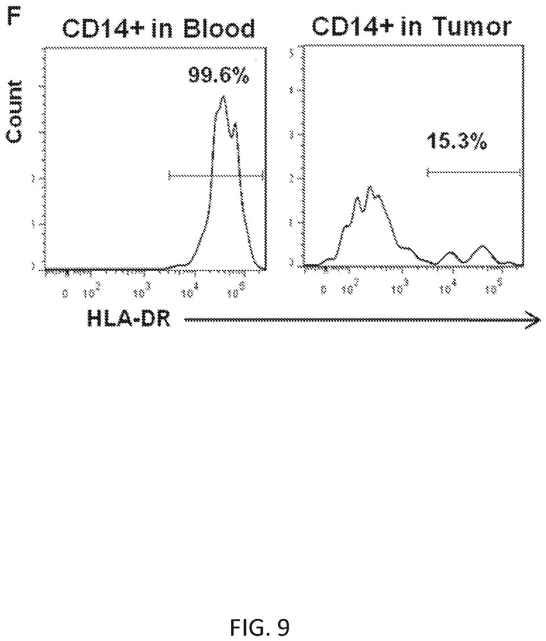

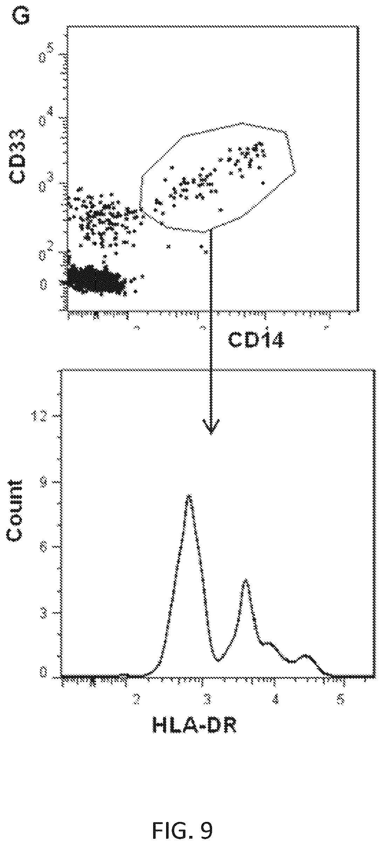

Figure 9: Neuroblastoma model in hu-NSG mice. (A) Six-week old NSG mice were sublethally irradiated and transplanted with human cord blood CD34+ hematopoietic stem cells (SCT). Human monocytes and B cells appeared in peripheral blood at 2 months and T cells at 3 months after SCT. NB cells were injected either under the renal capsule or intravenously (metastatic model) at 3.5 months after SCT. The adoptive transfer of NKT cells was performed at different intervals after tumor cell injection depending on the experimental setup. (B) After two rounds of positive selection, cord blood stem cell preparations had >95% CD34+ stem cells and <0.1 % CD3+ T cells. (C) Representative plots demonstrate a typical reconstitution of monocytes, T and B lymphocytes in peripheral blood of hu-NSG mice at the time of NB-cell injection. (D) IF staining of NB graft with anti-human CD45 mAb (red), X20 magnification. (E) FACS analysis of viable cells in tumor suspension for human CD45 and CD14. (F) CD14+ monocytic cells were analyzed for the level of HLA-DR expression in peripheral blood (left) and in NB tumor grafts of the same animals. Shown is a representative of five mice. (G) HLA-DR expression (bottom) was analyzed in TAMs from primary human NB tumor after gating on CD45+CD33+CD14+ cell (top). Shown is a representative of three primary tumors.

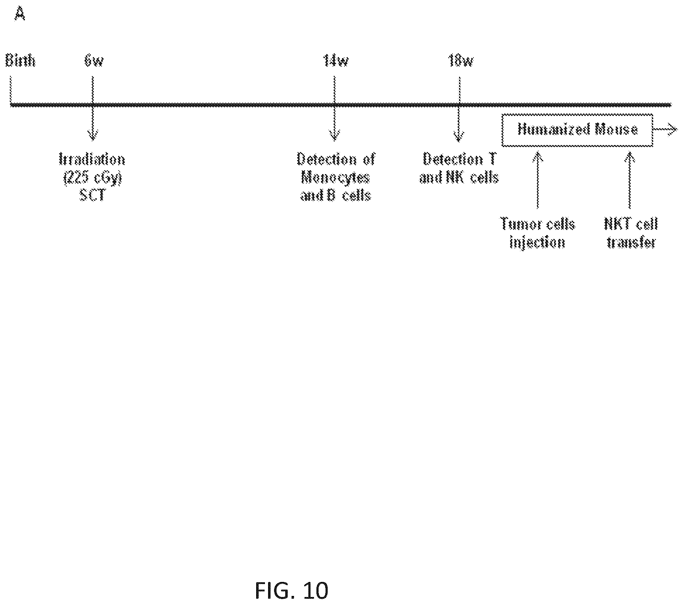

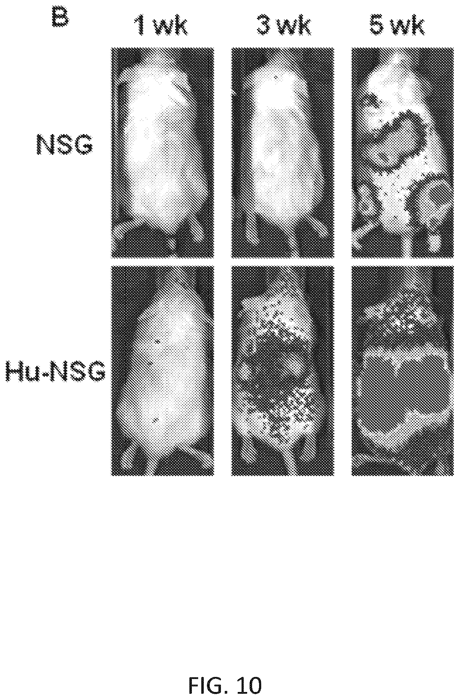

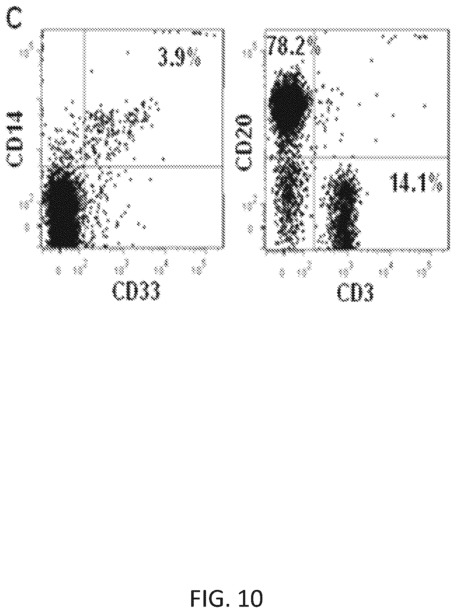

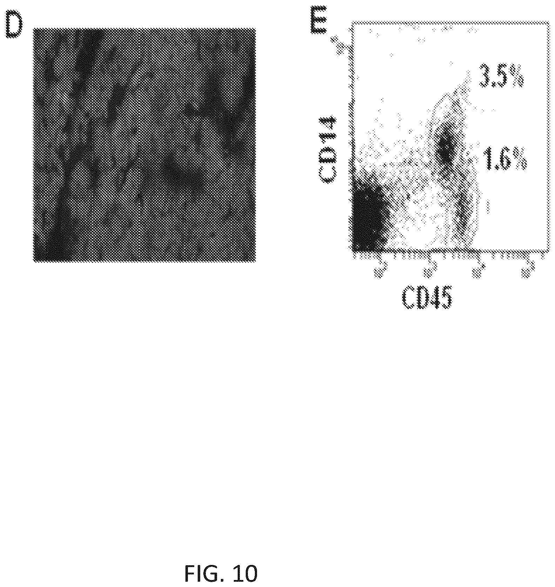

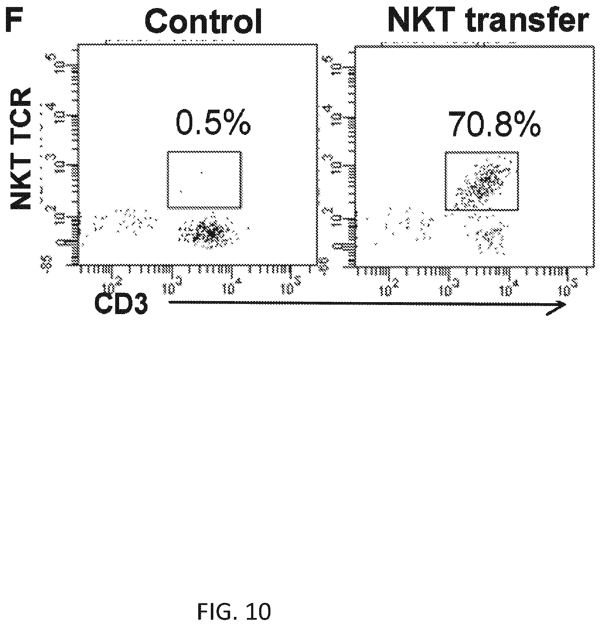

Figure 10. Neuroblastoma model in hu-NSG mice. (A) Six-week old NSG mice were sublethally irradiated and transplanted with human cord blood CD34+ hematopoietic stem cells (SCT). Human monocytes and B cells appeared in peripheral blood at 2 months and T cells at 3 months after SCT. CHLA-255/luc NB cells were injected either under the renal capsule (orthotopic model) or intravenously (metastatic model) at 3.5 months after SCT. The adoptive transfer of NKT cells was performed at different intervals after tumor cell injection depending on the experimental setup. (B) The metastatic tumor growth of CHLA-255/luc cells was compared in hu-NSG mice vs. NSG mice. Weekly BL imaging data are from representative of 5 mice per group (C) Representative plots demonstrate a typical reconstitution of monocytes, T and B lymphocytes in peripheral blood of hu-NSG mice at the time of NB-cell injection. (D) IF staining of NB graft with anti-human CD45 mAb (red), X20 magnification. (E) FACS analysis of viable cells in tumor suspension for human CD45 and CD14 (TAMs). (F). *Ex-vivo* expanded human NKTs (5X10⁷ per mouse) or PBS (control) were i/v injected to hu-NSG mice bearing 3-week established orthotopic NB grafts. The tumor-infiltrating leukocytes were analyzed by FACS on day 3 after NKT-cell transfer. After gating on hCD45⁺ cells, NKTs were identified as CD3⁺Va24-Ja18⁺ events. Data are from a representative of five mice per group.

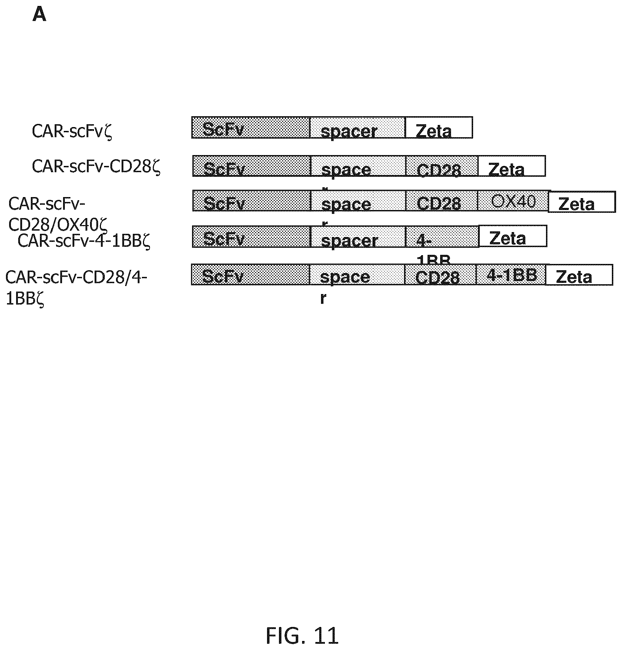

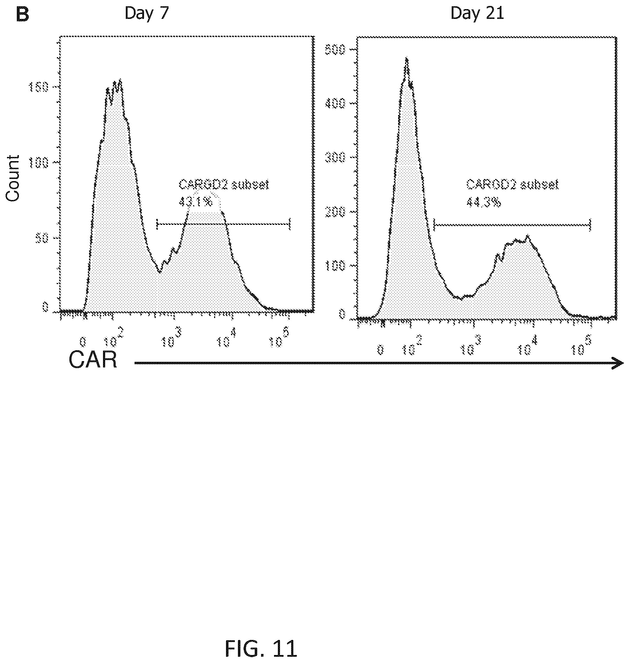

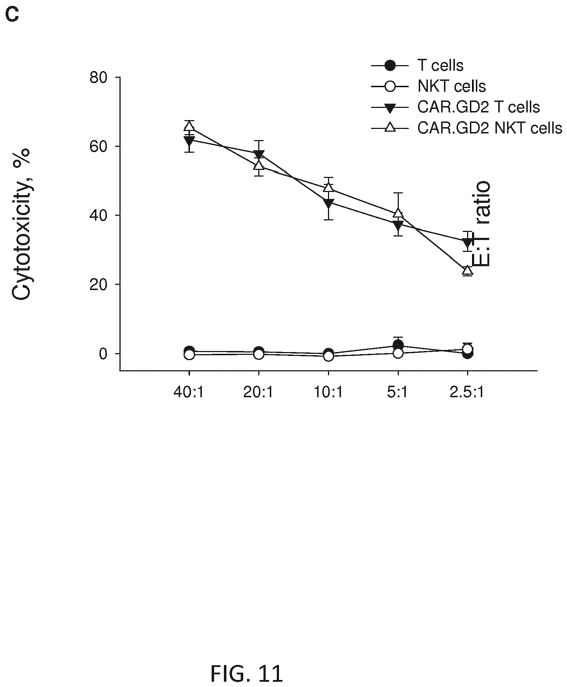

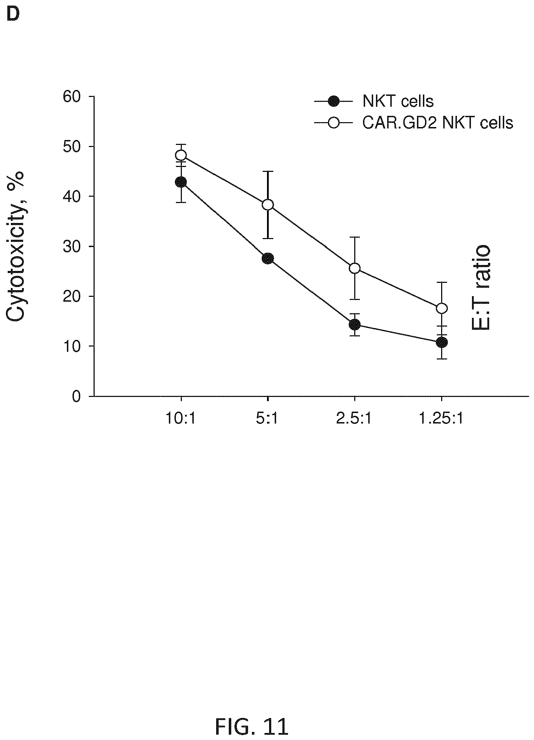

Figure 11. Expression of functional GD2.CARs in NKTs. (A) Schematic representation of CARs incorporating co-stimulatory moieties. (B) Typical expression of one of the CAR constructs (14g2a.CD28.0X40.zeta) in *ex vivo* expanding NKTs at days 7 and 21 after retroviral transduction as detected using a specific anti-idiotype antibody 1A7⁹. (C) *In vitro* cytotoxicity of CAR.GD2 NKT and T cells from the same individual against GD2+ CHLA-255 NB cells in 4-hr ⁵¹Cr-release assay. (D) *In vitro* cytotoxicity of CAR.GD2 and parental NKTs against CD1d+ J32 cells in 4-hr ⁵¹Cr-release assay. Shown are representatives of three independent experiments.

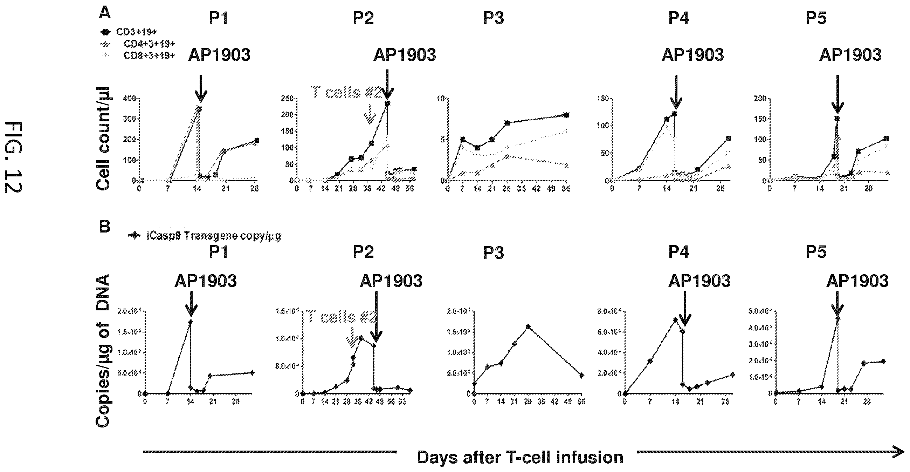

Figure 12. Pharmacologic activation of the iCasp-9 suicide gene efficiently eliminates gene-modified T cells in leukemia patients. (A) FACS analysis for iCasp9-transduced T cells from five patients receiving cellular therapy following HLA-haploidentical stem cell transplantation for relapsed leukemia. Patients 1, 2, 4 and 5 with high level engraftment developed skin/liver GvHD and received a single dose of the dimerizing drug AP1903. (**B**) Copies of the *iCasp9* transgene per µg of DNA from peripheral blood mononuclear cells, evaluated by real-time quantitative PCR amplification, for each patient at time points corresponding to those in panel B, before and after AP1903 infusion.

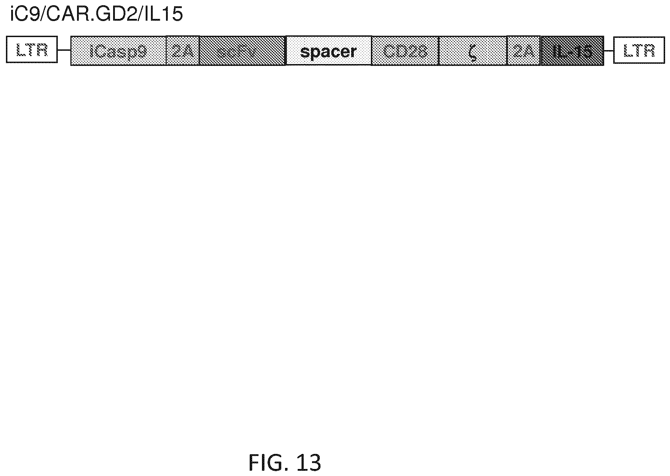

Figure 13. Construction of tricistronic retroviral vector expressing iCasp9/CAR.GD2/IL-15. The indicated genes were linked together using 2A sequence peptides derived from foot-and-mouth disease virus, and cloned into the SFG retroviral vector to generate the CAR.GD2 coexpressed with IL-15 and the inducible suicide gene caspase-9 (iCasp9/CAR.GD2-CD28/IL-15) in one retroviral vector.

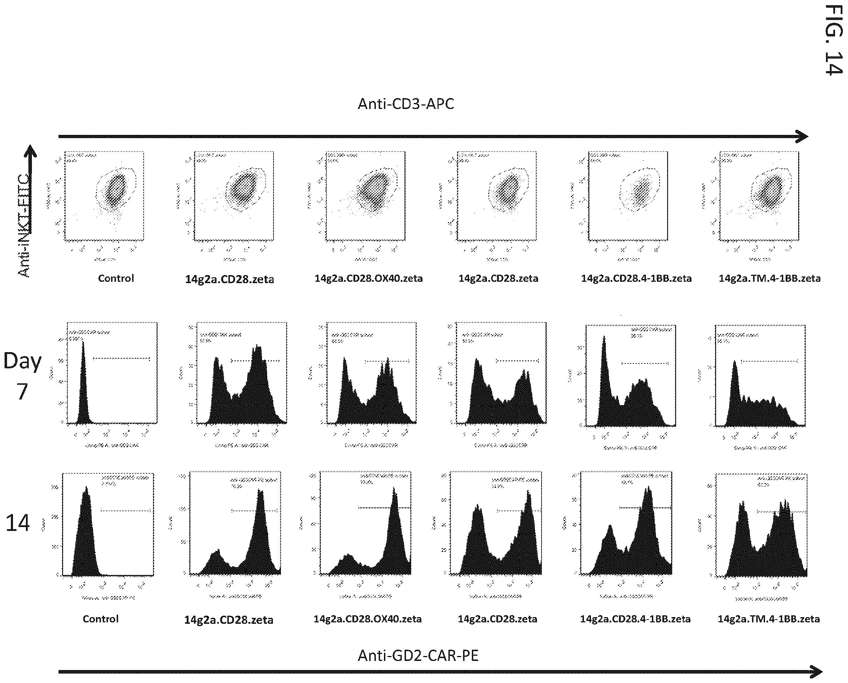

Figure 14 shows effective generation and expansion of CAR.GD2 NKTs.

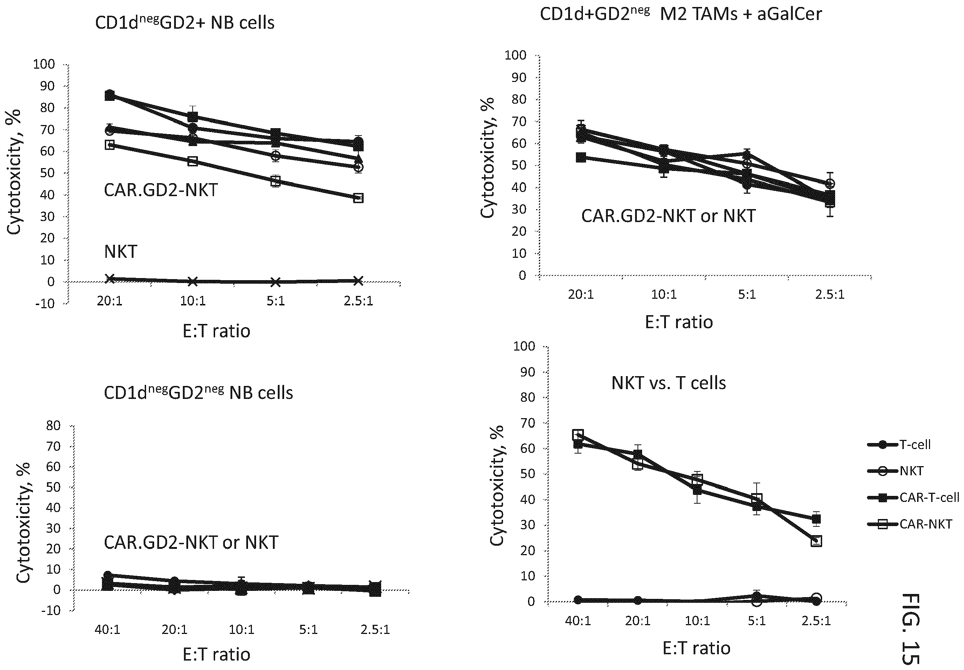

Figure 15 shows CAR.GD2 NKTs are endowed with dual specificity against GD2+ and CD1d+ targets.

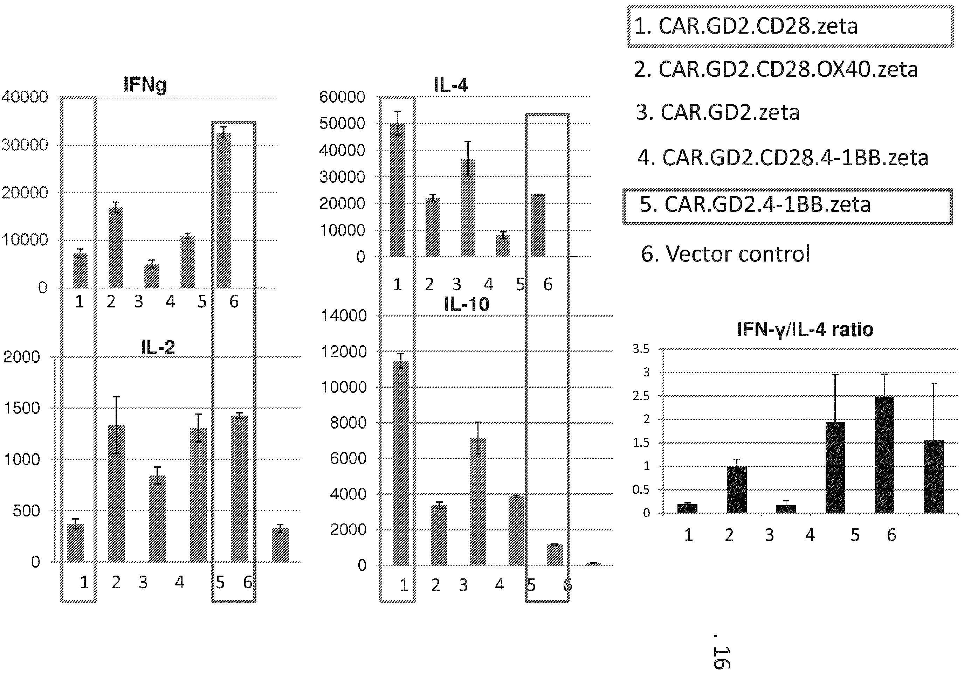

Figure 16 demonstrates co-stimulatory endodomains in CAR.GD2 constructs affect NKT-cell cytokine profile.

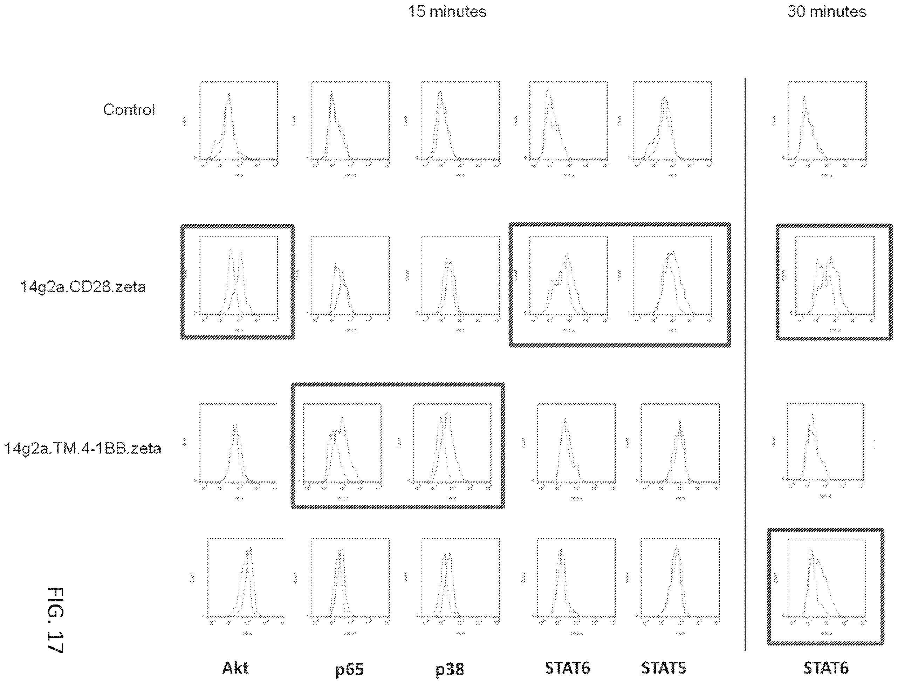

Figure 17 shows co-stimulatory endodomains in CAR.GD2 constructs affect Th1 and Th2 signaling pathways in NKTs.

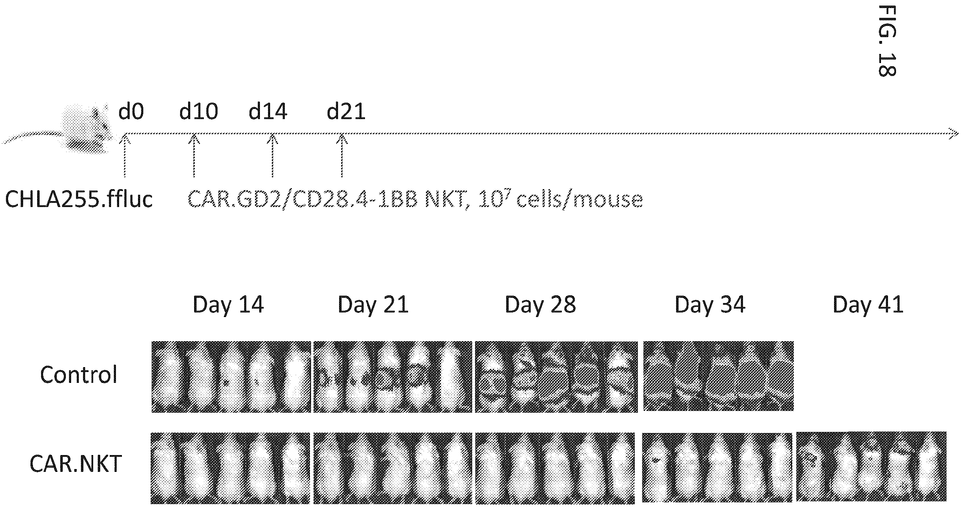

Figure 18 shows therapeutic efficacy of CAR.GD2 NKTs against NB mets in hu-NSG mice.

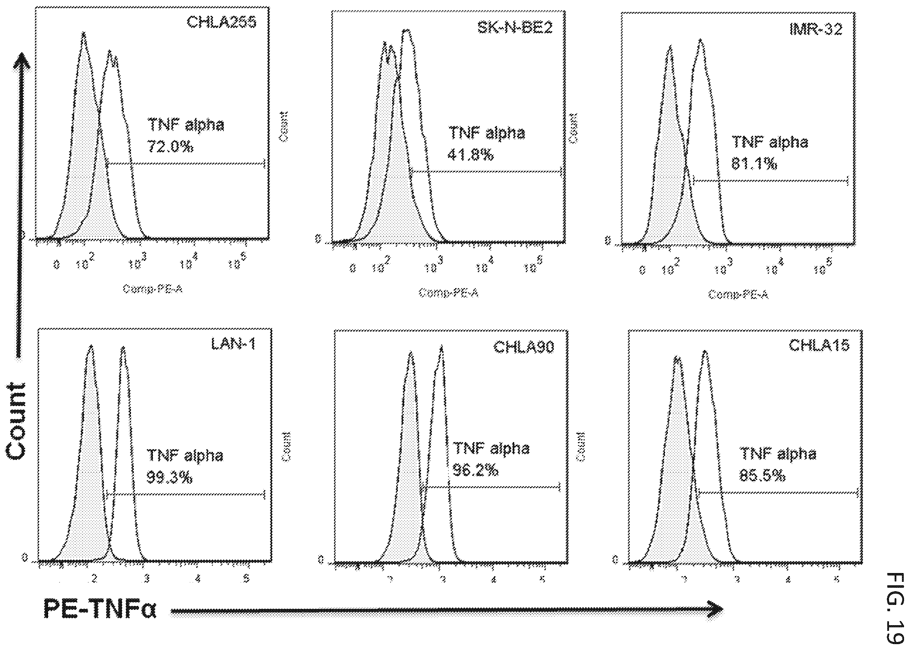

Figure 19 shows intracellular TNFα expression in NB cell lines. Cultured cells from the indicated human NB cell lines were fixed and permeabilized followed by intracellular staining with PE-conjugated anti-TNFa (transparent) or isotype control (grey) mAbs. Shown are representative FACS plots from one of three independent experiments.

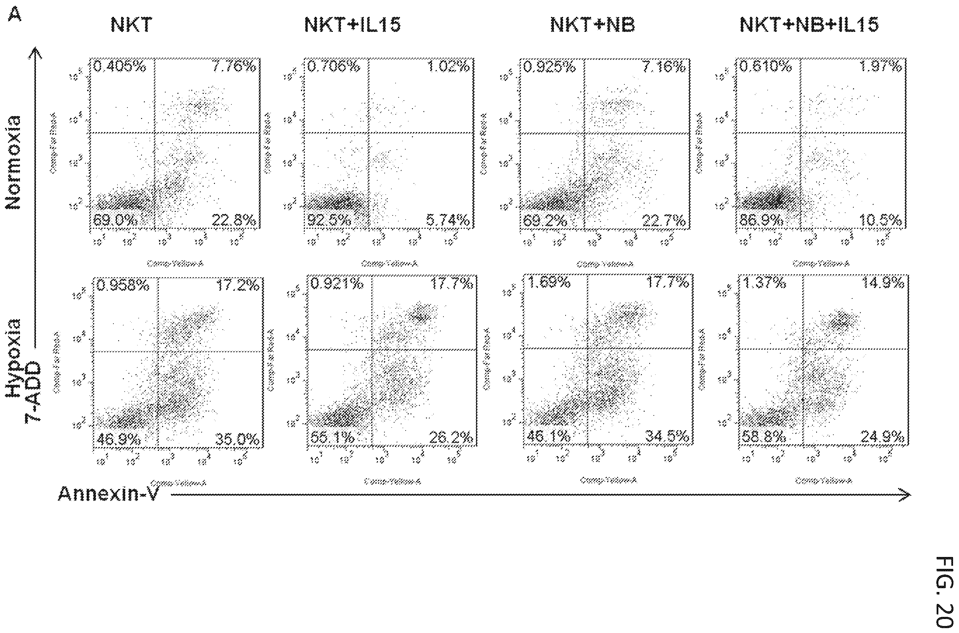

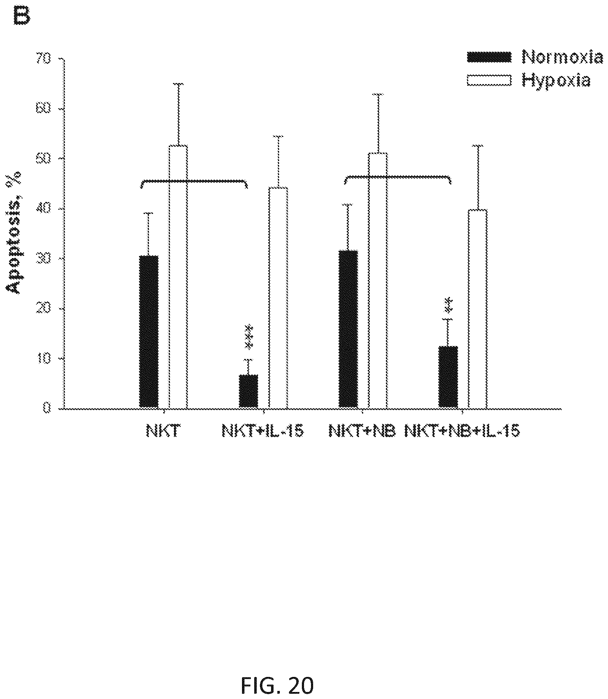

Figure 20 shows differential effect of IL-15 on NKT-cell apoptosis in normoxia and hypoxia. (A) NKTs were expanded from PBMC using stimulation with aGalCer and IL-2 for 7 days, then washed and cultured in the absence or presence of NB cells (1:1 ratio) in normoxia or hypoxia, with or without IL-15 at 10 ng/ml for 24 h followed by staining for Annexin-V and 7-AAD. Shown are representative FACS plots from one of four independent experiments. (B) Mean ± SD from 4 experiments, **P<0.01 ***P<0.001.

### DETAILED DESCRIPTION OF THE INVENTION

### I. Definitions

In keeping with long-standing patent law convention, the words "a" and "an" when used in the present specification in concert with the word comprising, including the claims, denote "one or more." Some embodiments of the invention may consist of or consist essentially of one or more elements, method steps, and/or methods of the invention. It is contemplated that any method or composition described herein can be implemented with respect to any other method or composition described herein.

The term "therapeutically effective amount" as used herein refers to that amount which, when administered to a subject or patient for treating a disease, is sufficient to effect such treatment for the disease, including to ameliorate at least one symptom of the disease.

### II. Certain Embodiments of the Invention

Tumor-infiltrating Natural Killer T cells (NKTs) associate with good outcomes in diverse cancers. However, the mechanisms by which NKTs interact with tumor microenvironments have remained enigmatic, precluding rational design of NKT-based immunotherapies. NKTs co-localize with tumor-associated macrophages in a newly identified innate response to tumor-induced hypoxia. In embodiments of the invention there is engineering of NKTs. In specific embodiments, NKTs are engineered for purposes of enhancing their growth under hypoxic conditions and/or facilitating their antitumor activity.

Hypoxia is a fundamental feature of malignant tumors such as neuroblastoma that reduces the effectiveness of conventional therapeutic agents. The inventors have identified a novel innate immune response to tumor-induced hypoxia, and in embodiments of the invention there is manipulation of the effector cells of this response to provide cancer immunotherapy. The invention provides critical insights into the mechanisms by which NKT lymphocytes infiltrate tumors and interact with the hypoxic tumor microenvironment. One can apply innovative cell engineering technology to selectively target hypoxic tissues inside neuroblastoma (for example) for immunotherapy with NKTs. These engineered cells can be combined with other forms of immunotherapy, such as tumor-specific T cells, for example. Hypoxia-targeting NKT-cell therapy is broadly applicable in diverse types of cancer in children and adults.

Thus, based on expertise with 1) human NKTs, 2) fusion constructs with an oxygen-dependent degradation domain, and 3) chimeric antigen receptors (CAR) for T-cell therapy, one can 1) discover the mechanism of NKT-cell localization to hypoxic tumors; 2) engineer NKTs, for example to express certain compounds in an oxygen-dependent manner to improve their survival and antitumor activity; and 3) evaluate combined immunotherapy with NKTs and tumor-specific CAR-T cells.

Embodiments of the present invention encompass targeting tumor microenvironment and/or targeting tumor cells by NKT cells. In some embodiments, the NKT cells are engineered to encompass a chimeric antigen receptor, and in some embodiments the NKT cells are engineered to express IIL-15, IL-2, IL-4, and/or IL-7.

In embodiments of the invention, there is effective immunotherapy of cancer using NKTs that increase their ability to attack both tumor cells and to attack the tissues that support their growth. NKTs localize to the tumor site in neuroblastoma patients, for example, and attack non-malignant cells called tumor-associated macrophages, which provide critical support for the survival and growth of the tumor cells. To work well, NKTs must survive in the hostile environment; upon genetic engineering with a survival factor such as IL-15 and/or with a cancer -targeting molecule called CAR.GD2, NKTs will survive at the tumor site and have antitumor efficacy both by killing the cancer directly and by disrupting the supporting environment. As proof of principle, NKTs from neuroblastoma patients were engineered so that they can make IL-15 and have on their surface new components (CAR.GD2) that can recognize the tumor cells directly. The anti-tumor use of these engineered NKTs is characterized using *in vitro* and *in vivo* experimental systems. One can utilize the gene-modified NKTs in neuroblastoma patients.

In embodiments of the invention, there are genetically modified human NKTs and a novel immunotherapeutic strategy of employing them. In specific embodiments, TAM-targeting NKTs are enabled with an additional specificity against the neuroblasts using CAR.GD2, which itself has shown to be effective in patients with recurrent NB when expressed by cytotoxic T cells. The expression of IL-15 in NKTs facilitates NKT-cell survival and antitumor activity in cancer patients. Moreover, in some embodiments there is a suicide switch in vectors in the NKTs to ensure the safety of the subsequent clinical use of the gene-modified NKTs. A unique model of NB in humanized mice may be utilized that allows evaluation of tumor localization of human NKTs and their interaction not only with human NB cells but also with human TAMs in the tumor tissues. The selected CAR.GD2 construct can be manufactured as clinical grade material, in certain aspects. Because of their inherent ability to target tumor-supportive stroma, CAR NKTs in some embodiments are more effective than CAR T cells in many adult and pediatric malignancies. Hence, a NKT cell-based therapeutic platform allows a major effect on cancer cell therapy.

In embodiments of the invention, there is an effective immunotherapy of cancer using natural and engineered properties of Vα24-invariant CD1d-reactive Natural Killer T cells (NKTs) to target both tumor-supportive stromal cells and tumor cells themselves. Tumor-infiltrating NKTs are associated with good outcomes in diverse cancers. Recent findings suggest that instead of attacking tumor cells directly, NKTs target CD1d-positive tumor-associated macrophages (TAMs), which provide essential stromal support for tumor cells. NKTs are attracted to and inhibited by hypoxic TAMs in neuroblastoma (NB) and their anti-metastatic activity can be rescued by transgenic expression of IL-15. However, in addition to attacking tumor-supportive stroma, the curative therapy may also require direct and specific attack against the tumor cells. Thus, in embodiments of the invention there are transduced human NKTs with a chimeric antigen receptor (CAR) that targets the GD2 antigen expressed by the neuroblasts (CAR.GD2) and represents a clinically validated therapeutic target in NB. Transgenic expression of CAR.GD2 renders NKTs highly cytotoxic against neuroblasts while retaining native TCR specificity and the ability to kill TAMs. Based on the recent clinical successes with CAR T cells, new CAR.GD2 constructs were generated that encode co-stimulatory endodomains (CD28, OX40, or CD137) with or without IL-15. To ensure the safety and clinical applicability of the gene modification, embodiments of the invention encompass transgenic expression of CAR.GD2 and IL-15 with the expression of the exemplary inducible caspase-9, forming a suicide switch. Therefore, embodiments of the invention include a bi/tricistronic vector encoding CAR.GD2 containing a co-stimulatory endodomain and/or IL-15 coupled with the inducible suicide switch, as such NKTs show safely enhanced survival and anti-tumor effector functions within the NB tumor environment. Encompassed in the invention are models of orthotopic and metastatic NB in humanized mice that allow *in vivo* testing of the dual specific NKT-cell activity against human NB grafts containing human TAMs.

### III. Embodiments of Chimeric Receptors of the Invention and Uses Thereof

In some embodiments, NKT cells are engineered to comprise an expression construct that encodes IL-15, IL-2, IL-4, and/or IL-7, and in specific embodiments the NKT cells alternatively or in addition comprise a chimeric antigen receptor.

In embodiments of the invention, there is utilized a chimeric antigen receptor (CAR) that is an engineered fusion of single -chain variable fragments (scFv), derived from monoclonal antibodies, that are fused to CD3-zeta transmembrane domain (for example) and intracellular endodomain(s). In embodiments of the invention, there are chimeric receptors that target GD2. In specific cases there are expression constructs the encode such chimeric receptors. In some embodiments the expression constructs are present in a NKT cell.

In specific embodiments, the CAR molecules result in the transmission of a zeta signal in response to recognition by the scFv of its target. An example of such a target is the disialoganglioside GD2, for example. NKT cells may be transduced with an expression construct that encodes the CAR, and such transduction may be oncoretroviral vector transduction, for example. Use of the NKT cell then allows it to recognize and kill target cells that express GD2 (e.g. neuroblastoma cells).

Although in particular embodiments any suitable intracellular domain is employed in the chimeric receptors of the invention, in specific embodiments it is part or all of the zeta chain of CD3. In specific embodiments, intracellular receptor signaling domains are those of the T cell antigen receptor complex, such as the zeta chain of CD3, also Fcγ RIII costimulatory signaling domains, CD28, DAP10, CD2, alone or in a series with CD3zeta, for example. In specific embodiments, the intracellular domain (which may be referred to as the cytoplasmic domain) comprises part or all of one or more of TCR Zeta chain, CD28, OX40/CD134, 4-1BB/CD137, FcεRIγ, ICOS/CD278, ILRB/CD122, IL-2RG/CD132, and CD40. One or multiple cytoplasmic domains may be employed, as so-called third generation CARs have at least 2 or 3 signaling domains fused together for additive or synergistic effect, for example.

An immunoreceptor according to the present invention can be produced by any means known in the art, though preferably it is produced using recombinant DNA techniques. A nucleic acid sequence encoding the several regions of the chimeric receptor can prepared and assembled into a complete coding sequence by standard techniques of molecular cloning (genomic library screening, PCR, primer-assisted ligation, site-directed mutagenesis, *etc*.)*.* The resulting coding region is preferably inserted into an expression vector and used to transform a suitable expression host cell line, preferably NKT cells, and the NKT cells may be autologous, in certain aspects, although in other cases they may be allogeneic.

Suitable doses for a therapeutic effect would be between about 10⁶ and about 10⁹ cells per dose, preferably in a series of dosing cycles. A preferred dosing regimen consists of four one-week dosing cycles of escalating doses, starting at about 10⁷ cells on Day 0, increasing incrementally up to a target dose of about 10⁸ cells by Day 5. Suitable modes of administration include intravenous, subcutaneous, intracavitary (for example by reservoir-access device), intraperitoneal, and direct injection into a tumor mass.

As used herein, a nucleic acid construct or nucleic acid sequence is intended to mean a DNA molecule that can be transformed or introduced into a NKT cell and be transcribed and translated to produce a product (*e.g.,* a chimeric receptor).

In the nucleic acid construct employed in the present invention, the promoter is operably linked to the nucleic acid sequence encoding the chimeric receptor of the present invention, *i.e*., they are positioned so as to promote transcription of the messenger RNA from the DNA encoding the chimeric receptor. The promoter can be of genomic origin or synthetically generated. A variety of promoters for use in T cells are well-known in the art (for example, native LTR of gammaretroviral vector, PGK and EF1), and in some cases the promoters are inducible. The promoter can be constitutive or inducible, where induction is associated with the specific cell type or a specific level of maturation, for example. Alternatively, a number of well-known viral promoters are also suitable. Promoters of interest include the β-actin promoter, SV40 early and late promoters, immunoglobulin promoter, human cytomegalovirus promoter, retrovirus promoter, and the Friend spleen focus-forming virus promoter. The promoters may or may not be associated with enhancers, wherein the enhancers may be naturally associated with the particular promoter or associated with a different promoter.

The sequence of the open reading frame encoding the chimeric receptor can be obtained from a genomic DNA source, a cDNA source, or can be synthesized (*e.g.*, *via* PCR), or combinations thereof. Depending upon the size of the genomic DNA and the number of introns, it may be desirable to use cDNA or a combination thereof as it is found that introns stabilize the mRNA or provide NKT cell-specific expression. Also, it may be further advantageous to use endogenous or exogenous non-coding regions to stabilize the mRNA.

For expression of a chimeric receptor of the present invention, the naturally occurring or endogenous transcriptional initiation region of the nucleic acid sequence encoding N-terminal component of the chimeric receptor can be used to generate the chimeric receptor in the target host. Alternatively, an exogenous transcriptional initiation region can be used that allows for constitutive or inducible expression, wherein expression can be controlled depending upon the target host, the level of expression desired, the nature of the target host, and the like.

Likewise, a signal sequence directing the chimeric receptor to the surface membrane can be the endogenous signal sequence of N-terminal component of the chimeric receptor. Optionally, in some instances, it may be desirable to exchange this sequence for a different signal sequence. However, the signal sequence selected should be compatible with the secretory pathway of NKT cells so that the chimeric receptor is presented on the surface of the NKT cell.

Similarly, a termination region may be provided by the naturally occurring or endogenous transcriptional termination region of the nucleic acid sequence encoding the C-terminal component of the chimeric receptor. Alternatively, the termination region may be derived from a different source. For the most part, the source of the termination region is generally not considered to be critical to the expression of a recombinant protein and a wide variety of termination regions can be employed without adversely affecting expression.

As will be appreciated by one of skill in the art, in some instances, a few amino acids at the ends of the GD2 can be deleted, usually not more than 10, more usually not more than 5 residues, for example. Also, it may be desirable to introduce a small number of amino acids at the borders, usually not more than 10, more usually not more than 5 residues. The deletion or insertion of amino acids may be as a result of the needs of the construction, providing for convenient restriction sites, ease of manipulation, improvement in levels of expression, or the like. In addition, the substitute of one or more amino acids with a different amino acid can occur for similar reasons, usually not substituting more than about five amino acids in any one domain.

The chimeric construct that encodes the chimeric receptor according to the invention can be prepared in conventional ways. Because, for the most part, natural sequences may be employed, the natural genes may be isolated and manipulated, as appropriate, so as to allow for the proper joining of the various components. Thus, the nucleic acid sequences encoding for the N-terminal and C-terminal proteins of the chimeric receptor can be isolated by employing the polymerase chain reaction (PCR), using appropriate primers that result in deletion of the undesired portions of the gene. Alternatively, restriction digests of cloned genes can be used to generate the chimeric construct. In either case, the sequences can be selected to provide for restriction sites which are blunt-ended, or have complementary overlaps.

The various manipulations for preparing the chimeric construct can be carried out *in vitro* and in particular embodiments the chimeric construct is introduced into vectors for cloning and expression in an appropriate host using standard transformation or transfection methods. Thus, after each manipulation, the resulting construct from joining of the DNA sequences is cloned, the vector isolated, and the sequence screened to ensure that the sequence encodes the desired chimeric receptor. The sequence can be screened by restriction analysis, sequencing, or the like.

The chimeric constructs of the present invention find application in subjects having or suspected of having cancer by reducing the size of a tumor or preventing the growth or re-growth of a tumor in these subjects. Accordingly, embodiments the present invention further relates to a method for reducing growth or preventing tumor formation in a subject by introducing a chimeric construct of the present invention into an isolated NKT cell of the subject and reintroducing into the subject the transformed NKT cell, thereby effecting anti-tumor responses to reduce or eliminate tumors in the subject. As is well-known to one of skill in the art, various methods are readily available for isolating these cells from a subject, for example, using cell surface marker expression or using commercially available kits (*e.g*., ISOCELL™ from Pierce, Rockford, Ill.).

It is contemplated that the chimeric construct can be introduced into the subject's own NKT cells as naked DNA or in a suitable vector. Methods of stably transfecting T cells by electroporation using naked DNA are known in the art. See, *e.g.,* U.S. Pat. No. 6,410,319. Naked DNA generally refers to the DNA encoding a chimeric receptor of the present invention contained in a plasmid expression vector in proper orientation for expression. Advantageously, the use of naked DNA reduces the time required to produce T cells expressing the chimeric receptor of the present invention.

Alternatively, a viral vector (*e.g.,* a retroviral vector, adenoviral vector, adeno-associated viral vector, or lentiviral vector) can be used to introduce the chimeric construct into NKT cells. Suitable vectors for use in accordance with the method of the present invention are non-replicating in the subject's NKT cells. A large number of vectors are known that are based on viruses, where the copy number of the virus maintained in the cell is low enough to maintain the viability of the cell. Illustrative vectors include the pFB-neo vectors (STRATAGENE®) disclosed herein as well as vectors based on HIV, SV40, EBV, HSV or BPV.

Once it is established that the transfected or transduced NKT cell is capable of expressing the chimeric receptor as a surface membrane protein with the desired regulation and at a desired level, it can be determined whether the chimeric receptor is functional in the host cell to provide for the desired signal induction. Subsequently, the transduced NKT cells are reintroduced or administered to the subject to activate anti-tumor responses in the subject. To facilitate administration, the transduced NKT cells according to the invention can be made into a pharmaceutical composition or made implant appropriate for administration *in vivo,* with appropriate carriers or diluents, which further can be pharmaceutically acceptable. The means of making such a composition or an implant have been described in the art (see, for instance, Remington's Pharmaceutical Sciences, 16th Ed., Mack, ed. (1980)). Where appropriate, the transduced T cells can be formulated into a preparation in semisolid or liquid form, such as a capsule, solution, injection, inhalant, or aerosol, in the usual ways for their respective route of administration. Means known in the art can be utilized to prevent or minimize release and absorption of the composition until it reaches the target tissue or organ, or to ensure timed-release of the composition. Desirably, however, a pharmaceutically acceptable form is employed which does not ineffectuate the cells expressing the chimeric receptor. Thus, desirably the transduced T cells can be made into a pharmaceutical composition containing a balanced salt solution, preferably Hanks' balanced salt solution, or normal saline.

A pharmaceutical composition of the present invention can be used alone or in combination with other well-established agents useful for treating cancer. Whether delivered alone or in combination with other agents, the pharmaceutical composition of the present invention can be delivered *via* various routes and to various sites in a mammalian, particularly human, body to achieve a particular effect. One skilled in the art will recognize that, although more than one route can be used for administration, a particular route can provide a more immediate and more effective reaction than another route. For example, intradermal delivery may be advantageously used over inhalation for the treatment of melanoma. Local or systemic delivery can be accomplished by administration comprising application or instillation of the formulation into body cavities, inhalation or insufflation of an aerosol, or by parenteral introduction, comprising intramuscular, intravenous, intraportal, intrahepatic, peritoneal, subcutaneous, or intradermal administration.

A composition of the present invention can be provided in unit dosage form wherein each dosage unit, *e.g.,* an injection, contains a predetermined amount of the composition, alone or in appropriate combination with other active agents. The term unit dosage form as used herein refers to physically discrete units suitable as unitary dosages for human and animal subjects, each unit containing a predetermined quantity of the composition of the present invention, alone or in combination with other active agents, calculated in an amount sufficient to produce the desired effect, in association with a pharmaceutically acceptable diluent, carrier, or vehicle, where appropriate. The specifications for the novel unit dosage forms of the present invention depend on the particular pharmacodynamics associated with the pharmaceutical composition in the particular subject.

Desirably an effective amount or sufficient number of the isolated transduced T cells is present in the composition and introduced into the subject such that long-term, specific, anti-tumor responses are established to reduce the size of a tumor or eliminate tumor growth or regrowth than would otherwise result in the absence of such treatment. Desirably, the amount of transduced T cells reintroduced into the subject causes a 10%, 20%, 30%, 40%, 50%, 60%, 70%, 80%, 90%, 95%, 98%, or 100% decrease in tumor size when compared to otherwise same conditions wherein the transduced T cells are not present.

Accordingly, the amount of transduced NKT cells administered should take into account the route of administration and should be such that a sufficient number of the transduced NKT cells will be introduced so as to achieve the desired therapeutic response. Furthermore, the amounts of each active agent included in the compositions described herein (*e.g.*, the amount per each cell to be contacted or the amount per certain body weight) can vary in different applications. In general, the concentration of transduced NKT cells desirably should be sufficient to provide in the subject being treated at least from about 1 × 10⁶ to about 1 × 10⁹ transduced NKT cells, even more desirably, from about 1 × 10⁷ to about 5 × 10⁸ transduced T cells, although any suitable amount can be utilized either above, *e.g.*, greater than 5 × 10⁸ cells, or below, *e.g.*, less than 1 × 10⁷ cells. The dosing schedule can be based on well-established cell-based therapies (see, *e.g.*, Topalian and Rosenberg (1987) Acta Haematol. 78 Suppl 1:75-6; U.S. Pat. No. 4,690,915) or an alternate continuous infusion strategy can be employed.

These values provide general guidance of the range of transduced NKT cells to be utilized by the practitioner upon optimizing the method of the present invention for practice of the invention. The recitation herein of such ranges by no means precludes the use of a higher or lower amount of a component, as might be warranted in a particular application. For example, the actual dose and schedule can vary depending on whether the compositions are administered in combination with other pharmaceutical compositions, or depending on interindividual differences in pharmacokinetics, drug disposition, and metabolism. One skilled in the art readily can make any necessary adjustments in accordance with the exigencies of the particular situation.

### IV. Combination Therapy

In certain embodiments of the invention, methods of the present invention for clinical aspects are combined with other agents effective in the treatment of hyperproliferative disease, such as anti-cancer agents. An "anti-cancer" agent is capable of negatively affecting cancer in a subject, for example, by killing cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer. More generally, these other compositions would be provided in a combined amount effective to kill or inhibit proliferation of the cell. This process may involve contacting the cancer cells with the expression construct and the agent(s) or multiple factor(s) at the same time. This may be achieved by contacting the cell with a single composition or pharmacological formulation that includes both agents, or by contacting the cell with two distinct compositions or formulations, at the same time, wherein one composition includes the expression construct and the other includes the second agent(s).

Tumor cell resistance to chemotherapy and radiotherapy agents represents a major problem in clinical oncology. One goal of current cancer research is to find ways to improve the efficacy of chemo- and radiotherapy by combining it with cell therapy. In the context of the present invention, it is contemplated that cell therapy could be used similarly in conjunction with chemotherapeutic, radiotherapeutic, or immunotherapeutic intervention, for example.

The present inventive therapy may precede or follow the other agent treatment by intervals ranging from minutes to weeks. In embodiments where the other agent and present invention are applied separately to the individual, one would generally ensure that a significant period of time did not expire between the time of each delivery, such that the agent and inventive therapy would still be able to exert an advantageously combined effect on the cell. In such instances, it is contemplated that one may contact the cell with both modalities within about 12-24 h of each other and, more preferably, within about 6-12 h of each other. In some situations, it may be desirable to extend the time period for treatment significantly, however, where several d (2, 3, 4, 5, 6 or 7) to several wk (1, 2, 3, 4, 5, 6, 7 or 8) lapse between the respective administrations.

Various combinations may be employed, present invention is "A" and the secondary agent, such as radio- or chemotherapy, is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

It is expected that the treatment cycles would be repeated as necessary. It also is contemplated that various standard therapies, as well as surgical intervention, may be applied in combination with the inventive cell therapy.

### A. Chemotherapy

In some embodiments, the invention is employed with chemotherapy. Although the chemotherapy may be of any kind, in specific embodiments the chemotherapy is useful for neuroblastoma. In specific embodiments, the chemotherapy comprises Adriamycin PFS (Doxorubicin Hydrochloride); Adriamycin RDF (Doxorubicin Hydrochloride); Clafen (Cyclophosphamide); Cyclophosphamide; Cytoxan (Cyclophosphamide); Doxorubicin Hydrochloride; Neosar (Cyclophosphamide); Vincasar PFS (Vincristine Sulfate); and/or Vincristine Sulfates.

Cancer therapies also include a variety of combination therapies with both chemical and radiation-based treatments. Combination chemotherapies include, for example, abraxane, altretamine, docetaxel, herceptin, methotrexate, novantrone, zoladex, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, farnesyl-protein tansferase inhibitors, transplatinum, 5-fluorouracil, vincristin, vinblastin and methotrexate, or any analog or derivative variant of the foregoing.

### B. Radiotherapy

Other factors that cause DNA damage and have been used extensively include what are commonly known as □-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Immunotherapy

Immunotherapeutics generally rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionuclide, ricin A chain, cholera toxin, pertussis toxin, *etc.*) and serve merely as a targeting agent. Alternatively, the effector may be a lymphocyte carrying a surface molecule that interacts, either directly or indirectly, with a tumor cell target. Various effector cells include cytotoxic T cells and NK cells.

Immunotherapy could thus be used as part of a combined therapy, in conjunction with the present cell therapy. The general approach for combined therapy is discussed below. Generally, the tumor cell must bear some marker that is amenable to targeting, *i.e.,* is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155.

### D. Genes

In yet another embodiment, the secondary treatment is a gene therapy in which a therapeutic polynucleotide is administered before, after, or at the same time as the present invention clinical embodiments. A variety of expression products are encompassed within the invention, including inducers of cellular proliferation, inhibitors of cellular proliferation, or regulators of programmed cell death.

### E. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment of the present invention, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present invention may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### F. Other agents

It is contemplated that other agents may be used in combination with the present invention to improve the therapeutic efficacy of treatment. These additional agents include immunomodulatory agents, agents that affect the upregulation of cell surface receptors and GAP junctions, cytostatic and differentiation agents, inhibitors of cell adhesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotic inducing abililties of the present invention by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with the present invention to improve the anti-hyerproliferative efficacy of the treatments. Inhibitors of cell adhesion are contemplated to improve the efficacy of the present invention. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with the present invention to improve the treatment efficacy.

### V. Embodiments of Kits of the Invention

Any of the compositions described herein may be comprised in a kit. In a non-limiting example, an expression construct, one or more reagents to generate an expression construct, cells for transfection of the expression construct, and/or one or more instruments to obtain autologous cells for transfection of the expression construct (such an instrument may be a syringe, pipette, forceps, and/or any such medically approved apparatus) may be provided in the kit. In some embodiments the kit also comprises one or more chemotherapeutic agents, including one or more chemotherapeutic agents for neuroblastoma, for example.

The kits may comprise one or more suitably aliquoted compositions of the present invention or reagents to generate compositions of the invention. The components of the kits may be packaged either in aqueous media or in lyophilized form. The container means of the kits may include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present invention also will typically include a means for containing the chimeric receptor construct and any other reagent containers in close confinement for commercial sale. Such containers may include injection or blow molded plastic containers into which the desired vials are retained, for example.

### EXAMPLES

The following examples are presented in order to more fully illustrate the preferred embodiments of the invention. They should in no way, however, be construed as limiting the broad scope of the invention.

### EXAMPLE 1

### TUMOR-ASSOCIATED MACROPHAGES SUFFOCATE NKT CELLS: AN IMMUNE ESCAPE MECHANISM AND A TARGET FOR THERAPY

Vα24-invariant Natural Killer T cells (NKTs) inhibit tumor growth *via* targeting tumor-associated macrophages (TAMs). Tumor progression therefore requires that TAMs evade NKT-cell activity *via* yet unknown mechanism. In embodiments, there is a subset of cells in neuroblastoma (NB) cell lines and primary tumors express membrane-bound (mb)TNFα. These pro-inflammatory tumor cells induce production of the chemokine CCL20 from TAMs *via* activation of the NF-kB signaling pathway, an effect that is amplified in hypoxia. Flow cytometry analyses of human primary NB tumors revealed selective accumulation of CCL20 in TAMs. Neutralization of the chemokine inhibited *in vitro* migration of NKTs toward tumor-conditioned hypoxic monocytes and *in vivo* localization to NB grafts in humanized NOD/SCID/IL2rgamma(null) (hu-NSG) mice. Hypoxia impairs NKT-cell viability and function so that NKT-cell trafficking toward CCL20-producing TAMs serves as a hypoxic trap for tumor-infiltrating NKTs. IL-15 protected NKTs from hypoxia, and transgenic expression of IL-15 in adoptively transferred NKTs dramatically enhanced their anti-metastatic activity compared with parental NKTs in the hu-NSG model. Thus, tumor-induced CCL20 production in hypoxic TAMs and consequent chemoattraction and inhibition of NKTs represents a novel mechanism of immune escape that can be reversed by adoptive immunotherapy with IL-15-transduced NKTs.

### EXAMPLE 2

### CONTACT WITH NB CELLS AND HYPOXIA SYNERGISTICALLY INDUCE CCL20 IN HUMAN MONOCYTES

To explain the observed co-localization of NKTs with TAMs in primary human NB (Song *et al.*, 2009), it was considered that TAMs upon the influence of tumor cells and/or hypoxic environment actively chemoattract NKTs. To further characterize this, the inventors performed an *in vitro* migration experiment using dual-chamber wells separated by 5 µM pore membrane. Human *ex vivo* expanded NKTs were added to the upper chambers and allowed to migrate for 3 h into the lower wells, which contained CHLA-255 NB cells, freshly isolated (negative selection) human monocytes from peripheral blood, or 1:1 mixture of NB cells and monocytes. Before adding NKTs, the plates with NB cells and monocytes were incubated in normoxic (20% O₂) or hypoxic (1% O₂) conditions for 48 h. Consistent with previous observations, NB cells were chemoattractive for NKTs in normoxic conditions (Metelitsa *et al.*, 2004). Surprisingly, NKTcell migration to NB cells was nearly abrogated in hypoxia (Fig. 1A). In contrast, the rate of NKT-cell migration toward the co-culture of NB cells with monocytes nearly doubled under hypoxic conditions (P < 0.001) while monocytes alone had little chemoattractive activity in either normoxia or hypoxia. These data suggest that an interaction between NB cells and monocytes under hypoxia results in induction (up-regulation) of chemokine(s) that chemoattract NKTs.

To examine the overall effect of hypoxia on the chemokine gene expression profile of NB cells and monocytes, we incubated monocytes and CHLA-255 NB cells in normoxic or hypoxic conditions for 36 h followed by RNA isolation and quantitative RT-PCR for 11 CC and CXC chemokine genes that are known to have corresponding receptors on human NKTs (Metelitsa *et al.*, 2004; Kim *et al.*, 2002; Kim *et al.*, 2002; Thomas *et al.*, 2003; Song *et al.*, 2007). Fig. 1B demonstrates that mRNA expression of CCL20, CCL5, CCL4, and CCL3 was up-regulated while that of CCL2 was downregulated in hypoxia compared with normoxia (P<0.001). Unlike other chemokines, CCL2 is expressed at high levels in NB cells and additional experiments with NB cells alone confirmed that hypoxia downregulates CCL2 expression in NB cells both at RNA and protein levels. This finding explains the observed loss of NB-cell chemoattraction for NKTs in hypoxia. To examine whether the up-regulation of mRNA expression of CCL20, CCL5, CCL4, and CCL3 results in the increased production of the corresponding proteins, supernatants were analyzed from the same experimental conditions by ELISA and it was found that only CCL20 production was significantly up-regulated in the co-culture of monocytes with NB cells compared with monocytes alone (NB cells do not produce detectable CCL20). Moreover, the effect of the co-culture on CCL20 up-regulation was amplified up to 70 fold in hypoxic compared with normoxic conditions (P < 0.001, Fig. 1C). Despite the observed variability in the magnitude of CCL20 production by monocytes from 13 different individuals, hypoxia invariably increased it in all cases. The kinetics analysis demonstrated that up-regulation of CCL20 reached maximum after 36 h of co-culture in normoxia, but continued to rise for at least 48 h in hypoxia (Fig.7A).

To unambiguously determine the cellular source of CCL20, we cultured monocytes alone or with NB cells in normoxic or hypoxic conditions and analyzed intracellular CCL20 accumulation by FACS using surface staining for CD14 to discriminate monocytes from NB cells. Fig. 1D demonstrates that either contact with NB cells or hypoxia alone could up-regulate CCL20 production in monocytes. The highest level of CCL20 expression was achieved when monocytes were co-cultured with NB cells in hypoxia that is consistent with the ELISA results in Fig. 1C. NB cells did not express detectable CCL20 in any tested condition. To examine the requirement of cell-cell contact between monocytes and NB cells for CCL20 induction in monocytes, NB cells were cultured with monocytes in the same wells or in the dual-chamber wells, separated by a 400 nM semi-permeable membrane. Monocytes failed to produce CCL20 in the absence of a direct contact with NB cells (Fig. 7C). To determine whether the induction of CCL20 in monocytes that were observed in the described *in vitro* experimental system occurs at the tumor site in NB patients, FACS was performed on cell suspensions prepared from freshly resected primary NB tumors at diagnosis. All tumor-derived monocytic cells expressed CCL20 while tumor cells and the majority of non-monocytic CD45+ tumor-infiltrating leukocytes (TILs) were negative (Fig. 1E). Therefore, TAMs in primary NB tumors produce CCL20, which expression is selectively induced in monocytic cells upon direct contact with NB cells and enhanced by hypoxia.

### EXAMPLE 3

### CCL20 IS REQUIRED FOR NKT-CELL MIGRATION TOWARD HYPOXIC NB/MONOCYTE CULTURE AND NB XENOGRAFTS IN HU-NSG MICE

CCL20 has been reported to be one of the most potent chemokines for human NKTs (Kim *et al.*, 2002; Thomas *et al.*, 2003). The analysis confirms that the majority of primary NKTs from peripheral blood express CCR6, the only receptor for CCL20. Moreover, CCR6 expression is preserved in *ex vivo* expanded NKTs (Fig. 8A). To determine the requirement of CCL20/CCR6 axis for the observed enhanced migration of NKTs toward the coculture of NB cells with monocytes in hypoxia (Fig. 1A), the *in vitro* migration study was repeated in the presence of chemokine-neutralizing mAbs. Consistent with previous reports, anti-CCL2 mAb effectively inhibited NKT-cell migration to NB or NB+monocytes co-culture under normoxia (Metelitsa *et al.*, 2004), but not under hypoxia. Only anti-CCL20 neutralizing mAb strongly inhibited NKT-cell migration in hypoxia (Fig. 2A).

To examine the relative contribution of CCL2 and CCL20 to the mechanism of NKT-cell *in vivo* localization to the tumor site, the inventors adapted a previously described CHLA-255/luc human NB model in immunodeficient mice (Song *et al.*, 2009) and instead of NOD/SCID, used hu-NSG mice (Fig. 9A). As it has been observed by others (Yahata *et al.*, 2002; Giassi *et al.*, 2008), hu-NSG mice had stable reconstitution of human myelomonocytic cells as well as B and T lymphocytes three months after transplantation with human cord blood CD34+ hematopoietic stem cells (SCT, Fig. 9B,C). CHLA-255/luc NB cells were injected under the renal capsule three and half months after SCT. Like in human NB tissues (Song *et al.*, 2009), TAMs represented a major subset of tumor-infiltrating leukocytes (Fig. 9D,E) and were enriched in a subset with M2 phenotype as determined by the downregulation of HLA-DR expression compared with CD14+ peripheral blood monocytes in the same mice (Fig. 9F). Importantly, similar HLA-DR'ow CD14+ cells were the dominant subset of TAMs in primary tumors from NB patients (Fig. 9G). Three weeks after NB-cell injection and clear evidence of tumor growth by bioluminescent imaging, mice were injected with human ex-vivo expanded NKTs and divided into groups to receive anti-human CCL2, anti-human CCL20 neutralizing mAb or isotype control mAb. A control group did not receive NKTs. On day 3 after NKT-cell transfer, mice were sacrificed and examined for NKT-ceillocalization to the tumor tissues. Figs. 2B,C demonstrate that animals, treated with antiCCL2 or anti-CCL20 mAb, had lower frequency of tumor-infiltrating NKTs among the tumor-infiltrating hCD45+ leukocytes compared with the IgG control group (25.9 ± 12.6% or 44.9 ± 6.3% vs. 74.3 ± 9.7%, respectively, P < 0.01, one-way ANOVA). While confirming the previously established role of NB-derived CCL2, these data establish the requirement of CCL20 for NKT-ceillocalization to the tumor site even though the latter chemokine is not produced by tumor cells, but induced in TAMs. Thus, NKTs effectively localize to NB tumors in hu-NSG mice and migrate toward TAMs in a CCL20-dependent manner.

### EXAMPLE 4

### MBTNFA ON NB CELLS INDUCES NF-KB ACTIVATION IN MONOCYTES THAT RESULTS IN CCL20 UP-REGULATION

The observed requirement for a cell-cell contact between NB cells and monocytes for CCL20 induction in monocytes and the known requirement of NF-kB activation for CCL20 expression (Battaglia *et al.*, 2008) prompted a search for candidate cell surface molecules on NB cells with pro-inflammatory properties. E. Goillot et al observed expression of TNFα protein in two NB cell lines by immunohistochemistry (Goillot *et al.*, 1992). The inventors have examined 3 MYCNamplified (SK-N-BE2, IMR32, LA-N1) and 3 MYCN-non-amplified (CHLA-255, CHLA-15, LA-N-2) NB cell lines by FACS and found that the majority of cells in all lines express TNFα intracellular (Fig. 19) as well as on the cell surface as a membrane-bound (mb) cytokine (Fig. 3A). No soluble TNFα has been detected by ELISA in the supernatants of all examined cell lines. Importantly, the presence of mbTNFα-positive subset in all seven examined primary NB tumors with the frequency ranging from 1.1% to 38.2% (12.2 ± 14%, Fig. 3B). The level of MYCN expression did not correlate with the frequency of mbTNFα-positive cells either in cell lines or primary tumors. The function blocking experiments demonstrated that a pre-incubation of NB cells with an anti-TNFa blocking mAb significantly inhibited their ability to induce CCL20 production in monocytes under both normoxic and hypoxic conditions (P < 0.001, Fig. 3C). Neither the frequency of TNFα -positive cells nor the level of TNFα expression in NB cells was affected by hypoxia. To examine the requirement of mbTNFα for the activation of NF-kB signaling in monocytes, monocytes were cultured alone (in non-adherent plates) or on the monolayer of NB cells (monocytes only loosely adhere to NB cell monolayer) in the presence of an isotype control or anti-TNFα blocking mAb in normoxic or hypoxic conditions. Fig. 3D demonstrates that IkBa, an IkB inhibitor, is phosphorylated (a required upstream event in NF-kB activation by extracellular stimuli) in monocytes upon the contact with NB cells and the IkBa phosphorylation was almost completely prevented in the presence of anti-TNFa blocking mAb. The hypoxic condition enhanced IkBα phosphorylation and this was also strongly inhibited by the anti-TNFα blocking mAb. Since contaminating NB cells could not be excluded as a source of phospho-lkBα in the above described monocyte preparations, we repeated this experiment and performed intracellular FACS analysis of IkBa and phosphorylated p65. After gating on CD5610w cells (monocytes), there was a decrease of IkBα expression (degradation upon phosphorylation) within 30 min after contact with NB cells, and the effect was abrogated in the presence of anti-TNFa blocking mAb (Fig. 3E). Consistent with the decrease of IkBα expression, there was an increase of phospho-p65 expression in monocytes and this was inhibited by antiTNFα blocking mAb in normoxic (Fig. 3F) and hypoxic conditions. Co-culture of monocytes with NB cells under hypoxia resulted in higher levels and more sustained p65 expression compared with normoxia (Fig. 3G). Therefore, NB contains a previously unknown subset of pro-inflammatory tumor cells that expresss mbTNFα, which is at least in part required for the induction of CCL20 production in TAMs *via* activation of NF-kB signaling, which is stabilized and enhanced by tumor-induced hypoxia.

NKT cells preferentially localize to hypoxic areas within tumor tissues. To examine the relative distribution of NKTs in hypoxic and normoxic areas of the tumor, we used a metastatic model of NB in hu-NSG mice. In this model, CHLA-255/luc cells were injected intravenously to produce metastatic growth in liver and bone/bone marrow (Fig. 6A), which are also the major metastatic sites in NB patients (Seeger *et al.*, 1996). On day 21, tumor-bearing mice were injected with CFSE-labeled NKTs and their localization in liver metastases was examined 3 days later. The areas of hypoxia in both primary and metastatic sites were visualized using intravital injection of EF5 followed by staining with anti-EF5 fluorochrom-conjugated mAb (Gacciabene *et al.*, 2011). The same tissues were co-stained with anti-CD11b mAb so that distribution of both NKTs and myeloid cells in normoxic and hypoxic tumor tissues was analyzed and quantified using 4-color confocal microscopy. In contrast to normal liver tissues in tumor-free hu-NSG mice, in which no staining for hypoxia was detected, metastatic tissues contained both normoxic and hypoxic areas, and more than 90% of NKTs and myelomonocytic cells were found in the latter. The quantitative analysis demonstrated that frequencies of NKT and CD11b+ cells per 1000 cells were 14.8 ± 6.3 and 20.8 ± 8.9 vs. 1.3 ± 1.6 and 1.1 ± 1.2 in hypoxic vs. normoxic areas, respectively (P<0.001). Therefore, NKTs co-localize with TAMs in the hypoxic tumor tissues.

### EXAMPLE 5

### NKT CELLS ARE INHIBITED BY CONTACT WITH NB CELLS AND HYPOXIA

Since NKT-cell mediated killing or inhibition of TAMs is important for their anti-tumor activity against CD1 d-negative tumors (Song *et al.*, 2009), it was considered how the hypoxic tumor environment that is at least in part responsible for NKT-cell trafficking toward TAMs affects their viability and function. NKTs expanded from PBMC of four donors using antigenic stimulation with aGalCer_were cultured in normoxic and hypoxic conditions for 24 h and NKT-cell viability was examined by trypan blue exclusion at different time intervals. In the absence of exogenous cytokines NKT-cell viability was maintained for 24 hr in normoxia, but more than 50% of cells died in hypoxia over the same period. Fig. 4A demonstrates that IL-2 and other cytokines which shared common gamma chain (IL-15, IL-4, IL-7) except IL-21 significantly improved NKT-cell survival in hypoxia (P < 0.01). While both cytokines significantly reduced the rate of NKT-cell apoptosis in normoxia, neither IL-2 nor IL-15 significantly protected NKTs from apoptosis in hypoxia conditions (P > 0.05) as mesured by Annexin-V/7-AAD staining (Fig. 20), suggesting that the observed effect on the absolute number of viable cells was mostly due to cytokine-supported NKT-cell proliferation as it is shown for IL-15-transduced NKTs in Fig. 6B and is consistent with the metabolic switch of prolifereating lymphocytes to glycolysis with reduced dependence on oxygen (Roos and Loos, 1973; Krauss et al,. 2001; Frauwirth *et al.*, 2002; Jones and Thompson, 2007).

To examine the effect of hypoxia on the functional activity of NKTs, the inventors activated NKT-cell TCR by adding agonistic 6B11 mAb and measured cytokine production in cell supernatants by ELISA. Fig. 4B demonstrates that after 24 hr exposure to hypoxia, IFNγ production by NKTss fell to 31.9 ± 6.1% and 25.7 ± 2.7% of the amount produced in normoxia when NKTs were cultured alone or with NB cells, respectively (P < 0.001). To examine the potential of IL-2Ry family cytokines to protect NKT-cell function from hypoxia, the inventors added saturating concentrations of these cytokines to NKT-cell cultures. IL-2 and IL-15 but not other cytokines rescued the IFNγ response of NKTs to TCR stimulation in the absence or presence of NB cells. Therefore, CCL20-mediated chemoattraction of NKTs toward hypoxic TAMs leads to the inhibition of NKT-cell functional activity and tumor escape from NKT-cell control that could be reversed by IL-2 or IL-15, in certain embodiments of the invention.

Several recent reports demonstrated that NKTs play a key role in liver and kidney ischemia-reperfusion injury (Lappas *et al.*, 2006; Li et al,. 2007) and in the genesis of the vaso-occlusive crisis in sickle cell disease (Wallace *et al.*, 2009; Wallace and Linden, 2010; Field *et al.*, 2011). The acute ischemia and inflammation in these conditions have been associated with spontaneous IFNγ production by NKTs both in mice and in humans. However, the direct effect of hypoxia on IFNγ expression in NKTs has not been evaluated. To examine the effect of hypoxia on spontaneous cytokine production by human NKTs, we cultured quiescent NKTs from four donors under normoxic or hypoxic conditions for different time intervals (2, 4, 6, 12, 24, and 48 hrs) and measured IFNγ and IL-4 production by ELISA. In the absence of antigenic stimulation, cytokines remained undetectable either in normoxia or hypoxia at any examined time interval, suggesting that hypoxia does not directly stimulate human NKTs.

### EXAMPLE 6

### IL-15 PROTECTS NKTS AND RESTORES THEIR ANTI-TUMOR POTENTIAL

IL-15 plays a critical role in NKT-cell development and homeostatic maintenance (30;31). The results of this study (Fig. 4B) suggest that IL-15 can also protect NKTs from the inhibitory effect of the hypoxic tumor microenvironment. Therefore, in embodiments of the invention transgenic expression of IL-15 in adoptively transferred NKTs would enhance their anti-tumor potential due to improved *in vivo* persistence and functionality at the tumor site. NKTs were transduced with a previously described retroviral construct, containing cDNA of human IL-15, inducible caspase-9 suicide gene (iCasp-9), and CD34 tag (Hsu *et al.*, 2007) to create NKTs/IL-15. Fig. 5A shows that ex-vivo expanded human NKTs could be stably transduced with the IL-15containing vector. IL-15 production by the transduced NKTs by ELISA. To examine the protective potential of transgenic IL-15 on NKT-cell function under hypoxia and in the presence of tumor cells, similar *in vitro* settings were used as described in Fig. 4B and measured TCR-induced NKT-cell proliferation using CFSE dilution assay. Consistent with the observed protective properties of the exogenous hrIL-15, NKTs/IL-15 had a significantly higher rate of proliferation upon hypoxia alone and in the presence of NB cells compared with parental NKTs (Fig. 5B, P < 0.01). The anti-apoptotic effect of transgenic IL-15 was significant only in normoxic conditions, as was the anti-apoptotic effect of exogenous IL15 (Fig. 20). The absolute cell count at the end of 5-day culture conclusively demonstrated that NKTs/IL-15 expanded significantly better than NKTs in all tested conditions (Fig. 6C). Therefore, NKTs engineered to express transgenic IL-15 are protected upon antigenic recognition in the hypoxic tumor microenvironment.

### EXAMPLE 7

### IL-15-TRANSDUCED NKTS HAVE POTENT ANTI-TUMOR ACTIVITY IN A METASTATIC NB MODEL IN HU-NSG MICE

To examine whether NKTs/IL-15 have a therapeutic advantage, a metastatic NB model in hu-NSG mice was utilized. Three and half months after SCT and upon confirmation of human hematopoietic reconstitution (Fig. 9B,C), mice were i/v injected with luciferase-transduced human NB cells, CHLA-255/luc. The therapeutic groups also received a single injection of either NKTs or NKTs/IL15. NSG mice that did not receive human CD34+ stem cells were used as a control group to assess the overall effect of human hematopoietic cells on the tumor growth. Fig. 6A demonstrates that metastatic growth in hu-NSG mice was dramatically enhanced compared with NSG mice, providing further support for the prominent role of BM-derived cells in enhancing NB growth. The immunotherapy with NKTs had significant but short-lived inhibitory effect on the metastatic growth. In contrast, a single injection of NKTs/IL-15 completely abrogated the tumor-promoting effect of the human hematopoietic environment (Fig. 6A,B). To determine whether the enhanced anti-tumor activity of NKTs/IL-15 remains CD1d-restricted, the inventors repeated the treatment of NB metastases in hu-NSG mice with NKTs/IL-15 after pre-treatment with anti-CD1d blocking or isotype control mAb. Fig. 6C demonstrates that anti-tumor efficacy of NKTs/IL-15 was inhibited by anti-CD1d mAb (P < 0.05), indicating that the effect of IL-15 at least in part depends on the function of CD1d-restricted NKTs although, due to incomplete inhibition, a contribution of NKT-independent effects of IL-15 such as activation of NK cells cannot be excluded. These data indicate that immunotherapy with NKTs/IL-15 can be effective in patients with metastatic NB and other types of cancer. This novel form of immunotherapy targeting tumor-supportive stroma may be combined with other forms of immunotherapy or chemotherapy that target tumor cells directly, in certain embodiments of the invention.

### EXAMPLE 8

### SIGNIFICANCE OF CERTAIN EMBODIMENTS

The subject matter herein reveals a novel mechanism of tumor escape from immune control by NKTs and provides the mechanistic insight into the development of NKT-cell based cancer immunotherapy. Because NKT-cell anti-tumor activity against CD1 d-negative tumors depends on their documented ability to co-localize and interact with CD1d+ TAMs (Song *et al.*, 2009), the elucidation of the mechanism by which NKTs traffic towards TAMs and understanding the effect of this process on NKT-cell function are useful for the rational design of NKT-cell based immunotherapy. Besides the previously described requirement for NB cell-derived CCL-2 (Metelitsa *et al.*, 2004), NKT-cell migration to the tumor site depends on CCL20, which is produced by TAMs inside the tumor tissues. CCL20 expression is induced in monocytic cells upon contact with NB cells and at least in part depends on mbTNFα, which is expressed on the surface of NB cells. Moreover, NB cell-induced CCL20 expression in monocytic cells is greatly amplified by hypoxia, which is known to attract TAMs (Mantovani *et al.*, 2008; Allavena *et al.*, 2008; Mantovani *et al.*, 2006). This indicates that a CCL-20 gradient directs NKT-cell trafficking toward hypoxic tumor tissues. Indeed, more than 90% of tumor-infiltrating NKTs are co-localized with macrophages in hypoxic areas of NB xenografts in hu-NSG mice. Hypoxia in turn inhibits NKT-cell ability to respond to an antigenic stimulation that explains how growing tumors can neutralize NKT-cell function and rescue tumor-supportive TAMs from the NKT-cell attack. Importantly, IL15 protects antigen-activated NKTs from hypoxia and NKTs/IL-15 have potent and long-lasting anti-tumor activity in a hu-NSG model of metastatic NB.

TAMs in primary NB tumors produce CCL20, expression of which is selectively induced in monocytic cells upon direct contact with NB cells and enhanced by hypoxia. Of interest, there is a differential requirement for CCL2 and CCL20 for NKT-cell *in vitro* migration toward a co-culture of NB cells with monocytes in normoxic and hypoxic conditions. While CCL2 was required for NKT-cell migration in normoxia, CL20 was largely responsible for their migration in hypoxia. The observed downregulation of CCL2 expression in NB cells under hypoxia combined with CCL20 induction in hypoxic monocytes suggests a two-step mode of NKT-cell migration in the tumor tissues: CCR2- or CCR4-mediated exit from circulation toward CCL2-producing NB cells in the oxygenated areas around blood vessels and then CCR6-mediated trafficking toward CCL20-producing TAMs in the hypoxic areas. The *in vivo* blocking experiments with anti-CCL20 neutralizing mAb further support the importance of CCL20 for NKT-cell localization to the tumor site. Therefore, in embodiments of the invention NKTs co-localize with TAMs as a part of a novel innate response to tumor-induced hypoxia. Such response could enable NKT cell-mediated targeting of TAMs at the early stages of tumor progression when TAMs playa major role in tumor vascularization and tumor cell survival (Mantovani *et al.*, 2008; Sica *et al.*, 2008; Sica and Bronte, 2007; Pietras *et al.*, 2009). Moreover, this mechanism is part of an evolutionary conserved role of NKTs in the negative regulation of chronic inflammation, in certain embodiments, and explain the paradoxical abilities of these cells to control autoimmunity whilst promoting tumor immunity, in specific embodiments. However, growing tumor may also use the same phenomenon for the immune escape by trapping NKTs in the hypoxic tissues and disabling their function. These two processes with opposite effects on tumor growth likely occur at the same time, and the balance between them may determine the disease outcome, in particular embodiments. The inventors sought the initiating inflammatory signal that triggers induction of CCL20 expression in monocytes upon their contact with tumor cells has identified an abundant expression of mbTNFα on the cell surface in all tested NB cell lines regardless of their MYCN status and the existence of a previously unknown mbTNFα+ NB cell subset in primary tumors. Demonstrated herein for the first time is that NB cells have potent pro-inflammatory properties as they, in a TNFα-dependent manner, activate NF-kB signaling pathway in monocytic cells that results not only in CCL20 expression, but also in the activation of a defined gene expression program, which is known to be a hallmark of tumor-promoting chronic inflammation (Pikarsky *et al.*, 2004; Greten *et al.*, 2004). For example, NF-kB activates IL-6 gene expression in monocytic cells, a known growth factor for MYCN-non-amplified NB cells, and the level of IL-6 mRNA expression negatively correlates with long-term disease-free survival in high-risk NB patients (Song *et al.*, 2009). The tumor-promoting role of TNFα-NF-kB axis is well-described in epithelial tumors both in mouse models of cancer and in cancer patients (Grivennikov *et al.*, 2010; Greten *et al.*, 2004; Szlosarek *et al.*, 2006; Affara and Coussens, 2007). These are common tumors in adults that can often be etiologically or pathogenetically linked to the pre-existing chronic inflammatory conditions such as hepatitis (Pikarsky *et al.*, 2004) or colitis (Greten *et al.*, 2004). In contrast, NB as well as other pediatric tumors arises during embryogenesis or early postnatal development in the absence of a pre-existing chronic inflammation (Maris *et al.*, 2007). The identification of a subset of NB cells in primary tumors with high levels of mbTNFα indicate that these cells initiate tumor-supportive inflammation and are useful to be targeted for therapy with TNFα antagonists, such as currently being tested in clinical trials in adults with epithelial cancers and hematologic malignancies (Szlosarek and Balkwill, 2003; Brown *et al.*, 2008; Friedberg *et al.*, 2008), for example.

NKT-cell viability and function are affected by hypoxia, but could be protected by IL-2 or IL-15. Studies in mice have demonstrated that NKT-cell development and homeostatic maintenance largely depend on IL-15 (Matsuda *et al.*, 2002). IL-15 also stimulates proliferation and enhances survival of human NKTs (Baev *et al.*, 2004). Human CD4-negative (mostly CD8/CD4-double negative, DN) NKTs express much higher levels of IL-2R∼ than CD4+ subset so that IL-15 preferentially expands DN NKTs (Baev *et al.*, 2004). Importantly, DN NKTs are more cytotoxic than CD4+ NKTs, and a recent report demonstrated that only DN NKTs are required for anti-tumor responses *in vivo* (Crowe *et al.*, 2005). This suggests that the expression of IL-15 in NKTs for therapeutic purposes would support expansion, persistence, and anti-tumor activity of DN NKTs in cancer patients. Transduction of NKTs with IL-15 cDNA protects them from the inhibitory effects of NB cells and hypoxia. Importantly, NKTs/IL-15 demonstrated a potent therapeutic activity against NB metastases in hu-NSG mice. Besides acting directly on NKTs and enhancing their activity against TAMs, locally produced IL-15 is expected to activate other anti-tumor immune effector cells such as NK and tumor-specific CD8 T cells (Walkdmann, 2006). To insure the safety of the potential clinical use of NKTs/IL-15, a suicide gene, iCasp-9, was incorporated that allows the elimination of transgeneic cells upon its pharmacologic activation with a non-toxic small molecular drug, AP20187 (Straathof *et al.*, 2005; Tey et al,. 2007; Quintarelli et al,. 2007). T cells expressing the iCasp-9 molecule are efficiently eliminated upon the pharmacologic activation of the suicide gene both *in vitro* and *in vivo* (Tey *et al.*, 2007; Quintarelli *et al.*, 2007), not only in a mouse model but also in lymphoma patients. Therefore, NKT/IL-15 therapy under iCasp-9 control is expected to be safe and needs to be tested in patients with recurrent/resistant NB.

The mechanism by which NKTs are attracted toward TAMs in tumor tissues is identified herein. In embodiments of the invention, this mechanism reflects a broader role of NKTs in the regulation of inflammation and is applicable not only in the context of tumor-associated inflammation, but also in chronic infectious and autoimmune diseases, for example. The enabling NKT-cell tumor localization and functional activity at the tumor site *via* pharmacological modulation or/and genetic engineering of NKTs as it is demonstrated herein leads to development of effective and broadly applicable immunotherapy of cancer.

### EXAMPLE 9

### EXEMPLARY MATERIALS AND METHODS

Human specimens. Seven primary NBs specimens were obtained from surgery of biopsy at diagnosis at Texas Childrens Cancer Center, Baylor College of Medicine according to IRB approved protocols H-26691 and H-6650. For FACS analysis, tissues were homogenized and digested with dispase II (Roche), collagenase (Sigma) and DNase I into single cell suspensions. The remaining tissues were embedded in OCT and maintained at -80°C. Cord blood was obtained from a cord blood bank at the MD Anderson Cancer Center under IRB approved protocol H-20911. Informed consent was obtained in accordance with institutional review board policies and procedures for research dealing with human specimens.

Cell lines. CHLA-15, CHLA-255, CHLA-255/luc, LA-N-1, LA-N-2, SK-N-BE(2), and IMR32 NB cell lines were established and maintained as previously described (8;25;47). 293T cells were purchased from ATCC and maintained in IMOM 10% FBS (Hyclone), and 2 mM GlutaMAX-1 (Gibco-BRL).

Plasmids and retrovirus production. Two SFG retroviral vectors, SFG.iCasp-9.2A..6.C034.2A.IL-15 and SFG.eGFP.FFluc, were constructed as previously described (32) and used to transduce NKTs. To produce retroviral supernatants, 293 T cells were cotransfected with 3 plasmids (Peg-Pam-e encoding for gag-pol, ORF encoding for the ROF114 viral envelop and the retroviral construct) using the Genejuice transfection reagent (Merck KGaA) and viral supernatants were collected 48 and 72 h later.

RNA isolation and Real-time RT-PCR. Total RNA from cell pellets was isolated using TRlzol (Invitrogen). The RNA quality was assessed with gel electrophoresis prior to reverse transcription into cONA using M-MLV reverse transcriptase with oligo dT priming (Invitrogen). qRT-PCR was performed with iQTM Sybr green supermix assay using the iCycier iQTM multicolor real-time PCR detection system (Bio-Rad). Primers were ordered from Sigma (Table 1).

**Table 1 Exemplary Real-time PCR primers**

| **Gene** | **Forward primer sequence (5'-3')** | **Reverse primer sequence (5'-3')** | **Length** |
|---|---|---|---|
| LAPTM5 | GGTCACACCTCTGAGTATG (SEQ ID NO:1) | GTGGAGGAGAAGAGAAACTC (SEQ ID NO:13) | 128 bp |
| CCL3 | TGGCTGCTCGTCTCAAAGTA (SEQ ID NO:2) | TGCAACCAGTTCTCTGCATC (SEQ ID NO:14) | 116 bp |
| CCL20 | CGTGTGAAGCCCACAATAA A (SEQ ID NO:3) | GCTGCTTTGATGTCAGTGCT (SEQ ID NO:15) | 122 bp |
| CXCL12A | CAGAGCTGGGCTCCTACTGT (SEQ ID NO:4) | GCATTGACCCGAAGCTAAAG (SEQ ID NO:16) | 117 bp |
| CXCL10 | GCAGGTACAGCGTACGGTT C (SEQ ID NO:5) | CAGCAGAGGAACCTCCAGTC (SEQ ID NO:17) | 124 bp |
| CCL17 | TGGAGCAGTCCTCAGATGT C (SEQ ID NO:6) | CTTCTCTGCAGCACATCCAC (SEQ ID NO:18) | 129 bp |
| CCL19 | CATCATTGGTGCCACTCAGA (SEQ ID NO:7) | ACACAGATCCTGCACACACC (SEQ ID NO:19) | 148 bp |
| CXCL11 | ATGCAAAGACAGCGTCCTC T (SEQ ID NO:8) | CAAACATGAGTGTGAAGGGC (SEQ ID NO:20) | 103 bp |
| CXCL16 | CAATCCCCGAGTAAGCATG T (SEQ ID NO:9) | CTACACGAGGTTCCAGCTCC (SEQ ID NO:21) | 119 bp |
| CCL5 | TGTACTCCCGAACCCATTTC (SEQ ID NO:10) | TACACCAGTGGCAAGTGCTC (SEQ ID NO:22) | 100 bp |
| CCL4 | GGATTCACTGGGATCAGCA C (SEQ ID NO:11) | CTTCCTCGCAACTTTGTGGT (SEQ ID NO:23) | 114 bp |
| CCL2 | AGGTGACTGGGGCATTGAT (SEQ ID NO:12) | GCCTCCAGCATGAAAGTCTC (SEQ ID NO:24) | 109 bp |

The relative change in gene expression was calculated based on the ΔCt method using housekeeping gene LAPTM5 (Lysosomal-associated transmembrane protein 5) as the control.

NKT cell expansion, culture, and transduction. NKT cell lines were expanded from PBMCs of healthy volunteers as previously described with modifications (Metelitsa *et al.*, 2004). Briefly, PBMCs were isolated from buffy coats by Ficoll-Hypaque gradient density centrifugation. NKTs were purified by anti-iNKT microbeads (Miltenyi Biotec). The negative PBMC fraction was irradiated (4000 Rad) and aliquoted. NKTs were stimulated with an aliquot of autologous PBMCs pulsed with α-galactosylceramide (100 ng/mL, Funakoshi Co.Ltd). rhlL-2 (200 U/ml, BOP, National Cancer Institute Frederick) was added at the second day and then every other day. NKTs were restimulated every two weeks with the remaining PBMC aliquotes. The phenotype and purity of NKTs were assessed using mAbs for CD3, Vα24-Jα18 (6B11), and CD4. Proliferating NKTs were transducted with retroviral supernatants on day 5 after re-stimulation in non-culture treated 24-well plates pre-coated with recombinant fibronectin fragment (FN CH-296; Retronectin, Takara Shuzo). The rate of NKT-cell transduction was measured by FACS with anti-CD34-PE mAb. The transduced NKTs then continued expansion in the presence of rhIL-2.

Resting NKTs (7-10 days after restimulation) were cultured under normoxic (20% O₂) or hypoxic (1% O₂ in a Hearcell 240i tri-gas incubator, Thermo Scientific) conditions for 24 or 48 h in the absence or presence of one of the following cytokines: rhlL-2 (NIH), IL-15, IL-4, IL-7 (10 ng/ml each, Peprotech), or IL-21 (eBiosciences) at the 200 U/ml each. The absolute number of viable cells was quantified using the trypan blue exclusion method.

Monocyte isolation, co-culture experiments. PBMC were isolated by gradient centrifugation from buffy coats purchased from Gulf Cost Regional Blood Center (Houston, TX). Monocytes were isolated by negative selection using Monocyte Isolation kit II (Miltenyi Biotec) according to the manufacturer's instructions. In co-culture experiments, monocytes were added directly to neuroblastoma cells or to the inserts separated by 0.4 µm membrane (Costar Corning) from neuroblastoma cells. Where indicated, neuroblastoma cells were preincublated with TNFα neutralizing antibody (1825, R&D Systems) or isotype control IgG1 (11711, R&D Systems) for 1 h before co-culture with monocytes. Cells were then cultured under hypoxic (1% O₂ or normoxic (20% O₂) conditions and collected supernatants at indicated time points.

Multiplex cytokine quantification assay and ELISA. Cytokines released by NKTs were assessed by CBAPlex beads (BD Biosciences) on FACSArray analyzer according to the manufacture's manual and as previously described (Metelitsa *et al.*, 2004). CCL20 in the coculture supernatants were determeined using Human CCL20/MIP-3 alpha Quantikine ELISA Kit (R&D Systems).

*In vitro* migration assay. The NKT-cell *in vitro* migration was assessed using permeable Transwell inserts (5 um, Corning Costar). Where indicated, supernatants from monocyte and NB cells in bottom chambers were pre-incubated with isotype control (clone 11711) or neutralizing antibody against CCL20 (67310) or CCL2 (24822) (R&D Systems) for 1 hr before adding NKTs in the upper chambers. Quantitative analysis of NKT-cell migration was performed by FACS as previously described (Song *et al.*, 2007).

Flow cytometry (FACS). To analyze the expression of CCL20 in monocytes, cells were first incubated with GolgiStop (BO Biosciences) for 4 h and then stained with the following surface markers: CD56-APC, CD14APC. Cy7, CD33-PE.Cy7 and CD45-PerCP (BO Biosciences). Cells were then fixed and permebalized with a Perm/Fix Kit (BO Biosciences) and intracellularly stained with CCL20-PE (BO Biosciences). To determine the level of CCR6 expression on NKTs, cells were surface stained with CD3-APC, 6B11-FITC, CD4-(PE.Cy7), and CCR6-PE (BO Biosciences).

To monitor the development of human hematopoiesis in hu-NSG mice, the following set of mAbs was utilized: anti-human CD45-FITC, anti-mouse CD45-PerCP, CD14-PE, CD33-PE.Cy7, CD3-APC (BO Biosciences) and CD20APC.Cy7 (Biolegend). To determine purity of the human CD34+ stem cells, the inventors used CD45-PerCP, CD3APC and CD34-PE (BO Biosciences). To analyze human leukocytes infiltrating primary neuroblastomas or tumor xenografts in hu-NSG mice: CD45-PerCP, CD3-APC, 6B11-FITC, CD14-APC.Cy7, HLADR-PE.Cy7, CD1d PE (BO Biosciences). Additionally, primary neuroblastomas were analyzed for surface marker expression using mAbs: CD45-PerCP, CD56-APC (BO Biosciences) and TNFα-FITC (R&D Systems).

To determine the NKT-cell proliferation, cells were first labeled with CFSE (Invitrogen) and then restimulated by anti-TCR agonistic mAb (6B11 or OKT3) followed by a 5-day culture with/without NB cells under hypoxic or normoxic conditions. Cells were then surface stained with the 6B11-PE and CD3-APC mAbs and analyzed by flow cytometry to determine CFSE dilution in CD3+6B11 + cells.

The expression levels of IkBα and phosphorylated NF-kB p65 were determined by FACS in monocytes according to the manufacturer's protocols (BD PhosFlow). Briefly, 1-2 x10⁶ ml⁻¹ monocytes were cultured with/without CHLA-255 neuroblastoma cell line (∼70-80% confluence) in the presence of neutralizing aTNFα or suitable isotype control for specified time points. After incubation, cells were fixed immediately by adding an equal volume of pre-warmed to 37C BD PhosFlow Cytofix fixation buffer; plates were incubated for additional10 min at 37C and then the cells were harvested. For permeabilization, BD Phosflow Perm Buffer IV, pre-cooled at -20C was drop wisely added to the cell pellet and incubated for additional 15-20 min at RT. The tubes were stored at -20C until stained with Alexa Fluor 488 phospho-specific anti-NFkB p65 mAb (or Pelabeled IkBa) and PE or APC-Iabeled anti-CD56 monoclonal antibody (mAb) to identify the NB and monocytes in co-culture.

The analysis was performed on a LSR-II four-laser flow cytometer (BD Biosciences) using BD FACDiva software v. 6.0 and FlowJo 7.2.5 (Tree Star, Inc).

*In vivo* experiments. NOD/SCID/IL2rgamma(null) (NSG) mice were bred in the TCH animal facility. Four-week old mice were irrradiated with 225 cGy and injected with human cord blood derived CD34+ stem cells as previously described (Yahata *et al.*, 2002; Giassi et al,. 2008). The frequency of CD34+ cells was >95% and the contamination by CD3+ cells was less than 0.1% in the stem cell transplants. Three months after SCT, reconstitution of human hematopoiesis was confirmed in peripheral blood by FACS and mice were i/v injected with human CHLA255/luc cells either under the renal capsule (localization experiments) as previously described (Kim *et al.*, 2002) or intravenously (therapeutic experiments). Tumor growth was indirectly assessed by weekly bioluminescent imaging (Small Animal Imaging Core facility, TCH). Where indicated, mice were injected intraperitoneally with 100 Ilg/mouse of neutralizing anti-CCL2 (24822), anti-CCL20 (67310), or isotype control (11711) mAb (R&D Systems). Some animals received intravenous injections of *ex vivo* expanded human NKTs (1-5X10⁷ cells). Before injection into animals, NKTs had been cultured with IL-2, 50 U/ml (Peprotech) for 7-10 days without TCR-stimulation to achieve resting phase when their trafficking pattern more closely resembled that of primary NKTs as we determined previously (Metelitsa *et al.*, 2008). Mice were sacrificed after 72 h, and cell suspensions prepared from tumors were analyzed by multi-color flow cytometry as described in "Flow cytometry" section. When indicated, NKT-cell anti-tumor efficacy was determined by bioluminescent imaging. Animal experiments were performed according to IACUC approved protocols.

Statistical analyses. In the *in vitro* and *in vivo* experiments, comparisons between groups were based on the two-sided unpaired Student's t-test or one-way ANOVA with the Tukey-Kramer post-test comparison of group means. Statistical computations were performed with GraphPad Prism™ 5.0 software (GraphPad Software).

### EXAMPLE 10

### TARGETING NEUROBLASTS AND NEUROBLAST-SUPPORTIVE MACROPHAGES WITH DUAL-SPECIFIC NKT CELLS

**Introduction.** The infiltration of primary tumors with Vα24-invariant Natural Killer T (NKT) cells is associated with good outcome in neuroblastoma and other types of cancer. Mechanistic studies revealed that instead of attacking tumor cells directly, NKT cells target CD1d-positive tumor-associated macrophages (TAMs). However, effective immune control of tumor may also require direct and specific attack against the tumor cells.

**Methods.** *Ex vivo* propagated human NKT cells were engineered using a retroviral vector encoding a chimeric antigen receptor (CAR) that targets GD2 ganglioside which is highly expressed by neuroblastoma cells and represents a clinically validated therapeutic target. The functional activity of the native TCR and CAR.GD2 in the gene-modified NKT cells was tested using CD1d+ TAMs and GD2+ neuroblastoma cells, respectively. Next, we encoded co-stimulatory endodomains *in cis* with the CAR.GD2 (CD28, CD134, CD137 and their combinations) to enable optimal CAR-mediated signaling for NKT cell cytotoxicity, cytokine production, proliferation, and survival.

**Results.** Expression of CAR.GD2 constructs rendered NKT cells highly cytotoxic against GD2-positive neuroblastoma cells while leaving their native CD1d-restricted cytotoxicity unaffected. Only the CAR.GD2 NKT cells that expressed the co-stimulatory endodomains from CD28, CD134, and/or CD137 underwent rapid proliferation upon specific stimulation sufficient to enable clinical scale expansion of the gene-modified NKT cells. While adoptive transfer of the parental NKT cells only transiently suppressed growth of metastatic neuroblastoma in humanized NOD/SCID/IL-2γ(null) mice, NKT cells expressing CAR.GD2 with CD28 or CD137 endodomains had potent and long-lasting anti-metastatic activity. Furthermore, there was a striking and previously unanticipated Th2-like (IL-4 and IL-10) and Th1-like (IFNγ and GM-CSF) polarization of NKT cells expressing CAR.GD2/CD28 and CAR.GD2/CD137, respectively.

**Conclusion.** NKT cells engineered to express CAR.GD2 with co-stimulatory endodomains can be expanded to clinical scale, target both neuroblasts and neuroblast-supportive TAMs, and have potent anti-tumor activity. In addition to directing NKT cell cytotoxicity toward tumor cells, CAR constructs that contain CD137 co-stimulatory endodomain can program NKT cells to produce large amounts of IFNγ and GM-CSF that in turn activate multiple types of anti-tumor effector cells. These results establish that NKT cells can serve as a novel platform for anti-tumor CAR therapy in neuroblastoma and other types of cancer.

### EXAMPLE 11

### PARTICULAR EMBODIMENTS OF THE INVENTION

The invention provides an effective immunotherapy of cancer using natural and engineered properties of NKTs to target both tumor-supportive stromal cells and tumor cells themselves.

The importance of NKTs for antitumor immunity and immunotherapy has been demonstrated in multiple models of cancer (Swann *et al.*, 2007; Dhodapkar, 2009; Song *et al.*, 2007). NKT-cell infiltration of primary tumors was associated with good outcome in children with NB (Metelitsa *et al.*, 2004), a finding that has since been extended by other researchers to adult malignancies (Tachibana et al,. 2005; Molling *et al.*, 2007). Recent findings indicate that instead of attacking tumor cells directly, NKTs target CD1d-positive TAMs, which provide essential stromal support for tumor cells (Song et al,. 2009; De Santo *et al.*, 2008). However, in addition to attacking tumor stroma, effective immune control of tumor may also require direct and specific attack against the tumor cells. The infusion of EBV-CTLs grafted with a CAR that targets the GD2 antigen expressed by the neuroblasts (CAR.GD2) provides objective tumor responses in patients with refractory/relapsed NB (Pule *et al.*, 2008; Louis *et al.*, 2011). In embodiments of the invention, gene-modified NKTs with CAR.GD2 have anti-tumor efficacy in NB *via* targeting of neuroblast-supportive TAMs and neuroblasts themselves.

The accumulated knowledge of human NKT cell biology suggests that, as with other T cells (Maher *et al.*, 2002; Porter *et al.*, 2011), the therapeutic potential of CAR-expressing NKTs could be increased by providing optimal co-stimulatory signals. To further characterize this, the inventors generated constructs of CAR.GD2 with exemplary co-stimulatory endodomains: CD28, OX40, and/or CD137. Furthermore, IL-15 protects NKTs from tumor-induced hypoxia and it was demonstrated that transgenic expression of IL-15 in NKTs dramatically enhances their anti-metastatic activity (Liu et al,. 2012). With this knowledge, the inventors generated new CAR.GD2 constructs that encode both co-stimulatory endodomains and IL-15 (bicistronic vectors). To ensure the safety and clinical applicability of the gene modification, we previously also generated tricistronic vectors in which CAR and IL-15 were coupled with the expression of the inducible caspase-9 (iCasp-9), which is activated by a non-toxic drug CID AP1903 (Quintarelli *et al.*, 2007; Hoyos *et al.*, 2010), forming a suicide switch that has been found to be safe and highly effective in a recent phase I clinical trial (Di *et al.*, 2011).

The following sets forth certain embodiments of the invention.

Expression of CAR.GD2 in NKTs is produced and their *in vitro* cytotoxicity is tested against NB cells and *in vivo* anti-tumor activity against NB xenografts in hu-NSG mice. NKT cells isolated from NB patients are *ex vivo* expanded and transduced with retroviral CAR.GD2 construct. CAR.GD2+ NKTs are tested for their *in vitro* cytotoxicity against GD2+ NB cell lines and for their native cytotoxicity against CD1d+ cells. *In vivo* studies of adoptively transferred CAR.GD2+ NKTs evaluate their persistence, tumor localization, and anti-tumor efficacy using established NB models in hu-NSG mice.

There is expression of CAR.GD2 with co-stimulatory endodomains in NKTs and their *in vivo* persistence and anti-tumor activity is characterized. NKTs are transduced with CAR.GD2 constructs designed with co-stimulatory moieties that are known to provide major co-stimulatory signals for NKTs: CD28, 4-1BB, CD28/CD137, or CD28/OX40. *In vitro* experiments select those constructs that in response to GD2 stimulation support superior NKT cell proliferation and Th1-biased cytokine response while keeping intact native NKT TCR specificity and the ability to kill TAMs. NKTs modified with these selected constructs are then evaluated for *in vivo* expansion, persistence, and increased anti-tumor efficacy in the hu-NSG NB models.

There is expression of CAR.GD2/IL-15 in NKTs and their *in vivo* persistence and anti-tumor activity is characterized. CAR.GD2/IL-15 is expressed in NKTs using a tricistronic vector that encodes CAR.GD2 with a co-stimulatory endodomain, IL-15, and iCasp9. CAR.GD2/IL-15 NKTs will be evaluated for the ability to support *in vivo* NKT cell expansion, long-term persistence, and increased anti-tumor efficacy in the hu-NSG NB models. Treatment with AP1903 will be used to test the efficacy of iCasp-9 suicide switch for the elimination of gene-modified NKTs.

There is production of a GMP grade retroviral vector and validation of its activity using NKTs from NB patients. Once the construct is selected, one can generate stable retroviral producer cell line and there is production of a stock of the clinical grade vector that is used to transduce patient-derived NKTs, which functional properties and antitumor activity are validated in *in vitro* and *in vivo* assays.

Specific embodiments are as follows: 1) evaluation of the role of CCL20/CCR6 axis in the mechanism of NKT-cell localization to the hypoxic tumor tissues; 2) expression of mbIL-7-ODDD in human NKTs for improved survival in hypoxia and to test the antitumor potential of mbIL-7-NKTs against human neuroblastoma xenografts in NOD/SCID mice; evaluation of the antitumor therapeutic potential of combined immunotherapy with NKTs or mbIL-7-NKTs and ant-GD2 CAR-T cells.

As demonstrated at least in Figs. 10-13, a previously unknown subset of cells in NB cell lines and primary tumors express membrane-bound (mb)TNFα. These pro-inflammatory tumor cells induced production of the chemokine CCL20 from TAMs *via* activation of the NF-kB signaling pathway, an effect that was amplified in hypoxia. Flow cytometry analyses of human primary NB tumors revealed selective accumulation of CCL20 in TAMs. Neutralization of the chemokine inhibited *in vitro* migration of NKTs toward tumor-conditioned hypoxic monocytes and localization of NKTs to NB grafts in mice. Hypoxia impairs NKT-cell viability and function. Thus, NKT cell trafficking toward CCL20-producing TAMs served as a hypoxic trap for tumor-infiltrating NKTs.

Because the expression of mbIL-7-ODDD in NKTs failed to rescue them from the inhibitory effect of hypoxia, as an alternative approach, the inventors tested other cytokines which share a common gamma chain. IL-2 and IL-15 protected antigen-activated NKTs from hypoxia. Moreover, transgenic expression of IL-15 in adoptively transferred NKTs dramatically enhanced their anti-metastatic activity in mice. Thus, tumor-induced chemokine production in hypoxic TAMs and consequent chemoattraction and inhibition of NKTs represents a mechanism of immune escape that can be reversed by adoptive immunotherapy with IL-15-transduced NKTs.

New models. To study the therapeutic potential of NKTs, the inventors have developed novel models of orthotopic and metastatic NB in hu-NSG mice that are reconstituted with human CD34+ hematopoietic stem cells. These unique models are of high clinical relevance because they allow for the first time to study tumor localization of human NKTs or other immune effector cells and their interaction not only with human NB cells but also with human stromal cells (*e.g.* TAMs) of hematopoietic origin in the tumor tissues.

The present invention provides a novel concept of immunotherapy in which tumor cells and tumor-supportive stromal cells are simultaneously targeted to achieve the maximal therapeutic efficacy. At this stage, the inventors use NKTs or IL-15 NKTs and CAR.GD2 T cells to targets TAMs and neuroblasts, respectively. CAR.GD2 T cells have already been clinically tested in NB patients, but the present invention provides combined immunotherapy of CAR.GD2 T cells with NKTs or IL-15 NKTs. In certain aspects of the invention NKTs are enabled with dual reactivity for targeting both neuroblasts and TAMs.

### EXAMPLE 12

### EXEMPLARY STUDIES

In embodiments of the invention, there is localization of human NKT cells to the tumor site in hu-NSG NB model. (see Liu et al, J. Clin. Invest (2012)).

In embodiments of the invention, IL-15 protects NKTs from inhibition by TAMs and enhances anti-metastatic activity.

IL-15 protected antigen-activated human NKTs from hypoxia, and transgenic expression of IL-15 in adoptively transferred NKTs dramatically enhanced their anti-metastatic activity in hu-NSG model of NB, Figs. 6 and 7 (Liu *et al*., 2012).

In embodiments of the invention, NKTs are modified to co-express functional CAR-GD2. Co-stimulatory moieties derived from CD28, 4-1BB, and OX40, for example, can be incorporated *in cis* within CAR molecules and provide co-stimulation to CAR-modified T cells (Pule *et al*., 2008; Maher *et al*., 2002; Vera *et al*., 2009). The inventors constructed a series of CARs that incorporate CD28, OX40 or both CD28/OX40 and CD28/4-1BB (Fig. 11A), and found that these molecules can be efficiently expressed in NKTs (Fig. 11B). Moreover, CAR expression remains stable for at least 3 weeks of NKT-cell *ex vivo* expansion (Fig. 11B). Side by side comparison of CAR.GD2 NKT and T cells from the same individuals demonstrated equally potent *in vitro* cytotoxicity against GD2-positive NB cells (Fig. 11C). No difference in GD2-specific cytotoxicity was observed between NKTs transduced with any of the 5 constructs. Importantly, CAR.GD2 NKTs retained their native TCR specificity and killed CD1d+ targets as efficiently as parental NKTs (Fig. 11D).

In embodiments of the invention, pharmacologic activation of the iCasp-9 suicide gene efficiently eliminates gene-modified T lymphocytes in leukemia patients. (see Di Stasi et. al., N. Engl. J. Med (2011*)).*

### Exemplary Strategies

### Express CAR.GD2 in NKTs and test their in vitro cytotoxicity against NB cells and in vivo anti-tumor activity against NB xenografts in hu-NSG mice.

Although CAR-modified EBV-specific CTLs have clinical efficacy in patients with several types of cancer, the dependence on EBV infection limits this approach, especially for young EBV-negative patients. Because NKTs do not depend on infection and have a similar functional profile with effector-memory T cells, one can test whether NKTs could serve as an alternative platform for CAR-redirected immunotherapy of cancer. A recent clinical trial demonstrated that targeting GD2 with CAR-modified EBV-CTLs can be effective in children with NB (Pule *et al*., 2008; Louis *et al*., 2011); one can use the same CAR.GD2 construct to transduce NKTs. These CAR.GD2+ NKTs are expanded and their anti-tumor potential against NB is tested using our established *in vitro* and *in vivo* experimental systems.
(a) Expression of CAR.GD2 in NKTs. Primary NKTs are isolated from PBMCs of 5 healthy individuals and 5 patients with stage 4 NB at diagnosis using biotin-6B11 mAb and anti-biotin magnetic beads (Miltenyi) followed by *in vitro* expansion with OKT3 and IL-2 as previously described (Metelitsa *et al*., 2004; Exley *et al*., 2008). To express CAR.GD2 in NKTs, OKT3-stimulated cells are transduced with the retroviral vector as described in Fig. 11B. One can also express CAR.GD2 in T cells from the same PBMC as previously described (de Santo *et al*., 2008). Expression of CAR.GD2 is detected by FACS analysis, and CAR.GD2+ cells are enriched using biotinilated anti-idiotype antibody 1A7 (PUle *et al*., 2008) and anti-biotin magnetic beads (Miltenyi) followed by additional expansion with OKT3 and IL-2.
(b) *In vitro* functional activity of CAR.GD2 NKTs. To test the cytotoxic potential of CAR.GD2 NKTs, one can grow GD2-positive NB cell lines (CHLA-255, LA-N1), GD2-negative NB cells (LA-N-6) as a negative control, and GD2^{neg}CD1d+ Jurkat J32 cells as a control for CD1d-restricted NKT cell cytotoxicity. The cytotoxicity is performed using 4-hr Calcein-AM assay with a range of effector to target ratios. CAR.GD2 T cells from the same individual can serve as a positive control. One can also measure NKT-cell cytokine response (IFNγ and IL-4, ELISA) and proliferation (CFSE dilution assay) after stimulation of CAR and native Vα24i TCR by GD2+ NB and CD1d+ J32 cells, respectively. Based on the results obtained with CAR.GD2 NKTs from 2 healthy individuals (Fig. 2B-D), one can expect that CAR.GD2 NKT cells from healthy donors and NB patients will acquire cytotoxicity against GD2+ NB cells while retaining their native TCR specificity and functional activity.
(c) *In vivo* persistence and tumor homing of CAR.GD2 NKTs. To examine the effect of CAR.GD2 expression on NKT cell persistence and tumor localization, one can transduce NKTs with eGFP-ffLuc followed by transduction with CAR.GD2 or vector control. Hu-NSG mice are grafted with human NB xenografts under the renal capsule and (in 2 weeks) i/v injected with 10⁷ control NKTs or CAR.GD2 NKTs. NKT-cell distribution and and tumor localization are monitored at 24, 48, and 72 hrs using BL imaging. After animal sacrifice, one can quantify the frequency of transferred human NKTs in blood, spleen, liver, bone marrow, and tumor tissues. The parental and gene-modified NKTs may be quantified both by qPCR and FACS, for example. Q-PCR can be performed on DNA samples from the indicated tissues using two sets of probe/primers: Vα24-Jα18 invariant TCRα gene and CAR transgene for all human NKTs and gene-modified NKTs, respectively. The frequency and absolute number of NKTs and CAR.GD2 NKTs can also be quantified by FACS using four-color immunofluorescence with antibodies against human CD3, Vβ11, Vα24-Jα18, and CAR. NKT and CAR.GD2 NKTs can also be compared for the expression of annexin-V and Ki-67+ to determine the rate of apoptosis and proliferation, respectively. Within the tumor tissues, one can examine whether CAR expression affects NKT-cell distribution between normoxic and hypoxic areas as well as their co-localization with TAMs and NB cells, using known confocal microscopy methods (Liu et. al., 2012) The results of these experiments demonstrate how CAR.GD2 expression affects NKT cell *in vivo* persistence, localization to the tumor site and reveal the mechanism of CAR.GD2 NKT-cell effector function within the tumor tissues, in certain aspects of the invention.
(d) Antitumor activity of CAR.GD2 NKTs in the orthotopic NB model in hu-NSG. Hu-NSG mice are grafted under the renal capsule with human ffLuc+ CHLA-255 NB cells and divided into three groups for treatment with 10⁷ NKTs, CAR.GD2 NKTs, or PBS as a control. The antitumor efficacy is evaluated by weekly BL imaging (for 4 weeks) and time-to-sacrifice. CAR.GD2 NKTs achieve greater antitumor activity, in specific embodiments of the invention, sustained longer compared to unmodified NKTs. Even though CAR.GD2 NKTs and CAR.GD2 T cells are equally cytotoxic *in vitro* (Fig. 11C), the former are expected to have a stronger therapeutic activity in mice due to killing or M1-polarization (*via* secretion of IFNγ and GM-CSF) of TAMs. NKTs are analyzed in tumor xenografts and in normal tissues as described above. One can also examine whether the therapeutic efficacy is associated with the direct NKT-cell targeting of tumor cells, the reduction of TAM frequency in tumor tissues, increased M1 polarization of remaining TAMs, and/or decreased levels of neuroblast-promoting human IL-6 (Song *et al*., 2009) inside the tumors and systemically. Pathological examination of normal tissues can also evaluate the toxicity of NKT cell immunotherapy. The results of these experiments show the *in vivo* therapeutic use of CAR.GD2 NKTs for clinical applicability.
(e) Antitumor activity of CAR.GD2 NKTs in the metastatic NB model in hu-NSG mice. When injected intravenously in hu-NSG mice, CHLA-255/luc cells provide an excellent metastatic model of NB that closely reproduces the pattern of metastases in NB patients (Fig. 10B). The adoptive transfer of human NKTs has only a transient effect in this metastatic NB model (Liu *et al*., 2012). To test whether CAR.GD2 expression renders NKTs protective against NB metastases, one can inject mice with CHLA-255/luc cells i.v. and divide them into the following groups to receive: 1) PBS (control); 2) parental NKTs; 3) CAR.GD2 NKTs; 4) CAR.GD2 T cells. The initial experiments are performed at day 7, which is at least one week before NB mets become detectable by BL imaging. If CAR.GD2+ NKTs are effective in this setting, one can repeat the same treatment after NB mets become detectable by BL imaging. Postmortem, NKTs and T cells are analyzed in tumor mets in different organs and in normal tissues as described above. One can observe if there is accumulation of CAR.GD2 NKTs compared with control NKTs in the bone marrow because it is the most frequent site of NB metastasis and relapse. The results of these experiments show the therapeutic potential of CAR.GD2 NKTs against metastatic NB, in particular aspects of the invention.

### Express CAR.GD2 with co-stimulatory endodomains in NKTs and test their in vivo persistence and anti-tumor activity.

Co-stimulation plays a critical role in the activation, expansion, and survival of NKTs. Based on current knowledge of the major co-stimulatory pathways in NKTs, four constructs were generated containing co-stimulatory endodomains and their combinations: CD28, 4-1BB, CD28/4-1BB and CD28/OX40, and the control construct that lacks co-stimulatory endodomains. One can determine which of these constructs provide the most effective co-stimulation for CAR.GD2 NKTs.

(a) *In vitro* activity of CAR.GD2 NKTs with different co-stimulatory moieties. First, one can test how expression of co-stimulatory moieties affects *in vitro* cytotoxicity, cytokine production, and proliferation of CAR-GD2 NKTs in response to GD2+ NB cells as well as CD1d+ J32 cells as described herein. Based on accumulated experience with T cells (Hoyos *et al*., 2010) and data with NKTs, one can expect that the cytotoxic activity of CAR.GD2 NKTs will not be influenced by co-stimulatory moieties. By contrast, the production of Th1/Th2 cytokines and proliferation will likely be influenced by different co-stimulatory signals. The *in vitro* screening can select those co-stimulatory CAR constructs that enhance NKT-cell expansion and polarize their cytokine profile toward Th1.

(b) *In vivo* homing and persistence of co-stimulatory CAR.GD2 NKTs. Those exemplary co-stimulatory moieties that are selected are evaluated for the ability to support *in vivo* persistence and tumor localization of CAR.GD2-modified NKTs. For example, to determine the effect of CD28 expression in CAR.GD2, one can transduce NKTs with eGFP-ffLuc followed by transduction with CAR.GD2 or CAR.GD2/CD28. Hu-NSG mice are grafted with human NB xenografts and i/v injected with CAR.GD2 or CAR.GD2/CD28 NKTs. NKT cell tumor localization and persistence is analyzed using BL imaging, FACS, and confocal microscopy as described herein. One can expect that NKTs expressing CAR.GD2/CD28 and/or other selected co-stimulatory constructs + will have a superior *in vivo* expansion, persistence, and accumulation at the tumor site, in specific embodiments of the invention.

(c) Antitumor activity of co-stimulatory CAR.GD2 NKTs in the orthotopic NB model in hu-NSG mice. As described here, one can use hu-NSG mice with established NB xenografts (with luminescent NB cells) and treat them with non-luminescent CAR.GD2, CAR.GD2/CD28, and/or other co-stimulatory constructs. The antitumor efficacy is evaluated by BL imaging once a week for 4 weeks. NKTs modified to express CAR.GD2 with co-stimulatory moieties will have a superior anti-tumor activity than those with CAR.GD2+ alone, in particular aspects of the invention.

(d) Antitumor activity of co-stimulatory CAR.GD2 NKTs in the metastatic NB model in hu-NSG mice. The same experimental groups as described above are tested in the metastatic NB model as described above. NKTs modified to express CAR.GD2 with co-stimulatory moieties have a superior anti-metastatic activity than those with CAR.GD2 alone, in specific embodiments of the invention.

### Express CAR.GD2/IL-15 in NKTs and test their in vivo persistence and anti-tumor activity.

To maximize the therapeutic potential of NKTs against NB, one can combine expression of IL-15 and CAR.GD2 that will enable long-term persistence of the adoptively transferred cells and provide the ability to kill both tumor-supportive TAMs and neuroblasts themselves. Additional safety of the transduced cells is provided by developing a vector, in which iCasp-9 suicide gene is cloned in conjunction with the CAR and IL-15 genes. To achieve effective expression of all three genes (IL-15, iCasp-9, and CAR.GD2) in NKTs, one can generate a single tricistronic retroviral construct (Fig. 13), analogous to one that has been shown to successfully express iCasp9, CAR.CD19-CD28 and IL-15 in T cells which allowed efficient and functional expression of all three genes (Hoyos et ao., 2010).
a) Expression and *in vitro* functional activity of IL-15 and CAR.GD2 in NKTs. Using the same procedure as described above, one can transduce NKTs with a tricistronic retroviral construct, which contains cDNA sequences for IL-15, CAR-GD2/CD28, and iCasp-9 (Fig. 13). To control for the effect of IL-15 or CAR.GD2 alone, one can compare IL-15/CAR.GD2-transduced NKTs with those transduced with either IL-15 or CAR.GD2 alone. One can also test the functionality of iCasp-9 suicide gene using *in vitro* treatment of the transduced cells with CID AP1903 as previously described (Quintarelli *et al*., 2007; Tey *et al*., 2007).
(b) *In vivo* homing and persistence of CAR.GD2/IL-15 NKTs. To examine whether IL-15 and CAR.GD2 coexpression enhances NKT cell tumor homing and persistence, one can transduce NKTs with eGFP-ffLuc followed by transduction with CAR.GD2/IL-15. Hu-NSG mice are grafted with human NB xenografts and i/v injected with CAR.GD2, IL-15+, CAR.GD2/IL-15+, or control NKTs (10⁷ cells/mouse). NKT-cell tumor localization is monitored at 1, 2, 3, 5, and 7 days using BL imaging followed by sacrifice and FACS analysis in blood, spleen, liver, bone marrow, and tumor tissues. The distribution of infiltrating NKTs within the tumor tissues is analyzed by confocal microscopy, including quantitative analysis of their frequency in hypoxic areas using EF5 staining (Liu *et al*., 2012). NKTs expressing IL-15 and CAR.GD2 will have a superior *in vivo* expansion and persistence as well as enhanced accumulation and survival at the tumor site including hypoxic areas, in specific embodiments of the invention.
(c) Control of CAR.GD2/IL-15-transduced NKTs *via* pharmacological caspase-9 activation. Since the retroviral construct incorporates the iCasp-9 (Fig. 12),one can test whether gene-modified NKTs can be efficiently eliminated by the pharmacologic activation of the suicide gene upon administration of the small molecule CID AP1903 (Quintarelli *et al*., 2007; Tey *et al*., 2007). After achieving sustained *in vivo* expansion/persistence of CAR.GD2/L-15-NKTs in hu-NSG mice, mice are i/p injected with CID AP1903. Mice are imaged before and 24 hr after drug injection. If the BL signal disappears, one can sacrifice mice and perform qPCR for the CAR transgene using DNA from multiple tissues to confirm the elimination of the transgenic NKTs. If the elimination is not complete, one can titrate the dose of CID AP1903 to achieve the full effect as described for T cells.
(d) Antitumor activity of CAR.GD2/IL-15 NKTs in hu-NSG NB models. As described above, one can use hu-NSG mice with established orthotopic or metastatic NB xenografts and treat them with CAR.GD2, IL-15, CAR.GD2/IL-15, or control NKTs. The antitumor efficacy is evaluated by BL imaging once a week for 4 weeks. CAR.GD2/IL-15 NKTs are more effective than CAR.GD2 or IL-15 NKTs, in certain embodiments.

### Produce a GMP grade retroviral vector and validate its activity using NKTs from NB patients.

The results of pre-clinical testing of NKTs transduced with various CAR.GD2 constructs or GAR.GD2/IL-15 above informs one which construct generates therapeutic NKTs that are safe and have high antitumor potential for NB patients. In specific embodiments, GAR.GD2/IL-15 has the highest therapeutic potential of the tested constructs. Alternative constructs (e.g. CAR.GD2/CD28, CAR.GD2/4-1.BB, or the 1^{st} generation CAR.GD2) may be utilized. A stable retroviral producer cell line is generated and a stock of the clinical grade vector that will be used to transduce patient-derived NKTs is produced.
a) To generate a clinical grade retroviral producer cell line and retroviral supernatant. One can generate the stable retroviral producer cell line, producing viral particles encoding CAR.GD2/IL-15 or one of the alternative constructs. The packaging cell line PG13 that makes GaLv pseutotype retroviral particles will be generated as previously decribed (Pule *et al*., 2008; Savoldo *et al*., 2011). A stable producer line with a viral titer >10⁶ v.p. per mL (based on HeLa cell infectivity) may be produced. Although the titer may decrease when bulk product is manufactured, it should still exceed 5x10⁵ v.p. per mL, which will provide an ample quantity of virus with adequate activity to transduce the NKT cells, in certain embodiments.
(b) To prepare gene-modified NKTs from NB patients. NKTs may be isolated from PBMCs of deceased NB patients using biotin-6B11 mAb and anti-biotin magnetic beads (Miltenyi) followed by *in vitro* expansion on OKT3-coated plates. To validate the activity of a clinical grade vector, OKT3-activated NKTs are transduced with retroviral supernatant using 24-well plates coated with recombinant retronectin fragments as previously described (Pule *et al*., 2008). After transduction, NKTs are expanded with IL-2 to reach the cell number required for the clinical protocol. Transduction efficiency is measured by quantifying the expression of CAR idiotype epitope.
(c) *In vitro* functional activity. The NKTs modified with the clinical grade vector are tested using *in vitro* functional experiments that are appropriate for the selected construct and described elsewhere herein.
(d) *In vivo* homing and persistence is examined as described elsewhere herein.
(e) Control of gene-modified NKTs *via* pharmacological caspase-9 activation is examined as described elsewhere herein.
(f) Antitumor activity in NB models is examined as described elsewhere herein.

It can be demonstrated that GMP-grade gene-modified NKTs can be produced from patient-specific PBMC in clinically-sufficient numbers and have antitumor activity while retaining their physiological functions.

### EXAMPLE 13

### PARTICULAR EMBODIMENTS OF NKTS WITH CHIMERIC ANTIGEN RECEPTORS

In embodiments of the invention, there is an NKT cell with one or more CARs, and the NKT cell may also express IL-15, IL-2, IL-4, and/or IL-7. See Figure 11A for exemplary CAR constructs.

FIG. 14 shows effective generation and expansion of CAR.GD2 NKTs. FIG. 15 shows CAR.GD2 NKTs are endowed with dual specificity against GD2+ and CD1d+ targets. FIG. 16 demonstrates co-stimulatory endodomains in CAR.GD2 constructs affect NKT-cell cytokine profile. FIG. 17 shows co-stimulatory endodomains in CAR.GD2 constructs affect Th1 and Th2 signaling pathways in NKTs. FIG. 18 shows therapeutic efficacy of CAR.GD2 NKTs against NB mets in hu-NSG mice.

Thus, FIGS. 14-18 demonstrate the following: 1) GD2.CARs can be stably expressed in human NKTs and a high-level transgene expression is maintained for the period sufficient for a clinical scale *ex vivo* expansion; 2) GD2-CAR NKTs exhibit dual specificity with high cytotoxic potential against GD2+ NB cells and CD1d+(M2) macrophages, but do not kill GD2 & CD1d negative cells; 3) CD28 and 4-1BB endodomains skew the cytokine response of CAR.GD2 NKTs to Th2 and Th1 pattern, respectively; 4) CD28-induced Th2 polarization is associated with activation of AKT and STAT6. 4-1BB-induced Th1 polarization is associated with activation of p65 NFκB & p38 MAPK pathways; and 5) GD2.CAR expressing NKTs have potent therapeutic activity in a metastatic model of neuroblastoma in hu-NSG mice, providing a rational for the clinical testing of dual specific NKTs in children with neuroblastoma. In embodiments of the invention, an expression construct in the NKT comprises 4-1BB or a combination of 4-1BB and CD28 so that Th1 polarization is induced, thereby leading to pathways relevant to cancer; in specific embodiments the CD28 permits enhanced proliferation of the NKT cells.

### REFERENCES

All patents and publications mentioned in this specification are indicative of the level of those skilled in the art to which the invention pertains. All patents and publications herein are incorporated by reference to the same extent as if each individual publication was specifically and individually indicated to be incorporated by reference in their entirety.

### PUBLICATIONS

Affara,N.I., and Coussens,L.M. 2007. IKKalpha at the crossroads of inflammation and metastasis. Cell 129:25-26.
Aliavena,P., Sica,A, Solinas,G., Porta,C., and Mantovani,A 2008. The inflammatory micro-environment in tumor progression: the role of tumor-associated macrophages. Grit Rev. Oncol. Hematol. 66:1-9.
Baev,D.V., Peng,X.H., Song,L., Barnhart,J.R., Crooks,G.M., Weinberg,K.I., and Metelitsa,L.S. 2004. Distinct homeostatic requirements of CD4+ and CD4-subsets of Valpha24-invariant natural killer T cells in humans. Blood 104:4150-4156 (2004).
Battaglia,F., Delfino,S., Merello,E., Puppo,M., Piva,R., Varesio,L., and Bosco,M.C. 2008. Hypoxia transcriptionally induces macrophage-inflammatory protein-3alpha/CCL-20 in primary human mononuclear phagocytes through nuclear factor (NF)-kappaB. J. Leukoc. BioI. 83:648-662.
Bendelac,A. et al. CD1 recognition by mouse NK1+ T lymphocytes. Science 268, 863-865 (1995).
Bendelac,A., Savage,P.B., & Teyton,L. The biology of NKT cells. Annu. Rev. Immunol. 25, 297-336 (2007).
Berzofsky,J.A., and Terabe,M. 2009. The contrasting roles of NKT cells in tumor immunity. Curro Mol. Med. 9:667-672.
Brown,E.R., Charles,K.A., Hoare,S.A., Rye,R.L., Jodrell,D.I., Aird,R.E., Vora,R., Prabhakar,U., Nakada,M., Corringham,R.E. et al 2008. A clinical study assessing the tolerability and biological effects of infliximab, a TNF-alpha inhibitor, in patients with advanced cancer. Ann. Oncol. 19:1340-1346.
Chang,D.H. et al. Sustained expansion of NKT cells and antigen-specific T cells after injection of alpha-galactosyl-ceramide loaded mature dendritic cells in cancer patients. J. Exp. Med. 201, 1503-1517 (2005).
Crowe,N.Y., Coquet,J.M., Berzins,S.P., Kyparissoudis,K., Keating,R., Pellicci,D.G., Hayakawa,Y., Godfrey,D.I., and Smyth,M.J. 2005. Differential anti-tumor immunity mediated by NKT cell subsets in vivo. J. Exp. Med. 202:1279-1288 (2005).
De Santo,C. et al. Invariant NKT cells reduce the immunosuppressive activity of influenza A virus-induced myeloid-derived suppressor cells in mice and humans. J. Clin. Invest 118, 4036-4048 (2008).
De,S.C., Arscott,R., Booth,S., Karydis,1., Jones,M., Asher,R., Salio,M., Middleton,M., and Cerundolo,V. 2010. Invariant NKT cells modulate the suppressive activity of IL-1 O-secreting neutrophils differentiated with serum amyloid A Nat. Immunol. 11:1039-1046.
Dhodapkar,M.V. Harnessing human CD1d restricted T cells for tumor immunity: progress and challenges. Front Biosci. 14, 796-807 (2009).
Dhodapkar,M.V., Geller,M.D., Chang,D.H., Shimizu,K., Fujii,S.I., Dhodapkar,K.M., and Krasovsky, J. 2003. A Reversible Defect in Natural Killer T Cell Function Characterizes the Progression of Premalignant to Malignant Multiple Myeloma. J. Exp. Med.
Di,S.A. et al. Inducible apoptosis as a safety switch for adoptive cell therapy. N. Engl. J. Med. 365, 1673-1683 (2011).
Dotti,G., Savoldo,B., & Brenner,M. Fifteen years of gene therapy based on chimeric antigen receptors: "are we nearly there yet?". Hum. Gene Ther. 20, 1229-1239 (2009).
Exley,M.A. et al. Selective activation, expansion, and monitoring of human iNKT cells with a monoclonal antibody specific for the TCR alpha-chain CDR3 loop. Eur. J. Immunol. 38, 1756-1766 (2008).
Fehniger,T.A. et al. Fatal leukemia in interleukin 15 transgenic mice follows early expansions in natural killer and memory phenotype CD8+ T cells. J. Exp. Med. 193, 219-231 (2001).
Friedberg,J., Jacobsen,E., Neuberg,D., Kutok,J., Munoz, O., Boussiotis,V., Reynolds,H., Fisher,D., Szot,A., Van Den,A.A. et al 2008. Targeting the follicular lymphoma microenvironment through blockade of TNFalpha with etanercept. Leuk. Lymphoma 49:902-909.
Giassi,L.J., Pearson,T., Shultz,L.D., Laning,J., Biber,K., Kraus,M., Woda,B.A, Schmidt,M.R., Woodland,R.T., Rossini,AA et al 2008. Expanded CD34+ human umbilical cord blood cells generate multiple Iymphohematopoietic lineages in NOD-scid IL2rgamma(null) mice. Exp. BioI. Med. (Maywood.) 233:997-1012.
Godfrey,D.I., Stankovic,S., & Baxter,A.G. Raising the NKT cell family. Nat. Immunol. 11, 197-206 (2010).
Goillot,E., Combaret,V., Ladenstein,R., Baubet,D., Blay,J.Y., Philip,T., and Favrot,M.C. 1992. Tumor necrosis factor as an autocrine growth factor for neuroblastoma. Gancer Res. 52:3194-3200.
Greten,F.R., Eckmann,L., Greten,T.F., Park,J.M., Li,Z.W., Egan,L.J., Kagnoff,M.F., and Karin,M. 2004. IKKbeta links inflammation and tumorigenesis in a mouse model of colitis-associated cancer. Cell 118:285296.
Grivennikov,S.I., Greten,F.R., and Karin,M. 2010. Immunity, inflammation, and cancer. GeI/140:883-899.
Hoyos, V. et al. Engineering CD19-specific T lymphocytes with interleukin-15 and a suicide gene to enhance their anti-lymphoma/leukemia effects and safety. Leukemia 24, 1160-1170 (2010).
Hsu,C. et al. Cytokine-independent growth and clonal expansion of a primary human CD8+ T-cell clone following retroviral transduction with the IL-15 gene. Blood 109, 5168-5177 (2007).
Ishikawa,A. et al. A phase I study of alpha-galactosylceramide (KRN7000)-pulsed dendritic cells in patients with advanced and recurrent non-small cell lung cancer. Clin. Cancer Res. 11, 1910-1917 (2005).
Kershaw,M.H. et al. A phase I study on adoptive immunotherapy using gene-modified T cells for ovarian cancer. Clin. Cancer Res. 12, 6106-6115 (2006).
Kim,C.H., Butcher,E.C., and Johnston,B. 2002. Distinct subsets of human Valpha24-invariant NKT cells: cytokine responses and chemokine receptor expression. Trends Immunol. 23:516-519.
Kim,C.H., Johnston,B., and Butcher,E.C. 2002. Trafficking machinery of NKT cells: shared and differential chemokine receptor expression among Valpha24(+)Vbeta11 (+) NKT cell subsets with distinct cytokineproducing capacity. Blood 100: 11-16.
Kim,D.H. et al. 4-1BB engagement costimulates NKT cell activation and exacerbates NKT cell ligand-induced airway hyperresponsiveness and inflammation. J. Immunol. 180, 2062-2068 (2008).
Kim,E.S., Serur,A., Huang,J., Manley,C.A., McCrudden,K.W., Frischer,J.S., Soffer,S.Z., Ring,L., New,T., Zabski,S. et al 2002. Potent VEGF blockade causes regression of coopted vessels in a model of neuroblastoma. Proc. Natl. Acad. Sci. U. S. A 99:11399-11404.
Kronenberg,M. & Gapin,L. The unconventional lifestyle of NKT cells. Nat. Rev. Immunol. 2, 557-568 (2002).
Kunii,N. et al. Combination therapy of in vitro-expanded natural killer T cells and alpha-galactosylceramide-pulsed antigen-presenting cells in patients with recurrent head and neck carcinoma. Cancer Sci.(2009)*.*
Lantz, O. & Bendelac,A. An invariant T cell receptor alpha chain is used by a unique subset of major histocompatibility complex class I-specific CD4+ and CD4-8- T cells in mice and humans. J. Exp. Med. 180, 1097-1106 (1994).
Liu,D. et al. IL-15 protects NKT cells from inhibition by tumor-associated macrophages and enhances antimetastatic activity. J. Clin. Invest(2012).
Louis,C.U. et al. Antitumor activity and long-term fate of chimeric antigen receptor-positive T cells in patients with neuroblastoma. Blood 118, 6050-6056 (2011).
Maher,J., Brentjens,R.J., Gunset,G., Riviere,I., & Sadelain,M. Human T-lymphocyte cytotoxicity and proliferation directed by a single chimeric TCRzeta /CD28 receptor. Nat. Biotechnol. 20, 70-75 (2002).
Mantovani,A, Aliavena,P., Sica,A, and Balkwill,F. 2008. Cancer-related inflammation. Nature 454:436-444.
Mantovani,A, Schioppa,T., Porta,C., Aliavena,P., and Sica,A 2006. Role of tumor-associated macrophages in tumor progression and invasion. Gancer Metastasis Rev. 25:315-322.
Maris,J.M., Hogarty,M.D., Bagatell,R., and Cohn,S.L. 2007. Neuroblastoma. Lancet 369:21 06-2120.
Matsuda,J.L., Gapin,L., Sidobre,S., Kieper,W.C., Tan,J.T., Ceredig,R., Surh,C.D., and Kronenberg,M. 2002. Homeostasis of V alpha 14i NKT cells. Nat. Immunol. 3:966-974.
Metelitsa,L.S. et al. Natural killer T cells infiltrate neuroblastomas expressing the chemokine CCL2. J. Exp. Med. 199, 1213-1221 (2004).
Metelitsa,L.S., Naidenko,O.V., Kant,A, Wu,H.W., Loza,M.J., Perussia,B., Kronenberg,M., and Seeger,R.C. 2001. Human NKT cells mediate anti-tumor cytotoxicity directly by recognizing target cell CD1 d with bound ligand or indirectly by producing IL-2 to activate NK cells. J. Immunol. 167:3114-3122.
Molling, J.W. et al. Low levels of circulating invariant natural killer T cells predict poor clinical outcome in patients with head and neck squamous cell carcinoma. J. Clin. Oncol. 25, 862-868 (2007).
Motohashi,S. et al. A phase I-II study of alpha-galactosylceramide-pulsed IL-2/GM-CSF-cultured peripheral blood mononuclear cells in patients with advanced and recurrent non-small cell lung cancer. J. Immunol. 182, 2492-2501 (2009).
Nieda,M. et al. Therapeutic activation of Valpha24+Vbeta11+ NKT cells in human subjects results in highly coordinated secondary activation of acquired and innate immunity. Blood 103, 383-389 (2004).
O'Konek,J.J., Iliarionov,P., Khursigara,D.S., Ambrosino,E., Izhak,L., Castillo,B.F., Raju,R., Khalili,M., Kim,H.Y., Howell,AR. et al 2011. Mouse and human iNKT cell agonist beta-mannosylceramide reveals a distinct mechanism of tumor immunity. J. Glin. Invest 121 :683-694.
Pietras,A., Hansford,L.M., Johnsson,A.S., Bridges,E., Sjolund,J., Gisselsson,D., Rehn,M., Beckman,S., Noguera,R., Navarro,S. et al 2009. HIF-2alpha maintains an undifferentiated state in neural crest-like human neuroblastoma tumor-initiating cells. Proc. Natl. Acad. Sci. U. S. A 106:16805-16810.
Pikarsky,E., Porat,R.M., Stein,!., Abramovitch,R., Amit,S., Kasem,S., Gutkovich-Pyest,E., Urieli-Shoval,S., Galun,E., and Ben-Neriah,Y. 2004. NF-kappaB functions as a tumour promoter in inflammation-associated cancer. Nature 431 :461-466.
Porcelli,S., Yockey,C.E., Brenner,M.B., & Balk,S.P. Analysis of T cell antigen receptor (TCR) expression by human peripheral blood CD4-8- alpha/beta T cells demonstrates preferential use of several V beta genes and an invariant TCR alpha chain. J. Exp. Med. 178, 1-16 (1993).
Porter,D.L., Levine,B.L., Kalos,M., Bagg,A., & June,C.H. Chimeric antigen receptor-modified T cells in chronic lymphoid leukemia. N. Engl. J. Med. 365, 725-733 (2011).
Pule,M.A. et al. Virus-specific T cells engineered to coexpress tumor-specific receptors: persistence and antitumor activity in individuals with neuroblastoma. Nat. Med. 14, 1264-1270 (2008).
Quintarelli,C. et al. Co-expression of cytokine and suicide genes to enhance the activity and safety of tumor-specific cytotoxic T lymphocytes. Blood 110, 2793-2802 (2007).
Reynolds,C.P., Tomayko,M.M., Donner,L., Helson,L., Seeger,R.C., Triche, T.J., and Brodeur,G.M. 1988. Biological classification of cell lines derived from human extra-cranial neural tumors. Prog. Clin. Biol. Res. 271 :291-306.
Riedl,S.J. & Salvesen,G.S. The apoptosome: signalling platform of cell death. Nat. Rev. Mol. Cell Biol. 8, 405-413 (2007).
Sato,N. et al. Development of an IL-15-autocrine CD8 T-cell leukemia in IL-15-transgenic mice requires the cis expression of IL-15Ralpha. Blood 117, 4032-4040 (2011).
Savoldo,B. et al. CD28 costimulation improves expansion and persistence of chimeric antigen receptor-modified T cells in lymphoma patients. J. Clin. Invest 121, 1822-1826 (2011).
Sica,A, and Bronte,V. 2007. Altered macrophage differentiation and immune dysfunction in tumor development. J. Glin. Invest 117:1155-1166.
Sica,A, Larghi,P., Mancino,A, Rubino,L., Porta,C., Totaro,M.G., Rimoldi,M., Biswas,S.K., Aliavena,P., and Mantovani,A 2008. Macrophage polarization in tumour progression. Semin. Gancer BioI. 18:349-355.
Song,L. et al. Oncogene MYCN regulates localization of NKT cells to the site of disease in neuroblastoma. J. Clin. Invest 117, 2702-2712 (2007).
Song,L. et al. Valpha24-invariant NKT cells mediate antitumor activity via killing of tumor-associated macrophages. J. Clin. Invest 119, 1524-1536 (2009).
Straathof,K.C. et al. An inducible caspase 9 safety switch for T-cell therapy. Blood 105, 4247-4254 (2005).
Swann,J., Crowe,N.Y., Hayakawa,Y., Godfrey,D.I., and Smyth,M.J. 2004. Regulation of antitumour immunity by CD1 d-restricted NKT cells. ImmunOl. Cell BioI. 82:323-331.
Swann, J.B., Coquet,J.M., Smyth,M.J., & Godfrey,D.I. CD1-restricted T cells and tumor immunity. Curr. Top. Microbiol. Immunol. 314, 293-323 (2007).
Szlosarek,P., Charles,K.A., and Balkwill,F.R. 2006. Tumour necrosis factor-alpha as a tumour promoter. Eur. J. Cancer 42:745-750.
Szlosarek,P.W., and Balkwill,F.R. 2003. Tumour necrosis factor alpha: a potential target for the therapy of solid tumours. Lancet Oncol. 4:565-573.
Tachibana,T. et al. Increased intratumor Valpha24-positive natural killer T cells: a prognostic factor for primary colorectal carcinomas. Clin. Cancer Res. 11, 7322-7327 (2005).
Tahir,S.M. et al. Loss of IFN-gamma production by invariant NK T cells in advanced cancer. J. Immunol. 167, 4046-4050 (2001).
Tey,S.K., Dotti,G., Rooney,C.M., Heslop,H.E., & Brenner,M.K. Inducible caspase 9 suicide gene to improve the safety of allodepleted T cells after haploidentical stem cell transplantation. Biol. BloodMarrow Transplant. 13, 913-924 (2007).
Thomas,S.Y., Hou,R., Boyson,J.E., Means,T.K., Hess,C., Olson,D.P., Strominger,J.L., Brenner,M.B., Gumperz,J.E., Wilson,S.B. et al 2003. CD1 d-restricted NKT cells express a chemokine receptor profile indicative of Th 1-type inflammatory homing cells. J. ImmunOl. 171 :2571-2580.
Till,B.G. et al. Adoptive immunotherapy for indolent non-Hodgkin lymphoma and mantle cell lymphoma using genetically modified autologous CD20-specific T cells. Blood 112, 2261-2271 (2008).
Uldrich,A.P. et al. NKT cell stimulation with glycolipid antigen in vivo: costimulation-dependent expansion, Bim-dependent contraction, and hyporesponsiveness to further antigenic challenge. J. Immunol. 175, 3092-3101 (2005).
van der Vliet,H.J. et al. Circulating myeloid dendritic cells of advanced cancer patients result in reduced activation and a biased cytokine profile in invariant NKT cells. J. Immunol. 180, 7287-7293 (2008).
Vera,J.F. et al. Accelerated production of antigen-specific T cells for preclinical and clinical applications using gas-permeable rapid expansion cultureware (G-Rex). J. Immunother. 33, 305-315 (2010)
Vera,J.F., Brenner,M.K., & Dotti,G. Immunotherapy of human cancers using gene modified T lymphocytes. Curr. Gene Ther. 9, 396-408 (2009).
Vinay,D.S. et al. CD137-deficient mice have reduced NK/NKT cell numbers and function, are resistant to lipopolysaccharide-induced shock syndromes, and have lower IL-4 responses. J. Immunol. 173, 4218-4229 (2004).
Waldmann,T.A. 2006. The biology of interleukin-2 and interleukin-15: implications for cancer therapy and vaccine design. Nat. Rev. ImmunOl. 6:595-601.
Yahata,T., Ando,K., Nakamura,Y., Ueyama,Y., Shimamura,K., Tamaoki,N., Kato,S., and Hotta,T. 2002. Functional human T lymphocyte development from cord blood CD34+ cells in nonobese diabetic/Shi-scid, IL-2 receptor gamma null mice. J. Immunol. 169:204-209.
Yanagisawa,K. et al. Hyporesponsiveness to natural killer T-cell ligand alpha-galactosylceramide in cancer-bearing state mediated by CD11b+ Gr-1+ cells producing nitric oxide. Cancer Res. 66, 11441-11446 (2006).
Yanagisawa,K., Seino,K., Ishikawa,Y., Nozue,M., Todoroki,T., and Fukao,K. 2002. Impaired proliferative response of V alpha 24 NKT cells from cancer patients against alpha-galactosylceramide. J. Immunol. 168:6494-6499.
Zaini,J. et al. OX40 ligand expressed by DCs costimulates NKT and CD4+ Th cell antitumor immunity in mice. J. Clin. Invest 117, 3330-3338 (2007).
Zou,W. Immunosuppressive networks in the tumour environment and their therapeutic relevance. Nat. Rev. Cancer 5, 263-274 (2005).

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. An engineered natural killer T-cell comprising an expression construct that encodes IL-2, IL-4, IL-7, IL-15 or a combination thereof for use in treatment

2. An engineered natural killer T-cell according to claim 1, wherein the cell encodes IL-2.

3. An engineered natural killed T-cell according to claim 1 or 2, wherein the cell encodes IL-15.

4. An engineered natural killer T-cell according to any one of claims 1 to 6 wherein the expression construct comprises an inducible suicide gene.

5. An engineered natural killer T-cell according to any one of claims 1 to 4, wherein the NKT cell has the properties of Vα24-invariant CD1d-reactive NKT cells.

6. An engineered natural killer T-cell according to any one of claims 1 to 5, wherein the NKT cells are engineered for the purpose of enhancing their growth under hypoxic conditions and/or facilitating their antitumor activity.

7. An engineered natural killer T-cell according to any one of claims 1 to 6, wherein the NKT cell are suitable for attacking tumor cells and also tissue that support tumor cell growth.

8. An engineered natural killer T-cell according to any one of claims 1 to 7, for use in the treatment of cancer.

9. An engineered natural killer T-cell as define in claim 8, wherein the cancer is selected from neuroblastoma, breast cancer, cervical cancer, ovary cancer, endometrial cancer, melanoma, bladder cancer, lung cancer, pancreatic cancer, colon cancer, prostate cancer, hematopoietic tumors of lymphoid lineage, leukemia, acute lymphocytic leukemia, chronic lymphocytic leukemia, B-celllymphoma, Burkitt's lymphoma, multiple myeloma, Hodgkin's lymphoma, NonHodgkin's lymphoma, myeloid leukemia, acute myelogenous leukemia (AML), chronic myelogenous leukemia, thyroid cancer, thyroid follicular cancer, myelodysplasia syndrome (MDS), tumors of mesenchymal origin, fibrosarcoma, rhabdomyosarcomas, melanoma, uveal melanoma, teratocarcinoma, neuroblastoma, glioma, glioblastoma, benign tumor of the skin, renal cancer, anaplastic large-cell lymphoma, esophageal squamous cells carcinoma, hepatocellular carcinoma, follicular dendritic cell carcinoma, intestinal cancer, muscle-invasive cancer, seminal vesicle tumor, epidermal carcinoma, spleen cancer, bladder cancer, head and neck cancer, stomach cancer, liver cancer, bone cancer, brain cancer, cancer of the retina, biliary cancer, small bowel cancer, salivary gland cancer, cancer of uterus, cancer of testicles, cancer of connective tissue, prostatic hypertrophy, myelodysplasia, Waldenstrom's macroglobinaemia, nasopharyngeal, neuroendocrine cancer myelodysplasia syndrome, mesothelioma, angiosarcoma, Kaposi's sarcoma, carcinoid, oesophagogastric, fallopian tube cancer, peritoneal cancer, papillary serous mullerian cancer, malignant ascites, gastrointestinal stromal tumor (GIST), and a hereditary cancer syndrome selected from Li-Fraumeni syndrome and Von Hippel-Lindau syndrome (VHL).

10. An engineered natural killer T-cell according to claim 1 to 9, wherein the NKT cells are for parenteral administration.

11. An engineered natural killer T-cell according to any one of claims 1 to 10, wherein the NKT cells are autologous.

12. An engineered natural killer T-cell according to any one of claims 1 to 10, wherein the NKT cells are allogenic.

13. An engineered natural killer T-cell according to any one of claims 1 to 12, wherein the therapeutic dose in the range 10⁶ to 10⁹ cells.
